# EUROPEAN PATENT APPLICATION

(11) **EP 1 731 507 A1**
(43) Date of publication of application: **13.12.2006**
(21) Application number: 05290914.0
(22) Date of filing: 26.04.2005
(51) Int. Cl.: C07D 211/84, C07D 413/04, C07D 211/82, C07D 211/86, C07D 215/20, C07D 215/24, C07D 215/36, C07D 217/24, C07D 217/22, A61K 31/47, A61K 31/44, A61P 25/28

(54) **New heterocyclic compounds, their preparation and their use as medicaments, in particular as anti-alzheimer agents**

(71) Applicant: Institut National des Sciences Appliquees de Rouen (INSA), 76131 Mont Saint Aignan Cedex (FR)
(72) Inventor: Marsais, Francis, 76000 Rouen (FR); Bohn, Pierre, 76300 Sotteville Les Rouen (FR); Levacher, Vincent, 76230 Bois-Guillaume (FR)
(74) Representative: Hirsch & Associés

(57) **Abstract**

The invention is related to compound which comprises at least one radical C=Y, Y being O or S, and an oxidable and non protonable nitrogen atom N wherein the distance (d) between the at least one carbon atom of the radical group C=Y and the nitrogen atom, when oxidized, is comprised between 0.3 and 0.8 nanometers.

The invention is related to new heterocyclic compounds defined by formula G, 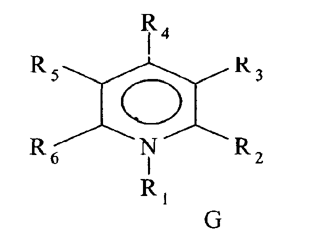 their preparation, to pharmaceutical compositions comprising them and to their use as therapeutic agents, particularly in the treatment of neurodegenerative or Alzheimer disease.

## Description

### FIELD OF THE INVENTION.

The invention relates to new heterocyclic compounds, their preparation, to pharmaceutical compositions comprising them and to their use as therapeutic agents, particularly in the treatment of neurodegenerative or Alzheimer disease.

### BACKGROUND OF THE INVENTION.

The present invention relates to the prevention, treatment and amelioration of neurodegenerative or Alzheimer's disease, and more particularly to the prevention, treatment and amelioration of Alzheimer's disease with new heterocyclic compounds which acts as inhibitors of central cholinesterase enzyme following the indirect cholinomimetic pathway described in the following bibliographic references:
**1)-**Kasa P, Rakonczay Z, Gulya K. The cholinergic system in Alzheimer's disease. Prog Neurobiol. 1997 Aug; 52(6):511-35.
**2)-**Francis PT, Palmer AM, Snape M, Wilcock GK. The cholinergic hypothesis of Alzheimer's disease: a review of progress.
**3)-**Davies P, Maloney AJF. Selective loss of central cholinergic neurones in Alzheimer's Disease.Lancet. 1976 ; ii : 1403.
**4)-**Bartus R. T., Dean R. L., Beer B and Lippa A.S. The cholinergic hypothesis of geriatric memory dysfunction.Science 1982 ; 217 : 408-17.
**5)-**Taylor P. Development of acetylcholinesterase inhibitors in the therapy of Alzheimer's Disease. Neurology 1998 ; 51(1): S30-S35

Alzheimer's disease (AD) is characterised by a progressive, inexorable loss of cognitive function associated with an excessive number of senile plaques in the cerebral cortex and subcortical gray matter, which also contains amyloid and neurofibrillary tangles consisting of tau protein. While early-onset forms of AD account for 2%-7% of cases, the common form affects persons greater than 60 years old, and its incidence increases as age advances. Million of humans have AD, and the annual cost of the disease is very high.

Today, only few cholinesterase inhibitors agent are known and used against AD. These agents are Donepezil (1), Galantamine (2) and Rivastigmine (3). However the major drawback with these molecules is the loss of their therapeutical effect with time.

Thus the need of increasing the daily doses increases the side effects, until the interruption of the treatment. It is known that these side effects are specifically caused by the peripheral activity of these molecules on cholinesterase enzyme.

There is a need for new cholinesterase inhibitors agent which could act against neurodegenerative diseases. There is a need for anti Alzheimer molecules which decrease or avoid the side effects of the known commercial drugs. Specifically there is a need for new compounds which could act as anti-Alzheimer agents without interacting with peripheral cholinesterase enzyme.

### SUMMARY OF THE INVENTION.

The present invention relates to compound comprising at least one radical C=Y, Y being O or S, and an oxidable and non protonable nitrogen atom N wherein the distance (d) between the at least one carbon atom of the radical group C=Y and the nitrogen atom, when oxidized, is comprised between 0.3 and 0.8 nanometers, preferably between 0.4 and 0.7 nm.

According to one aspect of the invention, the compound is an acetylcholinesterase inhibitor, at least 500 or preferably at least 1000 times more active in central nervous system CNS than in peripheral nervous system PNS.

According a further aspect of the invention, the compound is an acetylcholinesterase inhibitor, at least 500 or preferably at least 1000 times more active in central nervous system CNS under its oxidized form than in peripheral nervous system PNS under its non oxidized form

Another object of the invention is a compound of formula G 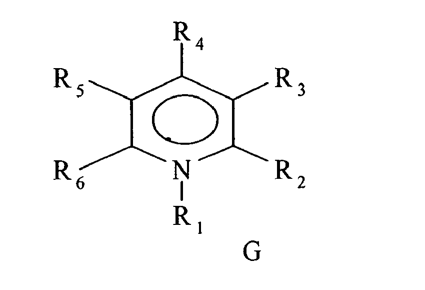 wherein:
the dotted circle line represents one double bond between CR₅-CR₆, and another double bond between either CR₂-CR₃ or CR₃-CR₄ ; and either
a) R₁ R₂ R₃ R₄ R₅ R₆ which may be identical or different are hydrogen, OH, (C₁-C₈) alkyl, aryl, heteroaryl, (C₁-C₈) alkylaryl, aryl (C₁-C₈) alkyl, alkoxy, hydroxy (C₁-C₈) alkyl, alkoxy (C₁-C₈) alkyl, phenyl, (CH₂)ₙ-COOH, Z, Z₁;
   or
b) R₄ and R₅ or c) R₅ and R₆ taken together with the carbon atoms to which they are attached form a 6-membered aromatic ring or form a 5- or 6-membered heterocyclic ring being optionally substituted by one or more identical or different group defined as OH, (C₁-C₈) alkyl, aryl, heteroaryl, (C₁-C₈) alkylaryl, aryl (C₁-C₈) alkyl, alkoxy, hydroxy (C₁-C₈) alkyl, alkoxy (C₁-C₈) alkyl, phenyl, (CH₂)ₙ-COOH, Z, Z₁; in all case a) and b) and c);
   Z is a group defined by formula -(L)ₘ-Z₁, L is (C₁-C₈) alkyl, aryl, heteroaryl, phenyl, (C₁-C₈) alkylaryl, aryl(C₁-C₈) alkyl;
   Z₁ is defined by formula 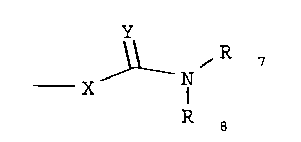 wherein X, Y is O, S; R₇, R₈ which may be identical or different are hydrogen, (C₁-C₈) alkyl, aryl, heteroaryl, (C₁-C₈) alkylaryl, phenyl, cyclopropyl, (CH₂)ₙ-COOH;
   wherein n and m are an integer ≥ 1, preferably m is comprised between 1 and 4 and n is comprised between 1 and 6; provided that at least one group R₁ to R₆ is Z or Z₁ and that R₁ is not H or Z₁;
   or a pharmaceutical salts or a stereoisomer thereof.

Another object of the invention is a compound of formula G⁺ 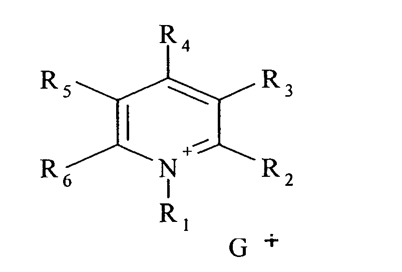 optionally under a ammonium salt form G⁺ W⁻ wherein W is the leaving group of an alkylating agent of formula R1-W, W being preferably halogen, *O*-triflate, sulfate, tosylate, mesylate, or under a pharmacological acceptable salt; and wherein R₁ R₂ R₃ R₄ R₅ R₆ have the same meaning as defined above.

In one embodiment the invention is related to a compound of formula G1a or G1b or G1⁺ 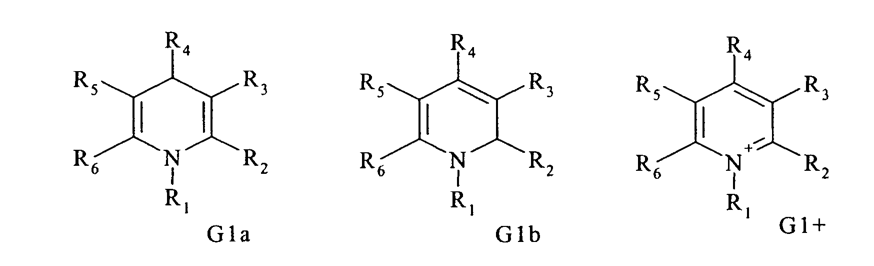 wherein R₁ R₂ R₃ R₄ R₅ R₆ are defined as above.

In one embodiment the invention is related to a compound of formula G2 or G2⁺ 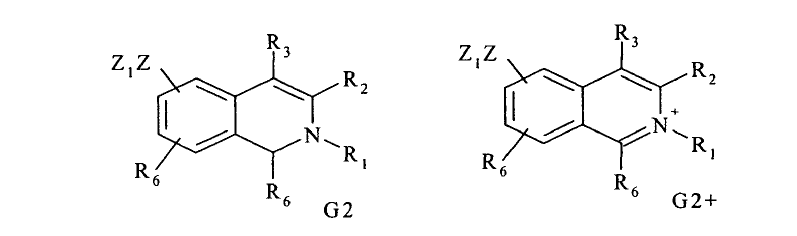 wherein R₁ R₂ R₃ R₆ Z Z₁ are defined as above.

In one embodiment the invention is related to a compound of formula G3a or G3b or G3⁺ 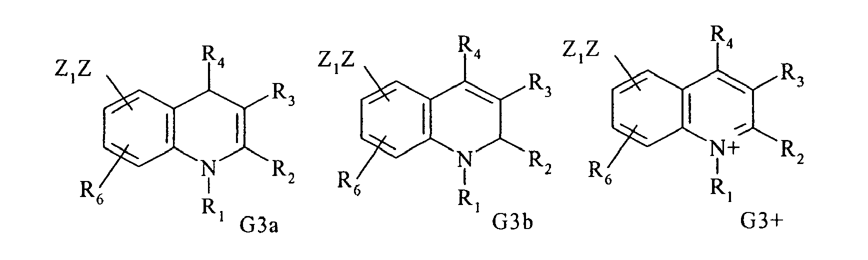 wherein R₁ R₂ R₃ R₄ R₆ Z Z₁ are defined as above.

In another embodiment the compound of the invention comprises an electron withdrawing group being in the position of the R₃ group, and R₃ is selected from the group comprising COOR, CONRR', CN, CF₃, -SO-R, -SO₂-R, -SO₂NRR', NO₂, halogen, or oxazolin, R, R' being a group alkyl, aryl, heteroaryl.

In yet another embodiment the compound of the invention is defined according to any one of the formula defined above wherein
R₁ is (C₁-C₄) alkyl, -(L)ₘ-Z₁ wherein L is aryl, m is 1;
R₂ is H, (C₁-C₄) alkyl, phenyl, aryl;
R₃ is COOR, CONRR', CN, CF₃, -SO-R, -SO₂-R, -SO₂NRR', NO₂ halogen, or oxazolin, R, R' being a group (C₁-C₄) alkyl, aryl;
Z₁ is 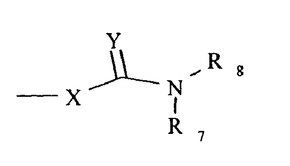 wherein X and Y are O, or X is O and Y is S or X is S and Y is O; R₇, R₈ which may be identical or different are hydrogen, (C₁-C₄) alkyl or (C₁-C₄) alkyl aryl or phenyl.

Another object of the invention is the compound of the following formula G'1, G'2, G'3. 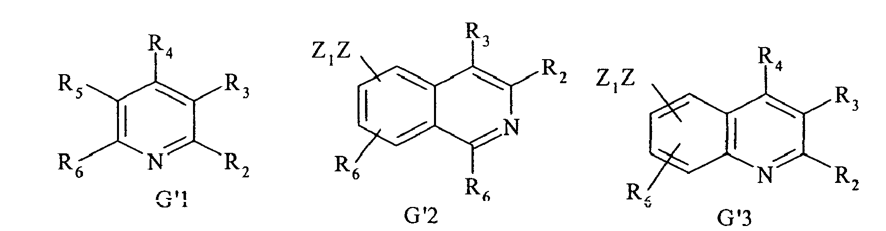 wherein R₂ R₃ R₄ R₅ R₆ Z, Z₁ have the same meaning as defined above.

Another object of the invention is the process for the preparation of a compound as defined above, which comprises the step of reduction of a compound of formula (G⁺ or Gᵢ⁺) W⁻ i being 1, 2 or 3, in the presence of a reducing agent.

In an another embodiment, the invention concerns the process for the preparation of a compound of formula (G⁺ or Gᵢ⁺)W⁻ i being 1, 2 or 3 as defined above, which comprises a step of quaternization of the nitrogen atom of a compound of formula G'1, G'2, G'3, by an alkylating agent R₁-W, R₁ being (C₁-C₈) alkyl, aryl, (C₁-C₈) alkylaryl, aryl(C₁-C₈) alkyl, alkoxy, hydroxy(C₁-C₈) alkyl, alkoxy(C₁-C₈) alkyl, phenyl, (CH₂)ₙ-COOH; W being a leaving group preferably selected from halogen, OTriflate, sulfate, tosylate, mesylate.

In a further embodiment of the invention the process for the preparation of a compound of formula G'1, G'2, G'3 comprises a step of carbamoylation of a compound of the following formula E1 or E2 or E3 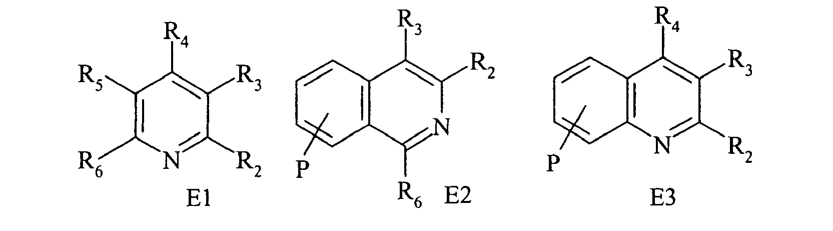 wherein P is OH, (L)ₘ OH and at least one R₅ or R₆ in formula E1 is OH or (L)ₘ OH; with an agent of formula W'-Z or W'-Z'_{1,} wherein Z'1 is 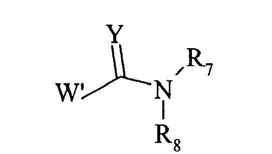 and W' is a leaving group selected from halogen, OTriflate, sulfate, tosylate, mesylate and R₂ R₃ R₄ R₅ R₆ L, m, Y, R₇, R₈ have the same meaning as defined above.

In yet another further embodiment the process for the preparation of compound of formula G⁺W or Gᵢ⁺ W⁻ i being 1, 2 or 3 as defined above and with R₁ being Z, comprises a step of quaternization of a compound of the formula E1, E2 or E3 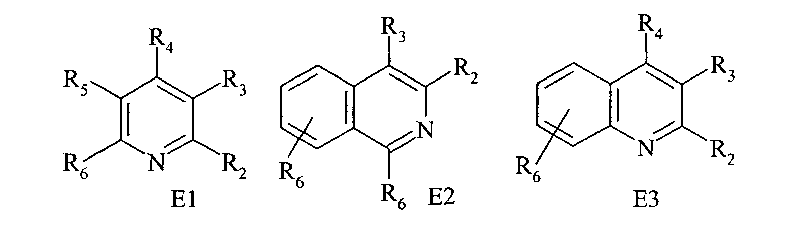 with an alkylating agent bearing a carbamate group of formula W-Z, and R₂ R₃ R₄ R₅, R₆, Z and W have the same meaning as defined above.

In a specific embodiment of the process, R₃ is hydrogen, alkyl, phenyl, (CH₂)ₙ-COOH with n being ≥ 1 or an electron withdrawing group selected from the group comprising COOR, CONRR', CN, CF₃, -SO-R, -SO₂-R, -SO₂NRR', NO₂ halogen, or oxazolin, R, R' being a group alkyl, aryl, heteroaryl.

The invention relates to a pharmaceutical composition comprising at least one compound of any one of formula G, G⁺, G1⁺, G1a, G1b, G2⁺, G2, G3a, G3b, G3⁺ and a pharmacologically acceptable carrier. In a specific embodiment, the invention relates to the pharmaceutical composition comprising at least one compound of formula G, G⁺, G1⁺, G1a, G1b, G2⁺, G2, G3a, G3b, G3⁺ for its use as an acetylcholinesterase inhibitor in the CNS.

The pharmaceutical composition according to the invention is used in the treatment of neurodegenerative diseases, preferably Alzheimer's disease in a human or other animal subject.

In another specific embodiment, the invention relates to the pharmaceutical composition comprising a compound of formula G⁺, G1⁺, G2⁺ or G3⁺ for its use as acetylcholinesterase inhibitor in the PNS, preferably for its use in the treatment of myasteny disease in a human or other animal subject.

Another object of the invention is the use of a safe and effective amount of a compound of any one of formula G, G1a, G1b, G2, G3a, G3b as defined above to for the manufacture of a prodrug for the treatment of disorders associated to neurodegenerative diseases in a human or other animal subject, wherein said treatment comprises administering said prodrug to said subject.

Another object of the invention is the use of a safe and effective amount of a compound of any one of formula G+, G1+, G2+, G3+ as defined above for the manufacture of a drug for the treatment of disorders associated to neurodegenerative diseases in a human or other animal subject, wherein said treatment comprises delivering said drug to the PNS of said subject.

Another object of the invention is the compounds of formula G or G⁺ as defined above, the names of which follow;
*1*. Ethyl 1-methyl-7-(*N,N*-dimethylcarbamate)-1,4-dihydroquinoline-3-carboxylate;
*2*. Ethyl 1-methyl-5-(*N,N*-dimethylcarbamate)-1,4-dlhydroquinoline-3-carboxylate;
*3*. Ethyl 1-methyl-5,7-di(*N,N*-dimethylcarbamate)-1,4-dihydroquinoline-3-carboxylate;
*4*. Ethyl 1-methyl-5,8-di(*N*,*N*-dimethylcarbamate)-1,4-dihydroquinoline-3-carboxylate;
*5*. Ethyl 1-methyl-5-(*N,N*-dimethylthiocarbamate)-1,4-dihydro-5-*O*-quinoline-3-carboxylate;
*6*. Ethyl 1-methyl-5-(*N,N*-dimethylthiocarbamate)-1,4-dihydro-5-*S*-quinoline-3-carboxylate;
*7*. 1-Methyl-5-(*N,N*-dimethylcarbamate)-3-(*N,N*-diethylcarboxamido)-1,4-dihydroquinoline;
*8*. 1-Methyl-7-(*N,N*-dimethylcarbamate)-3-(4,5-dihydro-4,4-dimethyl-2-oxazolyl)-1,4-dihydroquinoline;
*9.* 1-Methyl-5-(*N,N*-dimethylcarbamate)-3-trifluoromethyl-1,4-dihydroquinoline;
*10.* (+/-)-1-Methyl-3-(4-methylphenylsulfinyl)-5-(*N,N*-dimethylcarbamate)-1,4-quinoline;
*11.* 1-Methyl-3-(4-methylphenylsulfonyl)-5-(*N,N*-dimethylcarbamate)-1,4-dihydroquinoline;
*12.* 1-Methyl-5-(*N,N*-dimethylcarbamate)-3-(*N*-phenylsulfomanide)-1,4-dihydroquinoline;
*13*. 1-Methyl-6,7-di(*N,N*-dimethylcarbamate)-3-nitro-1,4-dihydroquinoline;
*14.* Ethyl 1-methyl-2-phenyl-6,7-di(*N,N*-dimethylcarbamate)-1,4-dihydroquinoline-3-carboxylate;
*15.* Ethyl 1,2,4-trimethyl-7-(*N,N*-dimethylcarbamate)-1,4-dihydro-3-quinolinecarboxylate;
*16.* 2-Methyl-7-(*N,N*-dimethylcarbamate)-1,2-dihydroisoquinoline;
*17.* 2-Methyl-7-(*N,N*-dimethylthiocarbamate)-1,2-dihydro-7-*O*-isoquinoline;
*18.* 2-Methyl-7-(*N,N*-dimethylthiocarbamate)-1,2-dihydro-7-*S*-isoquinoline;
*19.* 1,2-Dimethyl-7-(*N,N*-dimethylthiocarbamate)-1,2-dihydro-7-*O*-isoquinoline;
*20*. Ethyl 2,3-dimethyl-6,8-di(*N,N*-dimethylcarbamate)-1,2-dihydro-4-isoquinolinecarboxylate;
*21*. 2,3-Dimethyl-6,8-di(*N,N*-dimethylcarbamate)-1,2-dihydro-4-isoquinolinecarboxamide;
*22*. 2,3-Dimethyl-4-(4,5-dihydro-4,4-dimethyl-2-oxazolyl)-6,8-di(*N,N-*dimethylcarbamate)-1,2-dihydroisoquinoline;
*23*. (+/-)-2,3-Dimethyl-4-(4-methylphenylsulfinyl)-6,8-di(*N,N-*dimethylcarbamate)-1,2-dihydroisoquinoline;
*24*. 2,3-Dimethyl-4-(methylphenylsulfonyl)-6,8-di(*N,N*-dimethylcarbamate)-1,2-dihydroisoquinoline;
*25.* 2,3-Dimethyl-4-(*N*-phenylsulfonamide)-6,8-di(*N,N*-dimethylcarbamate)-1,2-dihydroisoquinoline;
*26.* 2,3-Dimethyl-4-(trifluoromethyl)-6,8-di(*N,N*-dimethylcarbamate)-1,2-dihydroisoquinoline;
*27.* Ethyl 1-methyl-5-(*N,N*-dimethylcarbamate)-1,4-dihydropyridine-3-carboxylate
*28.* Ethyl 1-methyl-5-(*N,N*-dimethylthiocarbamate)-1,4-dihydro-5-*O*-pyridine-3-carboxylate;
*29.* Ethyl 1-methyl-5-(*N,N*-dimethylthiocarbamate)-1,4-dihydro-5-*S*-pyridine-3-carboxylate;
*30*. 1-Methyl-3-(methylsulfonyl)-5-(*N,N*-dimethylcarbamate)-1,4-dihydropyridine;
*31*. 1-Methyl-3-(*N*,*N*-diethylcarboxamide)-5-(*N*,*N*-dimethylcarbamate)-1,4-dihydropyridine;
*32.* (+/-)-1-Methyl-3-(methylsufinyl)-5-(*N,N*-dimethylcarbamate)-1,4-dihydropyridine;
*33*. 1-Methyl-3-(trifluoromethyl)-5-(*N*,*N*-dimethylcarbamate)-1,4-dihydropyridine;
*34*. 1-Methyl-3-(4,5-dihydro-4,4-dimethyl-2-oxazolyl)-5-(*N*,*N-*dimethylcarbamate)-1,4-dihydropyridine;
*35*. *N,N*-Diethyl-1-methyl-5-(*N,N*-dimethylcarbamate)-1,4-dihydro-3-pyridinesulfonamide;
*36*. Ethyl 1-[2-(*N*,*N*-dimethylcarbamate)benzyl]-1,4-dihydro-3-pyridinecarboxylate;
*37*. 1-[2-(*N,N*-dimethylcarbamate)benzyl]-3-(methylcarbamoyl)-1,4-dihydropyridine;
*38*. 1-[2-(*N,N*-dimethylcarbamate)benzyl]-3-(4,5-dihydro-4,4-dimethyl-2-oxazolyl)-1,4-dihydropyridine;
*39*. *N,N*-Diethyl-1-[2-(*N,N*-dimethylcarbamate)benzyl]-1,4-dihydro-3-pyridinesulfonamide;
*40*. Ethyl 1-[2-(*N,N*-dimethylcarbamate)benzyl]-1,4-dihydro-3-quinolinecarboxylate;
*41*. 1-[2-(*N,N*-dimethylcarbamate)benzyl]-1,4-dihydro-3-(dimethylcarbamoyl)-quinoline;
*42*. 1-[2-(*N,N*-dimethylcarbamate)benzyl]-1,4-dihydro-3-(4,5-dihydro-4,4-dimethyl-2-oxazolyl) quinoline;
*43. N,N*-Dimethyl-1-[2-(*N,N*-dimethylcarbamate)benzyl]-1,4-dihydro-3-quinolinesulfonamide;
*44.* Ethyl 2-[2-(*N,N*-dimethylcarbamate)benzyl]-1,2-dihydro-4-isoquinolinecarboxylate;
*45*. 2-[2-(*N,N*-dimethylcarbamate)benzyl]-4-(N-β-phenethylcarbamoyl)-1,2-dihydroisoquinoline;
*46.* 2-[2-(*N,N*-dimethylcarbamate)benzyl]-4-(4,5-dihydro-4,4-dimethyl-2-oxazolyl)-1,2-dihydroisoquinoline;
*47. N,N*-diethyl-2-[2-(*N,N*-dimethylcarbamate)benzyl]-1,2-dihydro-4-isoquinolinesulfonamide;
*48.* 1-[2-(*N,N*-dimethylcarbamate)benzyl]-3-(trifluoromethyl)-1,4-dihydropyridine;
*49.* 1-[2-(*N,N*-dimethylcarbamate)benzyl]-3-(trifluoromethyl)-1,4-dihydroquinoline;
*50.* 1-[2-(*N,N*-dimethylcarbamate)benzyl]-3-(trifluoromethyl)-1,2-dihydroisoquinoline;
*51.* Ethyl 1-methyl-2-[2-(*N,N*-dimethylcarbamate)phenyl]-1,4-dihydropyridine-3-carboxylate;
*52.* 1-Methyl-2-[2-(*N,N*-dimethylcarbamate)phenyl]-1,4-dihydro-*N,N-*dimethylnicotinamide;
*53.* 1-Methyl-2-[2-(*N,N*-dimethylcarbamate)phenyl]-3-(methylsulfonyl)-1,4-dihydropyridine;
*54. N*-Methyl 1-methyl-2-[2-(*N,N*-dimethylcarbamate)phenyl]-1,4-dihydro-3-pyridinesulfonamide;
*55*. Ethyl 1-methyl-6-[2-(*N*,*N*-dimethylcarbamate)phenyl]-1,4-dihydropyridine-3-carboxylate;
*56*. Ethyl 1-methyl-5-[2-(*N*,*N*-dimethylcarbamate)phenyl]-1,4-dihydropyridine-3-carboxylate;
*57*. Ethyl 1-methyl-2-[2-(*N,N*-dimethylcarbamate)phenyl]-1,4-dihydroquinoline-3-carboxylate;
*58*. Ethyl 1-methyl-8-[2-(*N,N*-dimethylcarbamate)phenyl]-1,4-dihydroquinoline-3-carboxylate;
*59*. 1-[2-(*N,N*-dimethylcarbamate)phenyl]-2-methyl-1,2-dihydroisoquinoline;
*60.* 2-Methyl-4-[2-(*N,N*-dimethylcarbamate)phenyl]-1,2-dihydroisoquinoline;
*61.* Ethyl 1-methyl-7-(*N,N*-dimethylcarbamate)-1,2-dihydroquinoline-3-carboxylate;
*62.* Ethyl 1-methyl-5-(*N,N*-dimethylthiocarbamate)-1,2-dihydro-5-*S*-quinoline -3-carboxylate;
*63.* 1-Methyl 5-(*N,N*-dimethylcarbamate)-3-(4,5-dihydro-4,4-dimethyl-2-oxazolyl)1,2-dihydroquinoline;
*64.* Ethyl 1-methyl-5-(*N,N*-dimethylcarbamate)-1,2-dihydropyridine-3-carboxylate;
*65.* Ethyl 1-methyl-5-(*N,N*-dimethylthiocarbamate)-1,2-dihydro-5-*S*-pyridine-3-carboxylate;
*66.* 1-Methyl-3-(*N,N*-diethylcarboxamido)-5-(*N,N*-dimethylcarbamate)-1,2-dihydropyridine
or their corresponding ammonium form thereof.

### DETAILED DESCRIPTION.

One object of the invention is a compound which should be considered as a prodrug when it is in its oxidable and non protonable or neutral nitrogen form. This prodrug has no activity against central or peripheral acetylcholinesterase, because the oxidable nitrogen atom is non protonable at physiological pH.

Indeed, in its neutral form only, this prodrug is able to go from the blood to the central nervous system (CNS) through the blood brain barrier (BBB).

Then in vivo, in the CNS, the prodrug is converted into its oxidized form and the resultant charged form named drug, is active for action.

Indeed, once oxidized, the prodrug is transformed into a cholinesterase inhibitor because the distance d between the at least one carbon atom of the radical group C=Y and the oxidized N⁺ atom is comprised between 0.3 and 0.8 nanometers. This distance allows the compound to be in the suitable form to block the active site of the central acetylcholinesterase.

In fact, due to the presence of the oxidized N⁺ group and the radical group C=Y which belongs to a carbamate or thiocarbamate derivative, the compounds of the invention will be inserted in the active site of the enzyme and recognized by the catalytic triad of serine, histidine and glutamic acid where the serine moiety mediates the esterase activity of the acetylcholine esterase.

Finally, due to its oxidized form in the CNS compartment, the drug is entrapped in the CNS and can not go back from CNS to periphery through the BBB.

These particular features of the compound of the invention involve an effective central anticholinesterasic activity and reduction, or better suppression, of side effects due to a peripheral anticholinesterasic action.

In addition, the compound of the invention is an acetylcholinesterase inhibitor, at least 500 times more active in central nervous system CNS than in peripheral nervous system PNS or preferably at least 1000 times more active in central nervous system CNS than in peripheral nervous system PNS.

In one embodiment, the compound of the invention is an acetylcholinesterase inhibitor, at least 500 times more active in central nervous system CNS under its oxidized form than in peripheral nervous system PNS under its non oxidized form or under the prodrug in its non oxidized form.

In another embodiment, the compound of the invention is an acetylcholinesterase inhibitor, at least 1000 times more active in central nervous system CNS under its oxidized form than in peripheral nervous system PNS under its non oxidized form or under the prodrug in its non oxidized form.

The compound of the invention is selected for presenting the particular feature of having a distance d comprised between 0.3 and 0.8 nanometers preferably between 0.4 and 0.7 nanometers.

This distance is calculated on the oxidized form of a defined compound from a modelisation Chemdraw or a Cerius software on a Silicon graphic station.
The adequate distance d is defined from the structure of acetylcholinesterase enzyme active site reported in the literature ("Structure of acetylcholinesterase complexed with E2020 (Aricept®): implications for the design of new anti-Alzheimer drugs." Structure Pages 297-307 Gitay Kryger, Israel Silman and Joel L Sussman; "The rationale for E2020 as a potent acetylcholinesterase inhibitor" Bioorganic and Medicinal Chemistry. Pages 1429-1446 Yoshiyuki Kawakami, Atsushi Inoue, Takatoshi Kawai, Misako Wakita, Hachiro Sugimoto and Anton J. Hopfinger; "Transition State Structure and Rate Determination for the Acylation Stage of Acetylcholinesterase Catalyzed Hydrolysis of (Acetylthio)choline" Siobhan Malany, Monali Sawai, R. Steven Sikorski, Javier Seravalli, Daniel M. Quinn, Zoran Radic, Palmer Taylor, Chanoch Kronman, Baruch Velan, and Avigdor Shafferman Journal of American Chemical Society. pp 2981 - 2987; "Direct determination of Acetyl-Enzyme Intermediate in the Acetylcholinesterase-catalysed Hydrolysis of Acetylcholine and acetylthiocholine" Harry C. Froede and Irwin B. Wilson The journal of Biological Chemistry, 1984, 259, pp 11010-11013.)

The present invention relates to compounds described by structural formula G 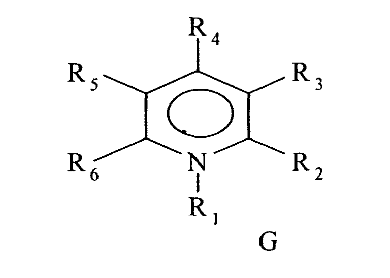 wherein the dotted circle line represents one double bond between CR₅-CR₆, and another double bond between either CR₂-CR₃ or CR₃-CR₄ ; and either
a) R₁ R₂ R₃ R₄ R₅ R₆ which may be identical or different are hydrogen, OH, (C₁-C₈) alkyl, aryl, heteroaryl, (C₁-C₈) alkylaryl, aryl (C₁-C₈) alkyl, alkoxy, hydroxy (C₁-C₈) alkyl, alkoxy (C₁-C₈) alkyl, phenyl, (CH₂)ₙ-COOH, Z, Z₁;
   or
b) R₄ and R₅ or c) R₅ and R₆ taken together with the carbon atoms to which they are attached form a 6-membered aromatic ring or form a 5- or 6-membered heterocyclic ring being optionally substituted by one or more identical or different group defined as OH, (C₁-C₈) alkyl, aryl, heteroaryl, (C₁-C₈) alkylaryl, aryl (C₁-C₈) alkyl, alkoxy, hydroxy (C₁-C₈) alkyl, alkoxy (C₁-C₈) alkyl, phenyl, (CH₂)ₙ-COOH, Z, Z₁; in all case a) and b) and c);
   Z is a group defined by formula -(L)ₘ-Z₁, L is (C₁-C₈) alkyl, aryl, heteroaryl, phenyl, (C₁-C₈) alkylaryl, aryl(C₁-C₈) alkyl;
   Z₁ is defined by formula 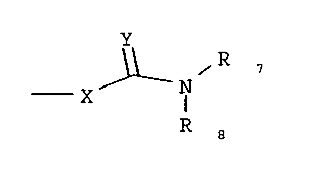 wherein X, Y is O, S; R₇, R₈ which may be identical or different are hydrogen, (C₁-C₈) alkyl, aryl, heteroaryl, (C₁-C₈) alkylaryl, phenyl, cyclopropyl, (CH₂)ₙ-COOH;
   wherein n and m are an integer ≥ 1, preferably m is comprised between 1 and 4 and n is comprised between 1 and 6;
   provided that at least one group R₁ to R₆ is Z or Z₁ and that R₁ is not H or Z₁; or a pharmaceutical salts or a stereoisomer thereof.

By "alkyl" radical containing 1 to 8 carbon atoms, is intended to mean methyl, ethyl, propyl, isopropyl, as well as butyl, pentyl or hexyl etc... linear or branched radical.

As used herein, "aryl" is intended to mean any stable monocyclic or bicyclic carbon ring of up to 7 atoms in each ring, wherein at least one ring is aromatic.
Examples of such aryl elements include, but are not limited to, phenyl, naphthyl, tetrahydro- naphthyl, indanyl, biphenyl, phenanthryl, anthryl or acenaphthyl. In cases where the aryl substituent is bicyclic and one ring is non-aromatic, it is understood that attachment is via the aromatic ring.

The terms "arylakyl" or "alkylaryl" radical, include an alkyl portion where alkyl is as defined above and aryl portion where aryl are as defined above. Examples of arylalkyl include, but are not limited to, benzyl, halobenzyl, phenylethyl, phenylpropyl, halophenylethyl, thienylethyl, thienylpropyl. Examplkes of alkylaryl include toluene, ethylbenzene, propylbenzene.

By "halo" or "halogen" radical or by halogen atom is intended to mean fluorine, chlorine, bromine or iodine.

"Cycloalkyl" as used herein is intended to include non-aromatic cyclic hydrocarbon groups, having the specified number of carbon atoms, which may or may not be bridged or structurally constrained. Examples of such cycloalkyls include, but are not limited to, cyclopropyl, cyclobutyl, cyclopentyl, cyclohexyl, adamantyl, cyclooctyl, cycloheptyl.

"Alkoxy" represents either a cyclic or non-cyclic alkyl group of indicated number of carbon atoms attached through an oxygen bridge. "Alkoxy" therefore encompasses the definitions of alkyl and cycloalkyl above.

The invention concerns also stereoisomer or a mixture of stereoisomers of the compound of formula G as defined above in any ratio, or a physiologically acceptable salt thereof.

In a preferred embodiment the compound of the invention as cited above comprises an electron withdrawing group in beta position from the nitrogen atom in order to increase the stability of the final compound of formula G. Thus the group R₃ being in the position as defined above is selected from the group comprising COOR, CONRR', CN, COR, CF₃, -SO-R, -SO₂-R, -SO₂NRR', NO₂, halogen or oxazolin, R, R' being an alkyl, aryl, heteroaryl group.

In a preferred embodiment of the invention, the compounds are selected from those of formula G1a or G1b: 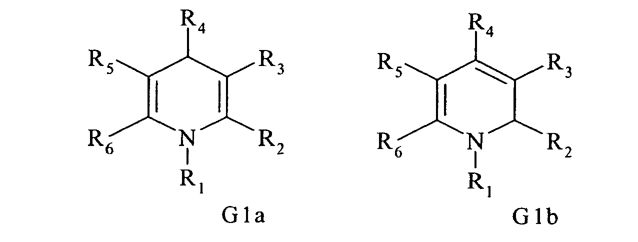 wherein R₁ R₂ R₃ R₄ R₅ R₆ have the same meaning as defined above.

Among the compounds of formula G1a, a quite particular subject of the invention is the compounds the names of which follow:
Ethyl 1-methyl 5-(*N,N*-dimethylcarbamate)-1,4-dihydropyridine-3-carboxylate;
Ethyl 1-methyl-5-(*N,N*-dimethylthiocarbamate)-1,4-dihydro-5-O-pyridine-3-carboxylate;
Ethyl 1-methyl-5-(*N,N*-dimethylthiocarbamate)-1,4-dihydro-5-S-pyridine-3-carboxylate;
1-Methyl-3-(methylsulfonyl)-5-(*N,N*-dimethylcarbamate)-1,4-dihydropyridine;
1-Methyl-3-(*N,N*-diethylcarboxamido)-5-(*N,N*-dimethylcarbamate)-1,4-dihydropyridine;
(+/-)-1-Methyl-3-(methylsufinyl)-5-(*N,N*-dimethylcarbamate)-1,4-dihydropyridine;
1-Methyl-3-(trifluoromethyl)-5-(*N*,*N*-dimethylcarbamate)-1,4-dihydropyridine;
1-Methyl-3-(4,5-dihydro-4,4-dimethyl-2-oxazolyl)-5-(*N*,*N*-dimethylcarbamate)-1,4-dihydropyridine;
*N*,*N*-diethyl-1-methyl-5-(*N,N*-dimethylcarbamate)-1,4-dihydro-3-pyridinesulfonamide;
Ethyl 1-[2-(*N,N*-dimethylcarbamate)benzyl]-1,4-dihydro-3-pyridinecarboxylate;
1-[2-(*N*,*N*-dimethylcarbamate)benzyl]-3-(methylcarbamoyl)-1,4-dihydropyridine;
1-[2-(*N,N*-dimethylcarbamate)benzyl]-3-(4,5-dihydro-4,4-dimethyl-2-oxazolyl)-1,4-dihydropyridine;
*N,N*-diethyl-1-[2-(*N,N*-dimethylcarbamate)benzyl]-1,4-dihydro-3-pyridinesulfonamide;
1-[2-(*N,N*-dimethylcarbamate)benzyl]-3-(trifluoromethyl)-1,4-dihydropyridine;
Ethyl 1-methyl-2-[2-(*N,N*-dimethylcarbamate)phenyl]-1,4-dihydropyridine-3-carboxylate;
1-Methyl-2-[2-(*N,N*-dimethylcarbamate)phenyl]-1,4-dihydro-*N,N-*dimethylnicotinamide;
1-Methyl-2-[2-(*N,N*-dimethylcarbamate)phenyl]-3-(methylsulfonyl)-1,4-dihydropyridine;
*N*-methyl-1-methyl-2-[2-(*N,N*-dimethylcarbamate)phenyl]-1,4-dihydro-3-pyridinesulfonamide;
Ethyl 1-methyl-6-[2-(*N,N*-dimethylcarbamate)phenyl]-1,4-dihydropyridine-3-carboxylate;
Ethyl 1-methyl-5-[2-(*N*,*N*-dimethylcarbamate)phenyl]-1,4-dihydropyridine-3-carboxylate.

Among the compounds of formula G1b a quite particular subject of the invention is the compounds the names of which follow:
Ethyl 1-methyl 5-(*N*,*N*-dimethylcarbamate)-1,2-dihydropyridine-3-carboxylate ;
Ethyl 1-methyl-5-(*N,N*-dimethylthiocarbamate)-1,2-dihydro-5-*S*-pyridine-3-carboxylate;
1-Methyl-3-(*N*,*N*-diethylcarboxamido)-5-(*N*,*N*-dimethylcarbamate)-1,2-dihydropyridine.

In a preferred embodiment of the invention, the compounds are selected from those of formula G2: 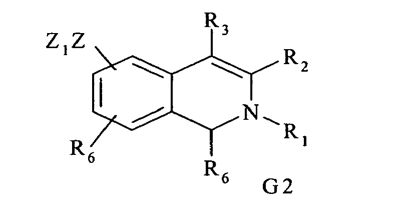 wherein R₁ R₂ R₃ R₆ Z Z₁ have the same meaning as defined above.

Among the compounds of formula G2 a quite particular subject of the invention is the compounds the names of which follow:
2-Methyl-7-(*N,N*-dimethylcarbamate)-1,2-dihydroisoquinoline;
2-Methyl-7-(*N,N*-dimethylthiocarbamate)-1,2-dihydro-7-*O*-isoquinoline;
2-Methyl-7-(*N,N*-dimethylthiocarbamate)-1,2-dihydro-7-*S*-isoquinoline;
1,2-Dimethyl-7-(*N,N*-dimethylthiocarbamate)-1,2-dihydro-7-*O*-isoquinoline;
Ethyl 2,3-dimethyl-6,8-di(*N,N*-dimethylcarbamate)-1,2-dihydro-4-isoquinolinecarboxylate;
2,3-Dimethyl-6,8-di(*N,N*-dimethylcarbamate)-1,2-dihydro-4-isoquinolinecarboxamide;
2,3-Dimethyl-4-(4,5-dihydro-4,4-dimethyl-2-oxazolyl)-6,8-di(*N,N-*dimethylcarbamate)-1,2-dihydroisoquinoline;
(+/-)-2,3-Dimethyl-4-(4-methylphenylsulfinyl)-6,8-di(*N,N*-dimethylcarbamate)-1,2-dihydroisoquinoline;
2,3-Dimethyl-4-[(4-methylphenyl)sulfonyl]-6,8-di(*N,N*-dimethylcarbamate)-1,2-dihydroisoquinoline;
2,3-Dimethyl-4-[N-phenylsulfonamide]-6,8-di(*N,N*-dimethylcarbamate)-1,2-dihydroisoquinoline;
2,3-Dimethyl-4-(trifluoromethyl)-6,8-di(*N,N*-dimethylcarbamate)-1,2-dihydroisoquinoline;
Ethyl 2-[2-(*N,N*-dimethylcarbamate)benzy]1,2-dihydro-4-isoquinolinecarboxylate;
2-[2-(*N*,*N*-dimethylcarbamate)benzyl]-4-(N-β-phenethylcarbamoyl)-1,2-dihydroisoquinoline;
2-[2-(*N,N*-dimethylcarbamate)benzyl]-4-(4,5-dihydro-4,4-dimethyl-2-oxazolyl)-1,2-dihydroisoquinoline;
*N,N*-diethyl-2-[2-(*N,N*-dimethylcarbamate)benzyl]-1,2-dihydro-4-isoquinolinesulfonamide;
1-[2-(*N,N*-dimethylcarbamate)benzyl]-3-(trifluoromethyl)-1,2-dihydroisoquinoline;
1-[2-(*N,N*-dimethylcarbamate)phenyl]-2-methyl-1,2-dihydroisoquinoline;
2-Methyl-4-[2-(*N,N*-dimethylcarbamate)phenyl]-1,2-dihydroisoquinoline.

In a preferred embodiment of the invention, the compounds are selected from those of formula G3a or G3b: 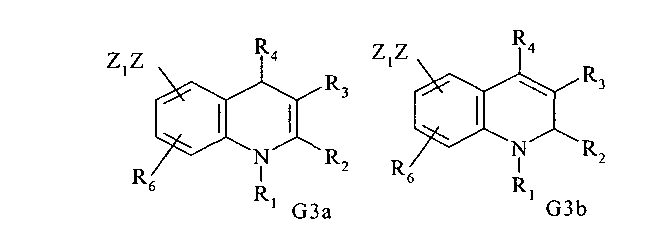 wherein R₁ R₂ R₃ R₄ R₆ Z Z₁ have the same meaning as defined above.

Among the compounds of formula G3a a quite particular subject of the invention is the compounds the names of which follow:
Ethyl 1-methyl-7-(*N,N*-dimethylcarbamate)-1,4-dihydroquinoline-3-carboxylate;
Ethyl 1-methyl-5-(*N,N*-dimethylcarbamate)-1,4-dihydroquinoline-3-carboxylate.;
Ethyl 1-methyl-5,7-di (*N,N*-dimethylcarbamate)-1,4-dihydroquinoline-3-carboxylate;
Ethyl 1-methyl-5,8-di(*N,N*-dimethylcarbamate)-1,4-dihydroquinoline-3-carboxylate;
Ethyl 1-methyl-1,4-dihydro-5-*O*-quinoline-*N*,*N*-dimethylthiocarbamate-3-carboxylate;
Ethyl 1-methyl-1,4-dihydro-5-*S*-quinoline-*N,N*-dimethylthiocarbamate-3-carboxylate;
1-Methyl-5-(*N,N*-dimethylcarbamate)-3-(*N,N*-diethylcarboxamido)-1,4-dihydroquinoline;
1-Methyl-7-(*N,N*-dimethylcarbamate)-3-(4,5-dihydro-4,4-dimethyl-2-oxazolyl)-1,4-dihydroquinoline;
1-Methyl-5-(*N,N*-dimethylcarbamate)-3-trifluoromethyl-1,4-dihydroquinoline;
(+/-)-1-Methyl-3-(4-methylphenylsulfinyl)-5-(*N,N*-dimethylcarbamate)-1,4-dihydroquinoline ;
1-Methyl-3-(4-methylphenylsulfonyl)-5-(*N,N*-dimethylcarbamate)-1,4-dihydroquinoline;
1-Methyl-5-(*N,N*-dimethylcarbamate)-3-(N-phenylsulfomanide)-1,4-dihydroquinoline;
1-Methyl-6,7-di(*N,N*-dimethylcarbamate)-3-nitro-1,4-dihydroquinoline;
Ethyl 1-methyl-2-phenyl-6,7-di(*N,N*-dimethylcarbamate)-1,4-dihydroquinoline-3-carboxylate;
Ethyl 1,2,4-trimethyl-7-(*N,N*-dimethylcarbamate)-1,4-dihydro-3-quinolinecarboxylate;
Ethyl 1-[2-(*N*,*N*-dimethylcarbamate)benzyl]-1,4-dihydro-3-quinolinecarboxylate;
1-[2-(*N*,*N*-dimethylcarbamate)benzyl]-3-(dimethylcarbamoyl)-1,4-dihydroquinoline;
1-[2-(*N*,*N*-dimethylcarbamate)benzyl]-3-(4,5-dihydro-4,4-dimethyl-2-oxazolyl)-1,4-dihydroquinoline;
*N*,*N*-dimethyl-1-[2-(*N*,*N*-dimethylcarbamate)benzyl]-1,4-dihydro-3-quinolinesulfonamide;
1-[2-(*N*,*N*-dimethylcarbamate)benzyl]-3-(trifluoromethyl)-1,4-dihydroquinoline;
Ethyl 1-methyl-2-[2-(*N,N*-dimethylcarbamate)phenyl]-1,4-dihydroquinoline-3-carboxylate ;
Ethyl 1-methyl-8-[2-(*N,N*-dimethylcarbamate)phenyl]-1,4-dihydroquinoline-3-carboxylate.

Among the compounds of formula G3b a quite particular subject of the invention is the compounds the names of which follow
Ethyl 1-methyl-7-(*N,N*-dimethylcarbamate)-1,2-dihydroquinoline-3-carboxylate ;
Ethyl 1-methyl-(*N,N*-dimethylthiocarbamate)-1,2-dihydro-5-*S*-quinoline-3-carboxylate ;
1-Methyl-5-(*N,N*-dimethylcarbamate)-3-(4,5-dihydro-4,4-dimethyl-2-oxazolyl)-1,2-dihydroquinoline.

Another object of the invention is the compound of formula G⁺ or G⁺1, G⁺2, G⁺3 wherein all the specific compounds cited above are in their corresponding positively charged form such as pyridinium or quinolinium or isoquinolinium form. 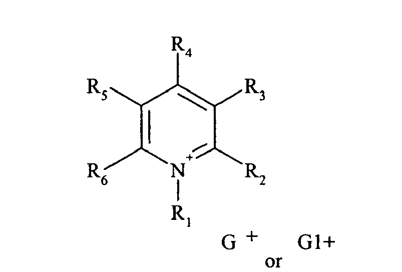 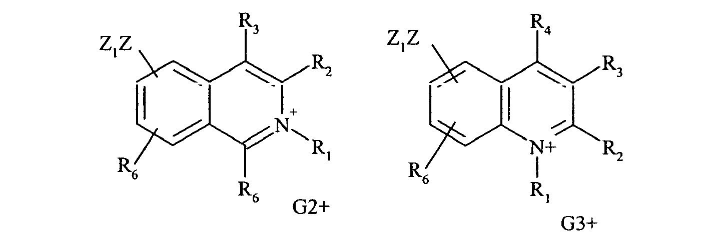 wherein R₁ R₂ R₆ R₃ R₅ R₆ Z Z₁ have the same meaning as defined above.

These compounds are either the starting product for obtaining the compound of respectively formula G, G1a, G1b, G2, G2a, G2b by a step of reduction, or the active drug which is liberated in vivo in the CNS.

As starting product they are under a salt form G⁺ W⁻ wherein W is the leaving group of the alkylating agent R1-W which is involved in the quaternization reaction step as explain below.

As an active drug in the CNS, they are in an ammonium non salted form.

As intermediate compounds for the preparation of compounds of formula G⁺, G⁺1, G⁺2, G⁺3 as described above, another object of the invention is the compounds of formula G' or G'1, G'2, G'3:

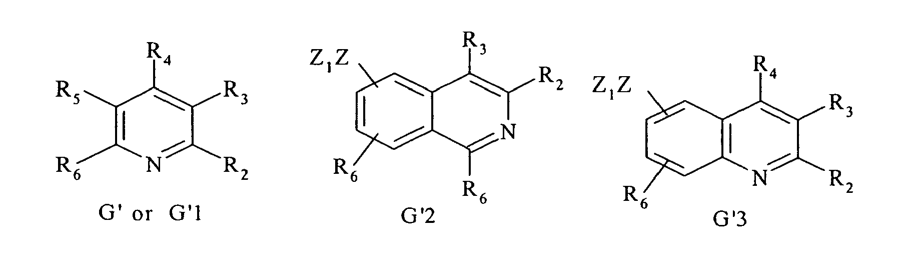

These compounds are starting products for obtaining, by a step of quatemarization with an alkylating agent R₁-W, the compounds of formula G⁺, G⁺1, G⁺2, G⁺3 according to the process explained below.

Another object of the invention is a process allowing the preparation of the compounds of formula general G and the sub family of compound of formula G1a G1b, G2, G3a, G3b as defined above.

This process comprises a stage of reduction of a compound of formula Gᵢ⁺ W⁻ is 1, 2 or 3 as defined above in the presence of a reducing agent.

Regioselective 1,4-reduction is carried out with sodium dithionite in the presence of sodium carbonate, but other reducing agents may be used in this step, for example NaBH₃CN and BNAH, providing the 1,4-dihydropyridine of formula G1a or 1,4-dihydroquinolines of formula G3a. The use of sodium borohydride give rise to the formation of a pure 1,2-dihydroisoquinoline of formula G2 or a mixture of 1,2-and 1,4-dihydroquinolines or 1,2- and 1,4-dihydropyridines. Chemical separation give rise to the formation of pure 1,2-dihydropyridine of formula G1b or 1,2-dihydroquinolines of formula G3b

Another object of the invention is a process allowing the preparation of the compounds of formula Gᵢ⁺W⁻, i being 1, 2 or 3 as defined above which comprises a first step of carbamoylation of a compound of formula E1 or E2 or E3:

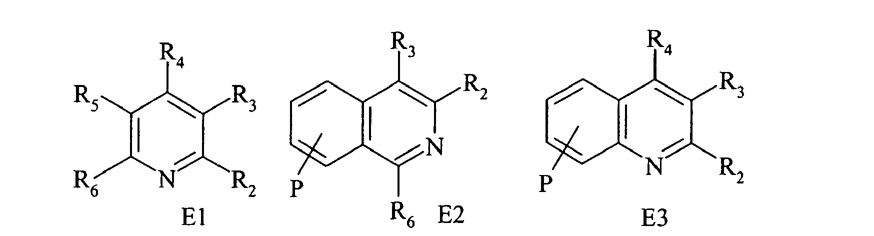

Carbamoylation is carried out in the presence of metal hydride such as sodium hydride with an agent of formula W'-Z or W'-Z'₁ such as for example a dialkylcarbamoyl halide in a solvent. Also thiocarbamoylation is achieved in the presence of dialkylthiocarbamoyl halide.

In this step the reaction of carbamoylation is carried out on starting compounds wherein either R₅ or R₆ or P are a hydroxy group.

The process comprises a following step of quaternization of the nitrogen atom by treatment of the resulting carbamate with all type of alkylating agents R₁-W in a solvant which provides the desired quinolinium salt.

The synthesis of the starting product of formula E1, E2, E3 is described below.
When the reaction sequence carbamoylation, quaternization and reduction steps is carried out on compounds of formula E1, E2, E3, the respective compound of formula G1a, G1b, G2, G3a, G3b are obtained.

The synthesis of the following pyridine derivatives of formula E1 is reported in the literature.
3-Methoxy-5-(methylsulfinyl)pyridine (Phosphorus, Sulfur and Silicon and the Related Elements, 1992, 66, 127-137);
3-Methoxy-5-(methylsulfonyl)pyridine (Tetrahedron, 1985, pages 173-1384)
Ethyl 5-methoxypyridine-3-carboxylate (Journal of medicinal chemistry, 2000, 43, 3168-3185);
3-Cyano-5-methoxypyridine (Journal of Medicinal Chemistry, 2000, 43, 3168-3185);

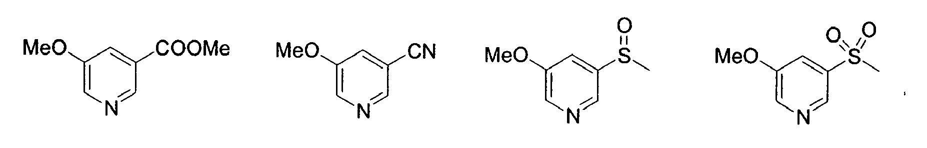

From these available pyridines, the desired carbamates, *O*-isoquinoline thiocarbamates and *S*-thiocarbamates of formula G1a or G1b substituted on the 3-position by an ester, a ketone, a trifluoromethyl, an amide, a sulfoxyde, a sulfone, a sulfonamide, an oxazoline are prepared following the same reaction sequence carbamoylation, quatemarization and reduction steps as that reported previously.

The synthesis of the isoquinolines derivatives of formula E2 are prepared as following:
The preparation of the following methoxy isoquinolines is reported in the literature by Pommeranz-Fritsch cyclisation.
   5-Methoxyisoquinoline (Bioorganic and Medicinal Chemistry, 1999, 2647-2666);
   6-Methoxyisoquinoline (Bioorganic and Medicinal Chemistry Letters, 2003, 1345-1348);
   7-Methoxyisoquinoline (Bioorganic and Medicinal Chemistry, 1999, 2647-2666);
   8-Methoxyisoquinoline (Bioorganic and Medicinal Chemistry, 1999, 2647-2666);
   5,7-Methoxyisoquinoline ((Tetrahedron, 37, 1981, pages 3977-3980);
   5,8-Methoxyisoquinoline (Journal of Chemical Society, Perkin Transactions 1: Organic and Bio-organic Chemistry, 1974, 2185-2190);
   6,8-Methoxyisoquinoline (Journal of Organic Chemistry, 32, 1967, page 2689-2692);
   6,7-Methoxyisoquinoline (Tetrahedron, 37, 1981, 3977-3980);
   7,8-Methoxyisoquinoline (Tetrahedron Letters, 38, 3159-3162);

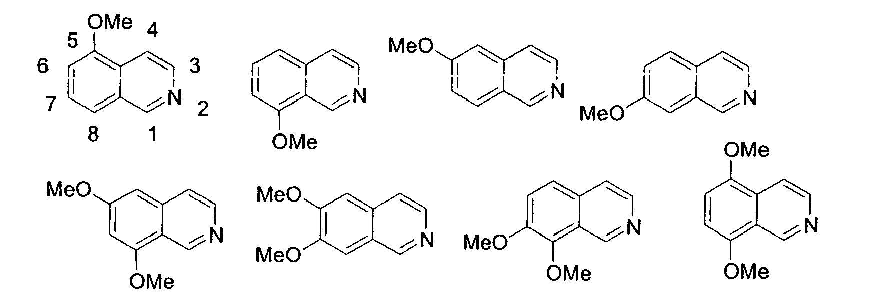

The previous methoxy isoquinolines are transformed into the desired isoquinoline carbamates, *O*-isoquinoline thiocarbamates and *S*-isoquinoline thiocarbamates of formula G2 according to the previous reaction sequence above, *O-*demethylation, carbamoylation, thiocarbamoylation, quaternization and reductions steps.

The desired 1-substituted isoquinolines are prepared by addition of an alkyllithium, a Grignard reagent or a cuprate on the corresponding isoquinolinium salts, affording the 1,2-dihydroisoquinolines.

The following 4-cyano isoquinolines and methyl isoquinoline-4-carboxylate derivatives are reported in the literature:
4-Cyano-6,7-dimethoxyisoquinolines (Canadian Journal of Chemistry, 1968, 46, 1160-1163);
3-Methyl 4-cyano-6,8-dimethoxyisoquinolines (Tetrahedron Letters, 1968, 44, 4631-4634);
Methyl 6-methoxyisoquinoline-4-carboxylate (EP1466604);

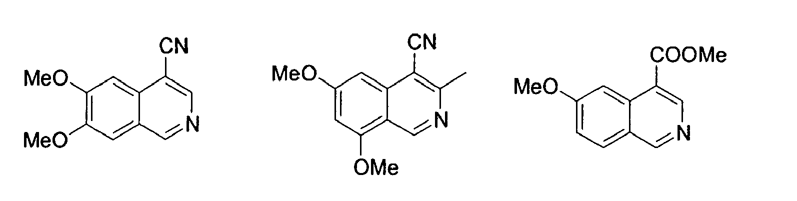

From these 4-cyanoisoquinolines and methyl isoquinoline-4-carboxylate, the desired carbamates, *O*-isoquinolinethiocarbamate and *S*-thiocarbamates of formula G2 substituted on the 4-position by an ester, a trifluoromethyl an amide, a sulfoxyde, a sulfone, a sulfonamide, an oxazoline are prepared following the same reaction sequence as that reported previously.

The quinolines derivative of formula E3 are prepared as following:
The synthesis of the following cyanoquinolines is reported in the literature (Tetrahedron Letters, 39, (1998) 4013-4016).

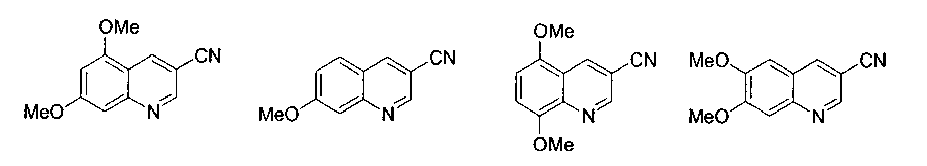

The preparation of the following ethyl quinoline-3-carboxylates is reported in the literature (Journal of Medicinal Chemistry 1995, 38, 950-957).

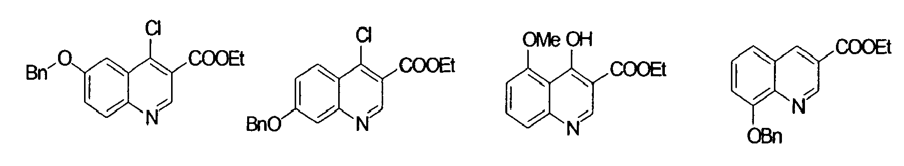

The cyanoquinolines cited above are treated with 48% HBr in aqueous solution to promote deprotection of the methoxy group and hydrolysis of the 3-cyano group. The corresponding carboxylic acids are then converted into esters with thionyl chloride in refluxing ethanol.

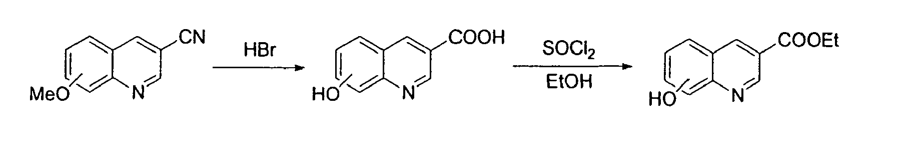

Carbamoylation is accomplished in the presence of sodium hydride with dimethylcarbamoyl chloride in THF. Treatment of the resulting carbamate with methyl triflate in dichloromethane provides the desired quinolinium salt. All type of alkylating agents can be used in the quaternization step (CH₃I, PhCH₂X, XCH₂COOR). Regioselective 1,4-reduction is carried out with sodium dithionite in the presence of sodium carbonate (NaBH₃CN and BNAH may be used in this step) providing the 1,4-dihydroqunolines. The use of sodium borohydride gives rise to the formation of a mixture of 1,2- and 1,4-dihydroquinolines.

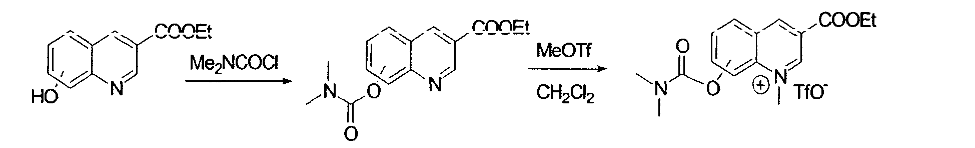

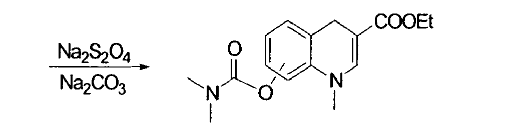

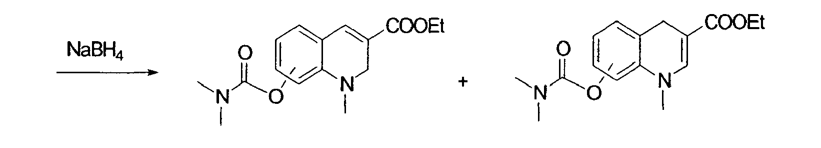

Thiocarbamoylation is achieved in the presence of dimethylthiocarbamoyl chloride. Quaternization and reduction of the corresponding O-quinoline thiocarbamates is accomplished as described above.

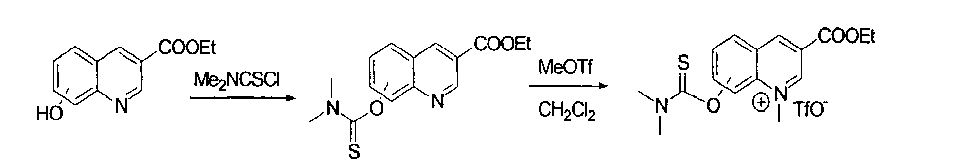

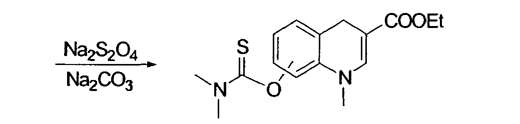

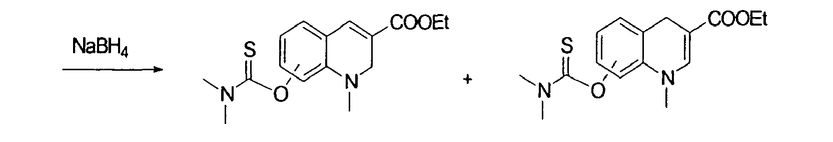

The *O*-quinolinethiocarbamate derivatives underwent thermal rearrangement to give rise to the corresponding *S*-quinolinethiocarbamates. Quaternization and reduction of the corresponding *S*-quinolinethiocarbamates is accomplished as described above.

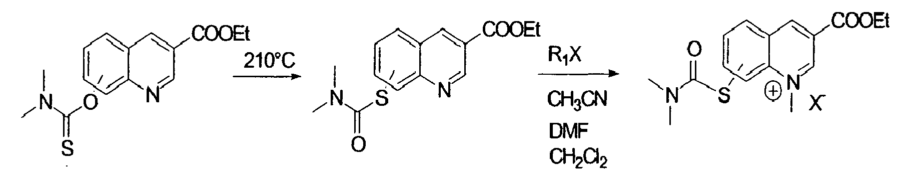

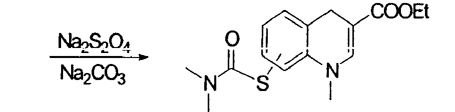

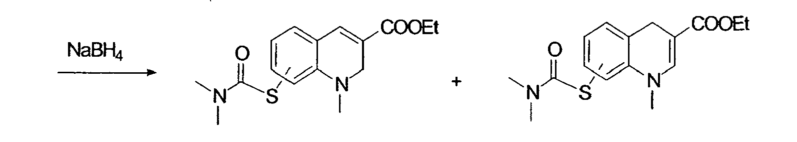

Ethyl 5-methoxyquinoline-3-carboxylate is prepared by chlorination of ethyl 4-hydroxy-5-methoxyquinoline-3-carboxylate with phosphorus oxychloride. Dechlorination was performed by hydrogenolysis. The *O*-demethylation, carbamoylation, thiocarbamoylation, quaternization and reduction steps are performed as described above.

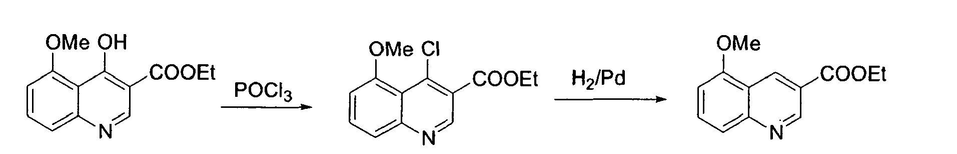

Otherwise, 5-methoxy isomers can be prepared from 2-amino-6-methoxybenzoic acid following the general procedure reported in the Journal of Medicinal Chemistry, (2001), 44, 822-833. Dechlorination of 4-chloro-5-methoxy quinoline-3-carbonitrile affords the desired 5-methoxyquinoline-3-carbonitrile. The *O*-demethylation, carbamoylation, thiocarbamoylation, quaternization and reduction steps are performed as described above.

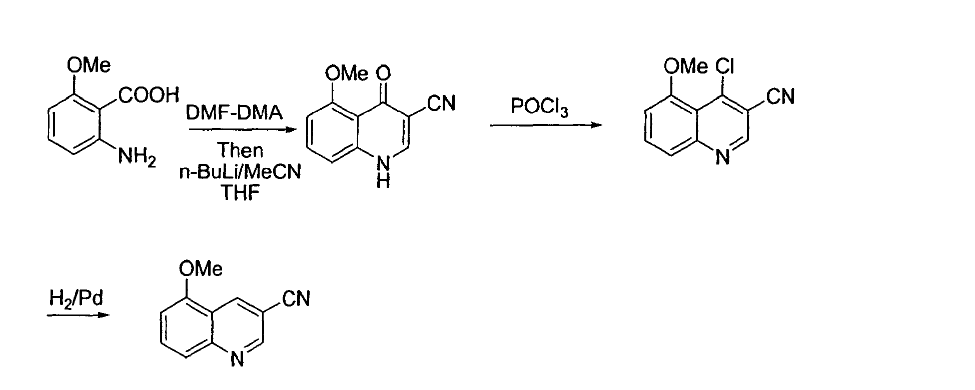

The 3-carboxamidoquinolines are prepared by amidification of 3-quinolinecarboxylic acid derivatives, followed by *O*-debenzylation. The carbamoylation, thiocarbamoylation, quaternization and reduction steps are performed as described above.

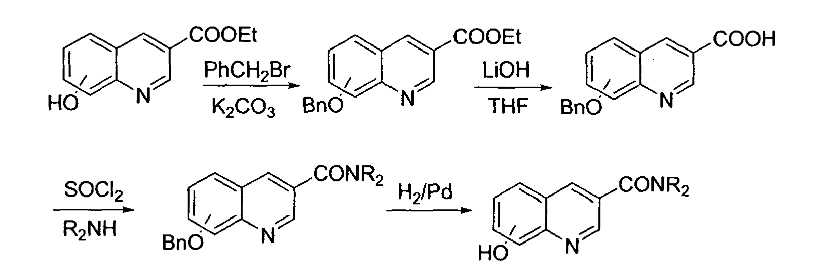

Alternatively, 3-carboxamidoquinolines are prepared from 3-cyano quinolines by partial hydrolysis using H₂O₂ in the presence of NaOH at 0°C. The deprotection of the methoxy group is achieved under mild conditions with BBr₃ in CH₂Cl₂. The carbamoylation, thiocarbamoylation, quaternization and reduction steps are performed as described above.

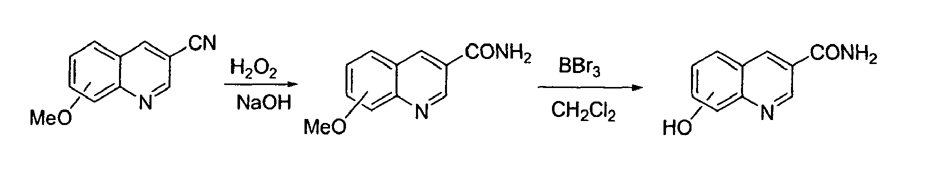

On the other hand, 3-carboxamidoquinolines are prepared from 3-cyano quinolines by hydrolysis under basic conditions to furnish 3-quinolinecarboxylic acid derivatives which are treated with SOCl₂ followed by addition of various amines (aromatic and aliphatic amines, aminoalcohols, aminoesters) to give rise to the desired 3-carboxamidoquinolines. The deprotection of the methoxy group is achieved under mild conditions with BBr₃ in CH₂Cl₂. The carbamoylation, thiocarbamoylation, quaternization and reduction steps are performed as described above.

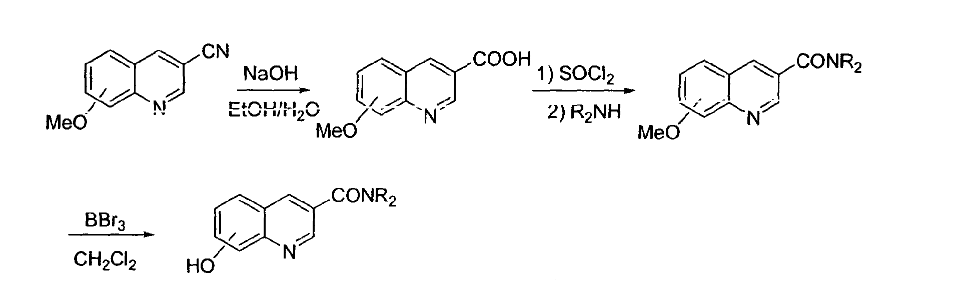

The 3-oxazolylquinolines are obtained in a one step by reaction of the cyano derivatives and an appropriate aminoalcohol. The reaction is carried out in refluxing chlorobenzene in the presence of ZnCl₂. Deprotection of the methoxy group is performed in the presence of BBr₃ in dichloromethane. The carbamoylation, thiocarbamoylation quatemisation and reductions steps are conducted as reported above.

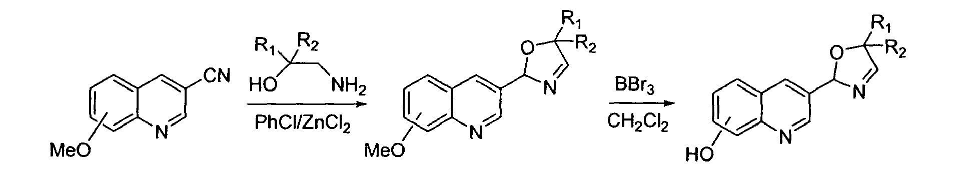

The 3-trifluoromethylquinoline derivatives are prepared in a one step by treatment of the carboxylic acids with Deoxo-Fluor. The *O*-demethylation, carbamoylation, thiocarbamoylation, quaternisation and reduction steps are conducted by the methods reported above.

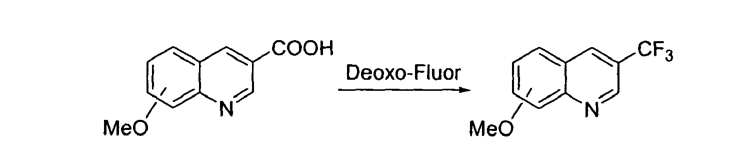

The 3-sulfinyl-substituted quinolines are prepared from the corresponding 3-bromoquinolines by lithium-bromine exchange using n-butyllithium followed by treatment of the resulting lithiated species with a suitable sulfinic ester. The 3-bromoquinolines were prepared *via* a Curtius rearrangement of the 3-carboxylic acids in the presence of diphenylphosphorazide (DPPA) in refluxing *t*-butanol. The resultant N-carbamates are diazotated with HBr-NaNO₂ followed by addition of molecular bromine. The *O*-demethylation, carbamoylation, thiocarbamoylation, quaternization and reductions steps are conducted as reported above.

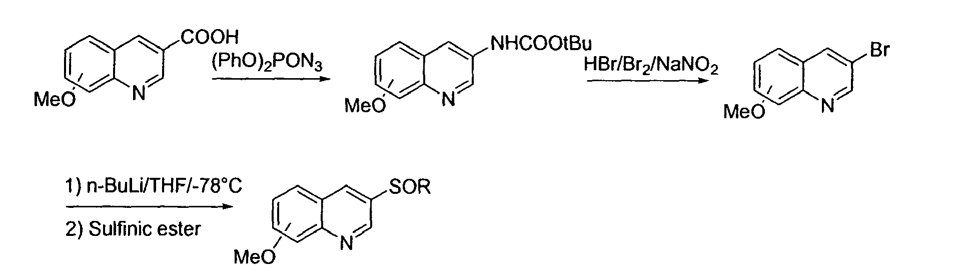

The synthesis of 3-alkylsulfonylquinolines is achieved by oxidation of the 3-sulfinylquinolines previously prepared using m-CPBA in dichloromethane. The *O-*demethylation, carbamoylation, thiocarbamoylation, quaternization and reductions steps are conducted as reported above.

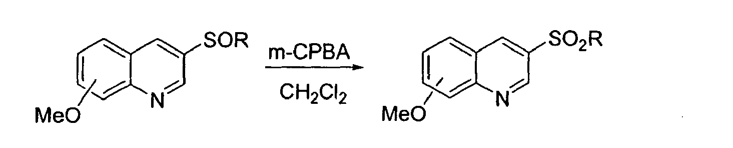

The preparation of 3-sulfonamidoquinolines is performed from the 3-(alkylsulfinyl)quinolines by Pummerer rearrangement with TFAA providing the corresponding thiol after treatment with NEt₃ in MeOH. The resultant thiols derivatives are treated with chlorine in AcOH to give the sulfonyl chloride derivatives which are subsequently reacted with different amines to obtain the required 3-sulfonamides-substituted quinolines. The *O*-demethylation, carbamoylation, thiocarbamoylation, quaternization and reductions steps are conducted as reported above.

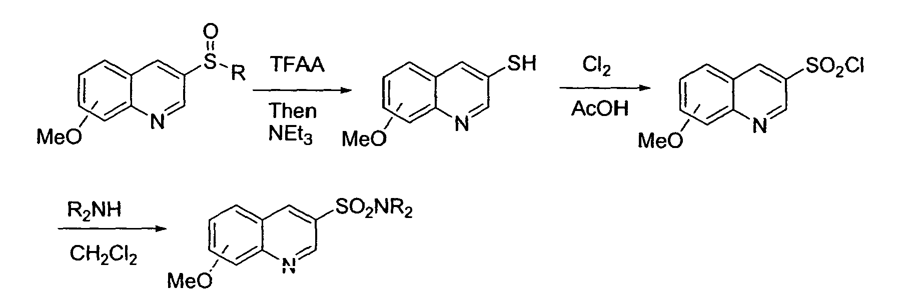

The synthesis of 3-nitroquinolines is achieved from the sodium salt of nitromalonaldehyde and various anilines as described in Journal of Medicinal Chemistry, 1994, 37, 2129-2137. The *O*-demethylation, carbamoylation, thiocarbamoylation, quaternization and reductions steps are conducted as reported above.

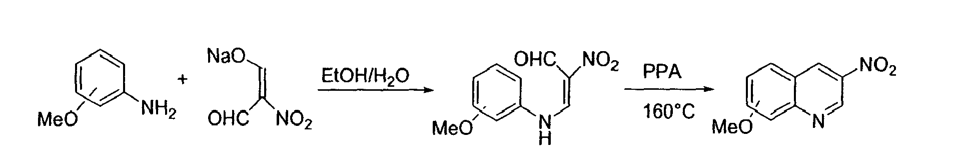

The desired 2-and/or-4-substituted quinolines are prepared by Friedländer or related methods (Organic Reaction, vol 28, 1982, page 37-201) by reaction of methoxy *ortho*-aminoacetophenones or methoxy *ortho*-aminobenzaldehydes with appropriate ketones. The reaction is conducted in various solvent (THF, EtOH, H₂O) under basic (KOH, NaOH, K₂CO₃, piperidine) or acidic condition (AcOH, p-TSA, HCl) The *O*-demethylation, carbamoylation, thiocarbamoylation, quaternization and reductions steps are conducted as reported above.

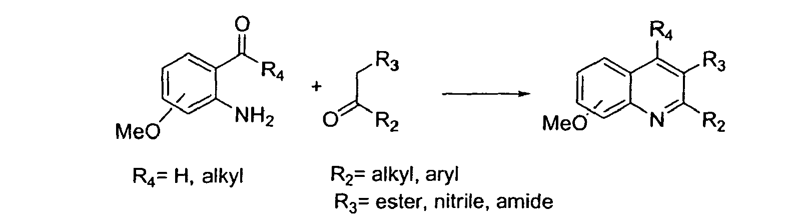

The required acetophenones and benzaldehydes used in this Friedländer approach are described in the following references. This list does not limit the scope of these acetophenones and benzaldehydes which can be used.
2-Amino-6-methoxyacetophenone (Eur. J. Org. Chem. (2001), 3247-3253);
2-Amino-5-methoxyacetophenone (Journal of Medicinal Chemistry (1987), 30(8), 1421-6);
2-Amino-4-methoxyacetophenone (Journal of Medicinal Chemistry (1989), 32(4), 807-26);
2-Amino-3-methoxyacetophenone (Journal of the Chemical Society, Abstracts (1945), 646-57);
2-Amino-4,6-dimethoxyacetophenone (Heterocycles, 2002, 57(1), 123-128);
2-Amino-4,5-dimethoxyacetophenone (Journal of Organic Chemistry (1954), 19 1117-23);
2-Amino-3,6-dimethoxyacetophenone (Journal of Medicinal Chemistry (1987), 30(8), 1421-6);
2-Amino-6-methoxybenzaldehyde (Journal of Medicinal Chemistry, 1993, 2689-22700);
2-Amino-4,5-dimethoxybenzaldehyde Tetrahedron 2001, 57, 3087-3098);
2-Amino-5-methoxybenzaldehyde (Tetrahedron Letters 2001, 42, 6589-6592);

Alternatively, the 4-substituted quinolines can be prepared from the 3-carboxamide or 3-oxazolyl or 3-cyanoquinolines previously mentioned by regioselective addition of an alkyllithium, a Grignard reagent or a cuprate affording the 1,4-dihydroquinolines. Oxidation of these intermediates by chloranil furnishes the desired 4-substituted quinolines. The *O*-demethylation, carbamoylation, thiocarbamoylation, quaternization and reductions steps are conducted as reported above.

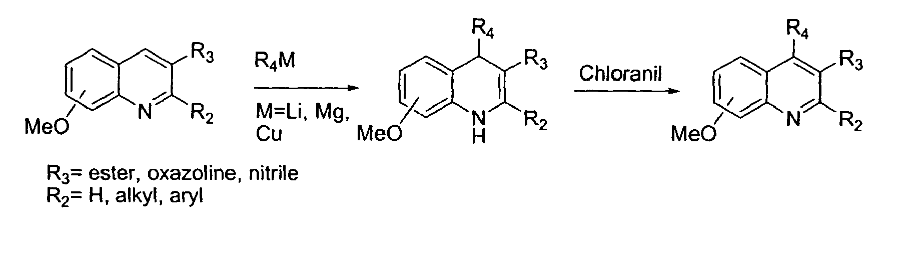

In another embodiment of the compound of formula G or G⁺ of the present invention, R₁ is an aromatic ring bearing a carbamate function linked to the nitrogen atom.

The process for the preparation of these compounds comprises a step of quaternarization of a compound of the formula E1, E2 or E3, with an alkylating agent bearing a carbamate function.

The synthesis of an alkylating agent bearing a carbamate function, such as the following carbamates wherein an aromatic ring bearing a carbamate function is described in the literature (Chemical papers, 1985, 39, 413-427).
Carbamic acid dimethyl-2-(chloromethyl)phenyl ester, carbamic acid dimethyl-3-(chloromethyl)phenyl ester, carbamic acid dimethyl-4-(chloromethyl)phenyl ester.

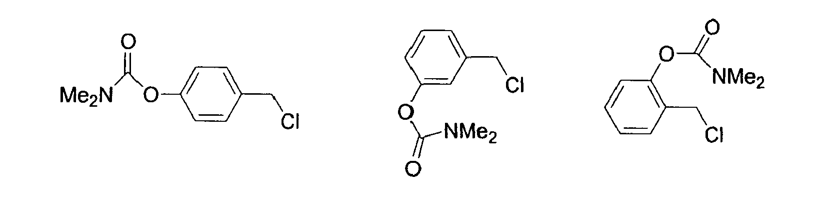

Other carbamates are prepared by reaction of the carbonochloridic acid-2-(chloromethyl)phenyl ester, carbonochloridic acid-3-(chloromethyl)phenyl ester and carbonochloridic acid-4-(chloromethyl)phenyl ester with various amines.

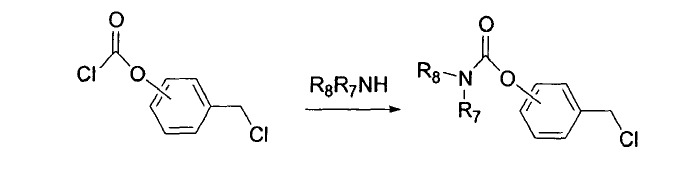

The quaternization of pyridines E1, isoquinolines E2 and quinolines E3 with the previous carbamates affords respectively the desired pyridinium G1+, isoquinoliniun G2+ and quinolinium G3+ salts. The desired dihydropyridines, dihydroquinolines and dihydroisoquinolines corresponding respectively to the formula G1a, G1b, G2, G3a, G3b are obtained by reduction as previously mentioned.

In another embodiment of the compound of the present invention, an aromatic ring bearing a carbamate function as described above is connected to the aromatic ring of formula G.

The preparation of this class of compounds makes use of a cross-coupling reaction between a heterocyclic aryl halide substrate (pyridine, quinoline, isoquinoline) and an aryl boronic acid substituted by an alkoxy group. The cross-coupling reaction takes place in the presence of palladium catalyst and a base (Na₂CO₃, K₂CO₃, Cs₂CO₃...) in DMF/H₂O. The reaction mixture is refluxed at 50-80°C for 12 hours.

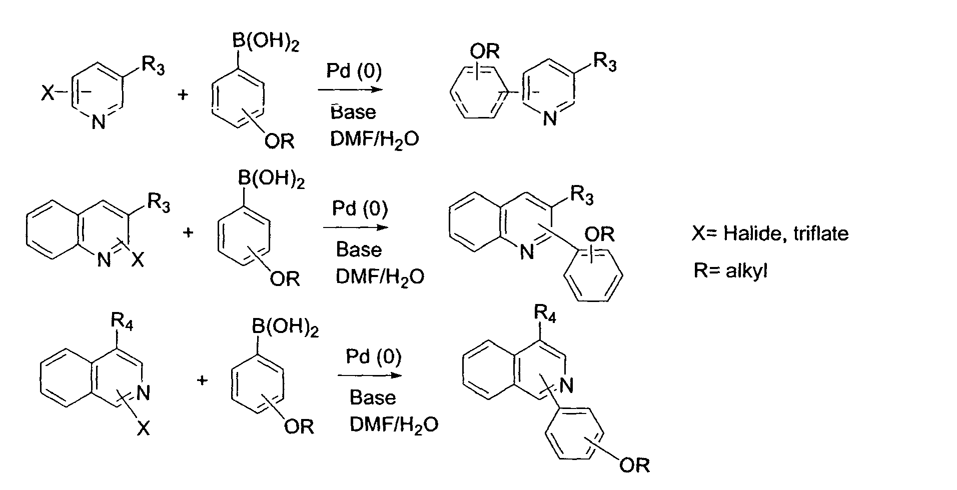

The *O*-demethylation, carbamoylation, thiocarbamoylation, quaternization and reduction steps are conducted as reported above.

The preparation of the following heterocyclic aryl halide is reported in the literature.
2-Iodo-3-pyridinecarbonitrile (Journal of Organic Chemistry (2002), 67(26), 9276-9287);
4-Iodo-3-pyridinecarbonitrile (Journal of Organic Chemistry (2002), 67(26), 9276-9287);
5-Chloro-3-pyridinecarbonitrile (Journal of Organic Chemistry (1974), 39(13), 1802-7);
6-Bromo-3-pyridinecarbonitrile (Journal of Organic Chemistry, 2001, 66, 1500-1502);
2-Chloro-3-quinolinecarbonitrile (Tetrahedron 2001, 57, 3087-3098);
Ethyl 5-chloroquinoline-3-carboxylate (Tetrahedron Letters 2001, 42, 3737-3740);
Ethyl 6-bromoquinoline-3-carboxylate (Tetrahedron Letters 2002, 43, 6209-6211) ;
Ethyl 7-chloroquinoline-3-carboxylate (Tetrahedron Letters 2002, 43, 6209-6211) ;
Ethyl 8-bromoquinoline-3-carboxylate (NO Patent WO 2001047891);
1-Bromoisoquinoline (Journal of Medicinal Chemistry 2002, 45, 740-743);
Methyl 1-chloroisoquinoline-4-carboxylate (Indian Journal of Chemistry (1972), 10(4), 341-3);
3-Bromoisoquinoline (Synthesis, 1987, 8, 693-6);
4-Bromoisoquinoline (Chemical & Pharmaceutical Bulletin (1997), 45(5), 928-931;
5-Bromoisoquinoline (Journal of Medicinal Chemistry (2002), 45(17), 3660-3668);
6-Bromoisoquinoline (Bioorganic & Medicinal Chemistry Letters (2002), 12(5), 827-832);
7-Bromoisoquinoline (Bioorganic & Medicinal Chemistry Letters (2002), 12(15), 2043-2046;
8-Bromoisoquinoline (ARKNOC [online computer file] (2000), 1(5), 823-842) ;
The preparation of the following arylboronic acids is reported in the literature;
2-Methoxyphenylboronic acid (New Journal of Chemistry (2002), 26(4), 373-375);
3-Methoxyphenylboronic acid (Organic Letters (2004), 6(21), 3711-3714);
4-Methoxyphenylboronic acid (Bioorganic & Medicinal Chemistry (2004), 12(10), 2553-2570);
4-Methoxy-1-naphthenyl boronate (Journal of the American Chemical Society (2000), 122(48), 12051-12052);
3-Methoxy-1-naphthylboronic acid (Journal of Organic Chemistry (1999), 64(26), 9430-9443);
2-(Methoxyphenyl)-1-naphthylboronic acid (Journal of Organic Chemistry (1999), 64(26), 9430-9443);
2-Methoxy-1-naphthylboronic acid (Organic Process Research & Development (2003), 7(3), 379-384);
1-Methoxy-2-naphthylboronic acid (Tetrahedron Letters (1999), 40(43), 9005-9007);
3-Methoxy-2-naphthylboronic acid (Tetrahedron Letters (1999), 40(43), 7599-7603);
6-Methoxy-2-naphthylboronic acid (Journal of Organic Chemistry (2004), 69(6), 2024-2032);

The products of general formula G have a very good in vitro activity as selective inhibitor of acetylcholinesterase.

The enzymatic inhibition was measured after ten minutes incubation with the products of general formula G at different concentrations. An IC₅₀ was determined and expressed by comparison with that of Donepezil (Donepezil is our reference). Inhibition of AChE was determined by Ellman's spectrophotometric method using acetylthiocholine (ACThCh) (Ellman, G.L., Courtney, K.KD., Andres, V., and Featherstone, R.M. A new and rapid colorimetric determination of acetylcholinesterase activity Biochem. Pharmacol. 1961, (7), 88-95).

These properties make said products as well as their salts with pharmaceutically acceptable acids and bases suitable for use as drugs in the treatment of diseases related to neurodegenerative diseases such as Alzheimer's disease, myasteny disease, light and early dementia...

Therefore an object of the present invention is, as drugs and in particular anti-Alzheimer drugs, the products of formula G as defined above as well as their salts with pharmaceutically acceptable acids and bases.

An object of the invention is also the pharmaceutical compositions containing as active ingredient in a safe an effective amount, at least one of the compounds according to the invention as defined above.

These compositions can be administered by buccal, rectal, parenteral, in particular intramuscular route or by local route as a topical application on the skin and the mucous membranes.

The compositions according to the invention can be solids or liquids and be presented in the pharmaceutical forms commonly used in human medicine, such as for example, plain or sugar-coated tablets, gelatin capsules, granules, suppositories, injectable preparations, ointments, creams, gels; they are prepared according to the usual methods. The active ingredient(s) can be incorporated with the excipients usually used in these pharmaceutical compositions, such as talc, arabic gum, lactose, starch, magnesium stearate, cocoa butter, aqueous or non aqueous vehicles, fatty substances of animal or vegetable origin, paraffin derivatives, glycols, various wetting, dispersing or emulsifying agents, preservatives.

These compositions can in particular be presented in the form of a powder intended to be dissolved extemporaneously in an appropriate vehicle, for example apyrogenic sterile water.

The dose administered is variable according to the condition treated, the patient in question, the administration route and the product considered. A safe an effective amount can be, for example, comprised between 0.041 g and 1 g per day by oral route in adults with the product described in Example 1 or also comprised between 0.25 g and 10 g per day by intramuscular or intravenous route.

Another object of the invention is a pharmaceutical composition comprising a compound of formula G+, G1+, G2+ or G3+ as described above for its use as a acetylcholinesterase inhibitor in the PNS.

Such a pharmaceutical composition is unable to cross the BBB but can reach the PNS through the blood stream. The lack of undesirable central effects (confusion, hypothermia...), as well as the selective inhibition of the peripheral acetylcholinesterase enzyme compared to butyrylcholinesterase, make such a pharmaceutical composition a good candidate for the treatment of myasteny disease in a human or other animal subject.

The compounds of the present invention have been prepared according to the procedures described below in the examples section.

The distance d of the compounds of the invention has been measured and are reported in table I below.

**Table 1**

| **Exemple n°** | Distance d nm | **Exemple n°** | Distance d nm |
|---|---|---|---|
| **1** | 5.9 | **35** | 0.46 |
| **2** | 6.1 | **36** | 0.41 |
| **3** | 5.87 (C5) -6.2 (C7) | **37** | 0.41 |
| **4** | 6.1 4.1 | **38** | 0.41 |
| **5** | 6.1 | **39** | 0.41 |
| **6** | 6.1 | **40** | 0.41 |
| **7** | 6.1 | **41** | 0.41 |
| **8** | 5.8 | **42** | 0.41 |
| **9** | 6.1 | **43** | 0.41 |
| **10** | 6.05 | **44** | 0.41 |
| **11** | 6.1 | **45** | 0.41 |
| **12** | 6.1 | **46** | 0.41 |
| **13** | 6.6 (C6) -5.4 (C7) | **47** | 0.41 |
| **14** | 6.6 (C6)- 5.4 (C7) | **48** | 0.41 |
| **15** | 5.6 | **49** | 0.41 |
| **16** | 7.1 | **50** | 0.41 |
| **17** | 7.1 | **51** | 0.38 |
| **18** | 7.1 | **52** | 0.38 |
| **19** | 7.1 | **53** | 0.3.8 |
| **20** | 7.05 (C6) -4.6 (C8) | **54** | 0.38 |
| **21** | 7.05 (C6) - 4.6 (C8) | **55** | 0.38 |
| **22** | 7.05 (C6) -4.6 (C8) | **56** | 0.56 |
| **23** | 7.05 (C6) -4.6 (C8) | **57** | 0.39 |
| **24** | 7.05 (C6) -4.6 (C8) | **58** | 0.61 |
| **25** | 7.05 (C6) -4.6 (C8) | **59** | 0.43 |
| **26** | 7.05 (C6) -4.6 (C8) | **60** | 0.56 |
| **27** | 4.6 | **61** | 0.52 |
| **28** | 4.6 | **62** | 0.64 |
| **29** | 4.6 | **63** | 0.52 |
| **30** | 4.6 | **64** | 0.47 |
| **31** | 0.46 | **65** | 0.47 |
| **32** | 0.46 | **66** | 0.47 |
| **33** | 0.46 | | |
| **34** | 0.46 | | |

### Examples.

### Example 1 Preparation of ethyl 1-methyl-7-(N,N-dimethylcarbamate)-1,4-dihydroquinoline-3-carboxylate.

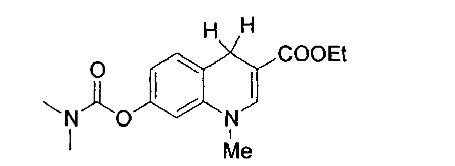

Stage A: 7-hydroxy 3-quinolinecarboxylic acid.
   A solution of 3-cyano-7-methoxyquinoline (20g, 110 mmol) [described in Tetrahedron Letters, 39 (23), 4013-4016, 1998] in 48% aqueous solution bromhydric acid (300 ml) is stirred and heated under reflux for 12 hours. The reaction mixture was then cooled to room temperature and neutralised by adding 20% aqueous KOH. The resulting precipitate was filtrated and dried under vacuum.
   In this way 15.2 g of the product of molecular formula C₁₀H₇NO₃ (MW =189.2) is recovered. The corresponding yield is 73%.
Stage B: ethyl 7-hydroxyquinoline-3-carboxylate
   To a solution of the compound obtained in stage A (15 g, 79 mmol) in EtOH (500 ml) was added dropwise SOCl₂ (40 mL). The resulting mixture was stirred under reflux during 12 hours. After adding water (200 mL), the pH was adjusted to 7.0 with 20% aqueous Na₂CO₃. The aqueous solution was extracted with CH₂Cl₂ (mL).The combined organic layers were evaporated and dried (MgSO₄) to afford 11g the product of molecular formula C₁₂H₁₁NO₃
Stage C: ethyl 7-(*N,N*-dimethylcarbamate)quinoline-3-carboxylate
   To a solution of compound obtained in stage C (300 mg, 1.38 mmol) in dry THF was added sodium hydride (as an suspension in oil, 72 mg, 1.5 mmol). The mixture was stirred at room temperature for 1 hour after which time dimethylcarbamoyl chloride (140 mL, 1.5 mmol) was added. The resulting mixture was refluxed for 12 hours. After Addition of water (10 mL) and extraction with CH₂Cl₂ (3 x 15 mL), the resulting combined organic phases were dried and evaporated under vacuum to give 360 mg of compound of molecular formula C₁₅H₁₆N₂O₄ in 90% yield.
Stage D: ethyl 1-methyl-7-(*N,N*-dimethylcarbamate)quinolinium-3-carboxylate triflate
   To a solution of compound obtained in stage C (300 mg, 1.0 mmol) in dry CH₂Cl₂ (25 mL) was added methyl triflate (130 µL, 1.1 mmol). The resulting solution was stirred at room temperature for 2h. Addition of Et₂O(10 mL) furnished a white precipitate which was filtered to give the desired quinolinium salt of molecular formula C₁₇H₁₉F₃N₂O₇S in a quantitative yield. Aspect: white powder.
   NMR spectrum of the proton
   In CDCl₃, at 300MMz, Chemicals shifts (ppm) and multiplicity: 9.78 (s, 1H, H₄), 9.44 (s, 1H, H₂), 8.35 (d, *J*= 9Hz, 1H, H₆), 8.28 (s, 1H, H₈), 7.87 (d, *J*= 9Hz, 1H, H₅), 4.74 (s, 3H, N-Me), 4.54 (q, 2H, CH₂-CH₃), 3.19 (s, 3H, N-methyl carbamate), 3.07 (s, 3H, N-methyl carbamate), 1.47 (t, 3H, CH₂-CH₃).
Stage E: ethyl N-methyl-7-(*N*,*N*-dimethylcarbamate-1,4-dihydroquinoline-3-carboxylate
   To a solution of the compound prepared in stage D (100 mg, 0.22 mmol) in water (6 ml) and CH₂Cl₂ (6 mL), were added in one portion sodium dithionite (190 mg, 1.1 mmol) and sodium carbonate (70 mg, 0.66 mmol). After stirring for 1 hour under a nitrogen atmosphere, Na₂S₂O₄ (190 mg, 1.1 mmol) and Na₂CO₃ (70 mg, 0.66 mmol) were added. After stirring for 1 hour, Na₂S₂O₄ (190 mg, 1.1 mmol) and Na₂CO₃ (70 mg, 0.66 mmol) were added and stirring was continued for an additional 1 hour. The aqueous phase was extracted with CH₂Cl₂ (3 x 15 ml), the combined organic layers were dried over Na₂SO₄ and evaporated under vacuum to give the compound of the molecular formula C₁₆H₂₀N₂O₄ in 91% yield. Aspect: yellow oil.
   NMR spectrum of the proton
   In CDCl₃, at 300MMz, Chemicals shifts (ppm) and multiplicity: 7.19 (s, 1H, H₂), 7.00 (d, J= 8.5 Hz, 1H, H₅), 6.67 (dd, *J*= 9Hz and 3 Hz, 1H, H₆), 6.49 (d, *J*= 3 HZ,
   1H, H₈), 4.17 (q, 2H, CH₂-CH₃), 3.73 (s, 2H, H₄), 3.18 (s, 3H, N-Me), 3.08 (s, 3H, N-methylcarbamate), 3.00 (s, 3H, N-methylcarbamate), 1.28 (t, 3H, CH₂-CH₃).

### Example 2 Preparation of ethyl 1-methyl-5-(N,N-dimethylcarbamate)-1,4-dihydroquinoline-3-carboxylate.;

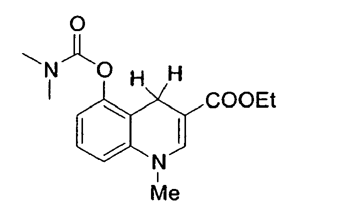

Stage A: ethyl 8-bromo-4-chloro-5-methoxyquinoline-3-carboxylate
   A solution of ethyl 8-bromo-4-hydroxy-5-methoxyquinoline-3-carboxylate (4.55 g, 14 mmol) [described in Journal of Medicinal Chemistry (1995), 38(6), 950-7] in POCl₃ (2.11g, 24 mmol) is heated on a steam bath for 15 min. The mixture was cooled on an ice bath and 90 mL of diluted aqueous ammonium hydroxide was added. The aqueous layer was extracted with ether and the combined organic phases were washed with water, dried over MgSO₄ and evaporated under vacuum.
Stage B: ethyl-5-methoxyquinoline-3-carboxylate.
   A solution of ethyl 8-bromo-4-chloro-5-methoxy-quinoline-3-carboxylate (4.1 g, 12 mmol)obtained in stage B and sodium acetate (1g, 12 mmol) in 120 mL of glacial acetic acid was hydrogenated in a Parr bomb at 2.5 atm pressure over a 10% Pd/C catalyst (0.85g) for 8 hours. The catalyst was removed by filtration and the reaction mixture was concentrated under reduced pressure. The residue was dissolved in methylene chloride (200 mL). The solution was washed with aqueous sodium bicarbonate solution (15%, 100 mL) and then with water, dried (MgSO₄) and filtered. Stage C: 5-hydroxy-3-carboxylic acid.
   The title compound is prepared as described in stage A of Example 1.
Stage D: ethyl 5-hydroxyquinoline-3-carboxylate
   The title compound is prepared as described in stage B of Example 1.
Stage E: preparation of ethyl 5-(*N,N*-dimethylcarbamate)quinoline-3-carboxylate
   The title compound is prepared as described in stage C of Example 1.
Stage F: preparation of ethyl 1-methyl-5-(*N,N*-dimethylcarbamate)quinolinium-3-carboxylate triflate
   The title compound is prepared as described in stage D of Example 1.
Stage G: preparation of ethyl 1-methyl-5-(*N,N*-dimethylcarbamate)-1,4-dihydroquinoline-3-carboxylate.
   The title compound is prepared as described in stage E of Example 1.

### Example 3 Preparation of ethyl 1-methyl-5,7-di(N,N-dimethylcarbamate)-1,4-dihydroquinoline-3-carboxylate

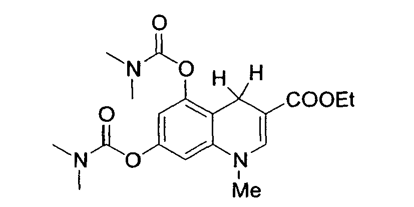

Stage A: 5,7-dihydroxy-3-quinolinecarboxylic acid
   A solution of 3-cyano-5,7-methoxyquinoline (20g, 110 mmol) [described in Tetrahedron Letters, 39 (23), 4013-4016, 1998] was treated as reported in stage A of Example 1. The compound of the molecular formula C₁₀H₇NO₄ (MW = 205.17) is obtained in 80% yield.
Stage B: ethyl 5,7-dihydroxyquinoline-3-carboxylate.
   The title compound is prepared as described in Stage B of Example 1
Stage C: ethyl 5,7-di(*N,N*-dimethylcarbamate)quinoline-3-carboxylate. The title compound is prepared as described in Stage C of Example 1.
Stage D: ethyl 1-methyl-5,7-di(*N,N*-dimethylcarbamate)quinolinium-3-carboxylate triflate
   The title compound is prepared as described in Stage D of Example 1.
Stage E: ethyl 1-methyl-5,7-di(*N,N*-dimethylcarbamate-1,4-dihydroquinoline-3-carboxylate.
   The title compound is synthesized as described in Stage E of Example 1.

### Example 4 Preparation of ethyl 1-methyl-5,8-di(N,N-dimethylcarbamate)-1,4-dihydroquinoline-3-carboxylate

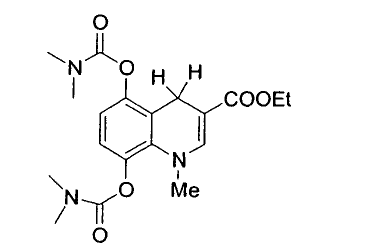

Stage A: 5,8-dihydroxy-3-quinolinecarboxylic acid
   A solution of 3-cyano-5,8-methoxyquinoline (20g, 110 mmol) [described in Tetrahedron Letters, 39 (23), 4013-4016, 1998] was treated as reported in stage A of Example 1. The compound of the molecular formula C₁₀H₇NO₄ (MW = 205.17) is obtained in 80% yield.
Stage B: ethyl 5,8-dihydroxyquinoline-3-carboxylate.
   The title compound is synthesized as described in Stage B of Example 1
Stage C: ethyl 5,8-di(*N,N*-dimethylcarbamate)quinoline-3-carboxylate.
   The title compound is prepared as described in Stage C of Example 1.
Stage D: ethyl 1-methyl-5,8-di(*N,N*-dimethylcarbamate)quinolinium-3-carboxylate triflate
   The title compound is prepared as described in Stage D of Example 1.
Stage E: preparation of ethyl 1-methyl-5,8-di(*N,N*-dimethylcarbamate-1,4-dihydroquinoline-3-carboxylate.
   The title compound is synthesized as described in Stage E of Example 1.

### Example 5 Preparation of ethyl 1-methyl-1,4-dihydro-5-O-quinoline-N,N-dimethylthiocarbamate-3-carboxylate

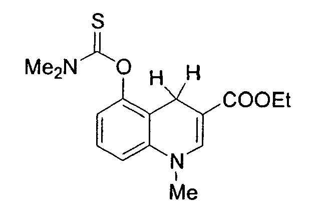

Stage A: ethyl *N,N*-(dimethylthiocarbamate)-5-*O*-quinoline-3-carboxylate
   A solution of ethyl 5-hydroxyquinoline-3-carboxylate prepared in stage D of example 2 (21.7g, 10 mmol) in DMF (40 ml) was cooled to 0°-5°C and treated with 60% sodium hydride (300 mg, 12.5 mmol) and stirred 15 min. Dimethylthiocarbamoyl chloride (1.23g, 10 mmol) was then added and the solution was stirred for 10 min at 5°C and 30 min at room temperature then at 60°C for 1 hour. The solution was cooled, diluted with IN-sodium hydroxide solution and extracted with with AcOEt (3x25mL). The combined organic layers were washed with IN-sodium hydroxide, brine, water, then brine, and dried over Magnesium sulfate and evaporated under reduced pressure. The obtained residue was chromatographed over silica-gel.
Stage B: ethyl 1-methyl-5-*O*-quinoliniumdimethylthiocarbamate-3-carboxylate triflate.
   The title compound is synthesized as described in stage D of Example 1.
Stage C: ethyl 1-methyl-1,4-dihydro-5-*O*-quinolinedimethylthiocarbamate-3-carboxylate
   The title compound is prepared as described in stage E of Example 1.

### Example 6 Preparation of ethyl 1-methyl-1,4-dihydro-5-S-quinoline-N,N-dimethylthiocarbamate-3-carboxylate;

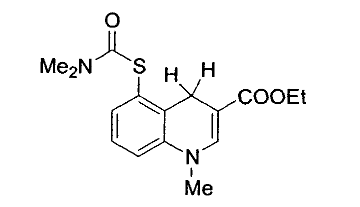

Stage A: ethyl (*N,N*-dimethylthiocarbamate)-5-*S*-quinoline-3-carboxylate ethyl (*N,N*-dimethylthiocarbamate)-5-*O*-quinoline-3-carboxylate prepared in stage A of example 5 was placed on a preheated oil bath at 210°C and heated for 5 hours and cooled to room temperature. The solid residue was chromatographed on silica-gel.
Stage B: ethyl 1-methyl-(*N,N*-dimethylthiocarbamate)-5-*S*-quinolinium-3-carboxylate triflate.
   The title compound is prepared as described in stage D of Example 1.
Stage C: ethyl 1-methyl-(*N*,*N*-dimethylthiocarbamate)-1,4-dihydro-5-*S*-quinoline-3-carboxylate
   The title compound is synthesized as described in stage E of Example 1.

### Exemple 7 Preparation of 1-methyl-5-(N,N-dimethyicarbamate)-3-(N,N-diethylcarboxamido)-1,4-dihydroquinoline;

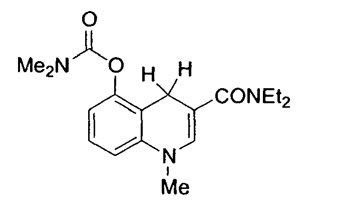

Stage A: ethyl 5-benzyloxyquinoline-3-carboxylate
   A solution of ethyl 5-hydroxyquinoline-3-carboxylate (2.17 g, 10 mmol) prepared in stage D of example 2, Na₂CO₃ (2.12 g, 20 mmol) and benzylbromide (1.71 g, 10 mmol) in DMF (20 mL) was stirred for 12 hours at room temperature. The reaction mixture was diluted with water (50 mL) and then extracted with with AcOEt (3x25mL). The combined organic layers were washed water, and dried over Magnesium sulfate and evaporated under reduced pressure. The obtained residue was chromatographed over silica-gel.
Stage B: 5-benzyloxy-3-quinolinecarboxylic acid.
   A solution of ethyl 5-benzyloxyquinoline-3-carboxylate (3.07 g, 10 mmol) prepared in stage A and LiOH(0.4 g, 20 mmol) in THF (20 mL)and water (0.5 mL) was refluxed for 2 hours. After cooling, the solvents were evaporated under vacuum. The solid residue was used in the next step without further purification.
Stage C: 5-benzyloxy-3-(*N,N*-diethylcarboxamido)quinoline
   A solution of 5-benzyloxy-3-quinolinecarboxylic acid (2.80 g, 10 mmol) prepared in stage B, oxalyl chloride (1.2 g, 10 mmol) in dichloromethane (50 mL) was added 2 drops of DMF. The resultant mixture was stirred for 2 hours at room temperature after which time diethylamine (2.19 g, 30 mmol) was added. The resulting solution was stirred for a further 3 hours. The reaction mixture was diluted with water (50 mL) and then extracted with with AcOEt (3x25mL). The combined organic layers were washed water, and dried over Magnesium sulfate and evaporated under reduced pressure. The obtained residue was chromatographed over silica-gel.
Stage D: 5-hydroxy-3-(*N,N*-diethylcarboxamido)quinoline.
   A solution of 5-benzyloxy-3-(*N*,*N*-diethylcarboxamido)quinoline (3.34 g, 10 mmol) prepared in stage C in methanol (50 mL) was added 10% Pd/C (400 mg). The solution was stirred for 24 hours at room temperature under a hydrogen atmosphere. The catalyst was filtered through a plug of cotton and washed with methanol. Evaporation of methanol afforded the desired compound.
Stage E: 5-(*N,N*-dimethylcarbamate)-3-(*N,N*-diethylcarboxamido)quinoline.
   The title compound is prepared according to the procedure described in stage C of example 1.
Stage F: 1-methyl-5-(*N,N*-dimethylcarbamate)-3-(*N,N*-ethylcarboxamido)quinolium triflate.
   The title compound is synthesized as described in stage D of exemple 1
Stage G: 1-methyl-5-(*N,N*-dimethylcarbamate)-3-(*N,N*-diethylcarboxamido)-1,4-dihydroquinoline.
The title compound is prepared as described in stage E of exemple 1.

### Example 8 : preparation of 1-methyl-7-(N,N-dimethylcarbamate)-3-(4,5-dihydro-4,4-dimethyl-2-oxazolyl)-1,4-dihydroquinoline;

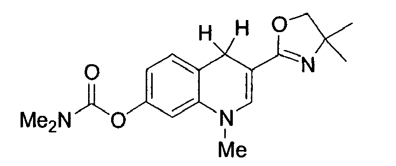

Stage A: 7-methoxy-3-(4,5-dihydro-4,4-dimethyl-2-oxazolyl)quinoline
   A solution of 7-methoxy-3-cyanoquinoline reported in Tetrahedron Letters 39, 1998, 4013-4016 (1.84 g, 10 mmol) and 2-amino-2-methyl-1-propanol (0.90 g, 10 mmol) in chlorobenzene under nitrogen atmosphere is refluxed for 2 days. The solvent is evaporated under vacuum to afford the desired compound
Stage B: 7-hydroxy-3-(4,5-dihydro-4,4-dimethyl-2-oxazolyl)quinoline
   A solution of 7-methoxy-3-(4,5-dihydro-4,4-dimethyl-2-oxazolyl)quinoline (2.56 g, 10 mmol) prepared in stage A and BBr₃ (7.41 g, 30 mmol) in dichloromethane (40 mL) are stirred for 12 hours at room temperature. The mixture was quenched with saturated sodium hydrogen carbonate. Extraction with dichloromethane, drying (MgSO₄) and evaporation furnished the desired compound.
Stage C: 7-(*N,N*-dimethylcarbamate)-3-(4,5-dihydro-4,4-dimethyl-2-oxazolyl)quinoline
   The title compound is synthesized according to the procedure reported in stage C of example 1 from 7-hydroxy-3-(4,5-dihydro-4,4-dimethyl-2-oxazolyl)quinoline prepared in stage B.
Stage D: 1-methyl-7-(*N,N*-dimethylcarbamate)-3-(4,5-dihydro-4,4-dimethyl-2-oxazolyl)quinolinium triflate
   The title compound is prepared according to the procedure reported in stage D of example 1 from 7-(*N,N*-dimethylcarbamate)-3-(4,5-dihydro-4,4-dimethyl-2-oxazolyl)quinoline prepared in stage C.
Stage E 1-methyl-7-(*N*,*N*-dimethylcarbamate)-3-(4,5-dihydro-4,4-dimethyl-2-oxazolyl)-1,4-dihydroquinoline
   The title compound is prepared according to the procedure reported in stage E of example 1 from 1-methyl-7-(*N*,*N*-dimethylcarbamate)-3-(4,5-dihydro-4,4-dimethyl-2-oxazolyl)quinolinium triflate prepared in stage D.

### Example 9 Preparation of 1-methyl-5-(N,N-dimethylcarbamate)-3-trifluoromethyl-1,4-dihydroquinoline;

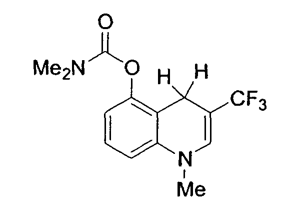

stage A: 5-methoxy-3-quinolinecarboxylic acid
   A solution of ethyl 5-methoxyquinoline-3-carboxylate (2.31 g, 10 mmol) prepared in stage B of example 2 and LiOH(0.4 g, 20 mmol) in THF (20 mL)and water (0.5 mL) was refluxed for 2 hours. After cooling, the solvents were evaporated under vacuum. The solid residue was used in the next step without further purification.
stage B: 5-methoxy-3-trifluoromethylquinoline according to a procedure reported in the Journal of Organic chemistry, 1999, 64, 7053.
   To a solution of 5-methoxy-3-quinolinecarboxylic acid (2 g, 10 mmol) prepared in stage A in dichloromethane was added bis(2-methoxyethyl)aminosulfur trifluoride (2.43 g, 11 mmol) under nitrogen and stirred for 16 hours at room temperature. The solution was poured into saturated NaHCO₃ and after CO₂ evolution ceased it was extracted with dichloromethane, dried, filtered and evaporated in vacuo. The resulting acyl fluoride intermediate (10 mmol) was added bis(2-methoxyethyl)aminosulfur trifluoride (4.42 g, 20 mmol)contained in a Teflon bottle equipped with a nitrogen inlet tube, and the mixture was heated at 85°C On completion, the solution was poured into saturated NaHCO₃ and after CO₂ evolution ceased it was extracted with dichloromethane, dried, filtered and evaporated in vacuo to give 5-methoxy-3-trifluoromethyl quinoline
Stage C: 5-hydroxy-3-trifluoromethylquinoline
   The title compound is prepared according to the procedure reported in stage B of example 8 from 5-methoxy-3-trifluoromethylquinoline prepared in stage B.
Stage D: 5-(*N,N*-dimethylcarbamate)-3-trifluoromethylquinoline
   The title compound is prepared according to the procedure reported in stage C of example 1 from 5-hydroxy-3-trifluoromethylquinoline synthesized in stage C.
Stage E: 1-methyl 5-(*N*,*N*-dimethylcarbamate)-3-trifluoromethylquinolinium triflate
   The title compound is prepared according to the procedure reported in stage D of example 1 from 5-(*N,N*-dimethylcarbamate)-3-trifluoromethyl quinoline prepared in stage D.
Stage F: 1-methyl 5-(*N,N*-dimethylcarbamate)-3-trifluoromethyl-1,4-dihydroquinoline
   The title compound is prepared according to the procedure reported in stage E of example 1 from 1-methyl-5-(*N,N*-dimethylcarbamate)-3-trifluoromethylquinolinium triflate prepared in stage E

### Example 10 Preparation of (+/-)-1-methyl-3-(4-methylphenylsulfinyl)-5-(N,N-dimethylcarbamate)-1,4-dihydroquinoline;

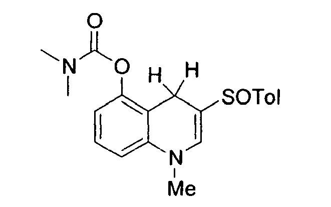

Stage A: 3-(*tert*-butoxycarbonylamino)-5-methoxyquinoline
   To a solution of 5-methoxy-3-quinolinecarboxylic acid (2 g, 10 mmol) prepared in stage A of example 9 in *tert*-BuOH (15 mL) was added NEt₃ (1.2 g, 12 mmol) and diphenyl azidophosphonate (DPPA) (2.1 mL, 10 mmol). The reaction mixture is refluxed for 24 hours. The solvent is evaporated under vacuum and the residue is dissolved in AcOEt (50 mL) and washed with a saturated NaHCO₃ solution. After drying, the solvent is evaporated under vacuum affording the title compound.
Stage B: 3-bromo-5-methoxyquinoline
   To a solution of 3-(tert-butoxycarbonylamino)-5-methoxyquinoline (2.74 g, 10 mmol) in aqueous 48% HBr (40 mL), NaNO₂ (2.1 g, 30 mmol) in water (20 mL) is added at 0°C. The reaction mixture is stirred 1 hour. The reaction mixture is extrated with CH₂Cl₂ (3 x 30 mL). After drying over MgSO₄, dichloromethane is evaporated affording the title compound.
Stage C: (+/-)-3-(4-methylphenylsulfinyl)-5-methoxyquinoline
   To a solution 3-bromo-5-methoxyquinoline (2.37, 10 mmol) prepared in stage B in dried THF (35 mL) is added a solution of isopropylmagnesium chloride (16.6 mL, 30 mmol) at -78°C. The solution is stirred at -78°C under nitrogen atmosphere for 3 hours. Menthyl sulfinate (8.8 g, 30 mmol) is added and the resulting solution is stirred for a further 24 hours. The reaction mixture is then hydrolysed with saturated aqueous NH₄Cl solution. After extraction of the aqueous phase, the combined organic layers are dried (MgSO4), evaporated giving the title compound.
Stage D: (+/-)-3-(4-methylphenylsulfinyl)-5-hydroxyquinoline
   The title compound is synthesized according to the procedure reported in stage B of Example 8 from (+/-)-3-(4-methylphenylsulfinyl)-5-methoxyquinoline prepared in stage C.
Stage E: (+/-)-3-(4-methylphenylsulfinyl-5-(*N,N*-dimethylcarbamate) quinoline
   The title compound is prepared according to the procedure reported in stage C of example 1 from (+/-)-3-(4-methylphenylsulfinyl-5-hydroxyquinoline prepared in stage D
Stage F: (+/-)-1-methyl-3-(4-methylphenylsulfinyl)-5-(*N,N-*dimethylcarbamate)quinolinium triflate
   The title compound is synthesized according to the procedure reported in stage D of example 1 from (+/-)-3-(4-methylpheny)sulfinyl)-5-(*N,N*-dimethylcarbamate) quinoline prepared in stage E.
Stage G: (+/-)-1-methyl-3-(4-methylphenylsulfinyl)-5-(*N,N*-dimethylcarbamate) 1,4-quinoline
   The title compound is prepared according to the procedure reported in stage E of example 1 from (+/-)-3-(4-methylphenylsulfinyl)-5-(*N,N-*dimethylcarbamate)quinolinium triflate prepared in stage F

### Example 11 Preparation of 1-methyl-3-(4-methylphenylsulfonyl)-5-(N,N-dimethylcarbamate)-1,4-dihydroquinoline;

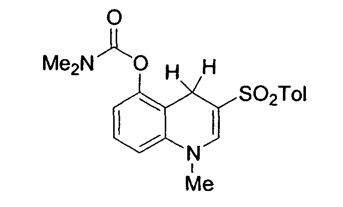

Stage A: 3-(4-methylphenylsulfonyl)-5-methoxyquinoline
   To a solution of (+/-)-3-(4-methylphenylsulfinyl)-5-methoxyquinoline (2.97 g, 10 mmol) prepared in stage B of example 10 in CH₂Cl₂ is added m-CPBA (2.13 g, 80%, 10 mmol). The solution is stirred at room temperature for 12 hours. A solution of 1M NaOH is added. After extraction of the aqueous phase with CH₂Cl₂, the combined organic layers are dried (MgSO₄) and the solvent evaporated giving the title compound.
Stage B: 3-(4-methylphenylsulfonyl)-5-hydroxyquinoline
   The title compound is synthesized according to the procedure reported in stage B of Example 9 from 3-(4-methylphenylsulfonyl)-5-methoxyquinoline prepared in stage A.
Stage C: 3-(4-methylphenylsulfonyl)-5-(*N,N*-dimethylcarbamate)quinoline
   The title compound is prepared according to the procedure reported in stage C of Example 1 from 3-(4-methylphenylsulfonyl)-5-hydroxyquinoline prepared in stage
Stage D: 1-methyl-3-(4-methylphenylsulfonyl)-5-(*N,N-*dimethylcarbamate)quinolinium triflate
   The title compound is prepared according to the procedure reported in stage D of example 1 from 3-(4-methylphenylsulfonyl)-5-(*N,N*-dimethylcarbamate)quinoline prepared in stage C
Stage E: 1-methyl-3-(4-methylphenylsulfonyl)-5-(*N,N*-dimethylcarbamate)-1,4-dihydroquinoline.
   The title compound is prepared according to the procedure reported in stage E of example 1 from 1-methyl-3-(4-methylphenylsulfonyl)5-(*N,N-*dimethylcarbamate)quinolinium triflate prepared in stage D.

### Example 12 Preparation of 1-methyl-5-(N,N-dimethylcarbamate)-3-(N-phenylsulfomanide)-1,4-dihydroquinoline;

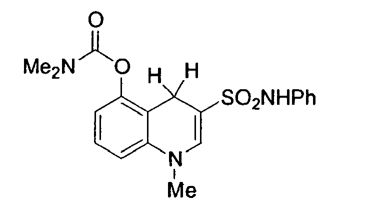

Stage A: 5-methoxy-3-methylthioquinoline
   To a solution of 3-bromo-5-methoxy quinoline (2,37 g, 10 mmol) prepared in stage B of example 10 in THF (25 mL) was added 2.5M n-buLi (4 mL, 10 mmol) at -78°C. The solution is stirred at -78°C for 45 min and dimethyl disulfide (1.8 g, 20 mmol) is added. The resultant solution is stirred for 3 hours and then quenched with saturated NH₄Cl (30 mL). Extraction with dichloromethane, drying, filtering and evaporation in vacuo afford the title compound
Stage B: 3-(methylsulfinyl)-5-methoxyquinoline.
   The title compound is prepared from 5-methoxy-3-methylthioquinoline (2.05 g, 10 mmol) prepared in stage A according the procedure reported in stage A of example 11.
Stage C: 5-methoxy-3-quinolinethiol.
   To a solution of 3-(methylsulfinyl)-5-methoxyquinoline (2.21 g, 10 mmol) in CH₂Cl₂ (50 mL) is added TFAA. The resulting solution is stirred at 20-50°C for 1 hour. The solution is then treated with NEt₃ affording the title compound.
Stage D: *N*-phenyl-5-methoxy-3-quinolinesulfomanide
   A solution of 5-methoxy-3-quinolinethiol (1.91, 10mmol) in AcOH is treated with chlorine for a few minutes. The solution is then treated with aniline (0.93 g, 10 mmol) to afford the title compound.
Stage E: *N*-phenyl-5-hydroxy-3-quinolinesulfomanide
   The title compound is synthesized according to the procedure reported in stage B of Example 8 from *N*-phenyl-5-methoxy-3-quinolinesulfomanide prepared in stage D.
Stage F: 3-(*N*-phenylsulfomanide)-5-(*N,N*-dimethyl carbamate)quinoline
   The title compound is prepared according to the procedure reported in stage C of Example 1 from *N*-phenyl-5-hydroxy-3-quinolinesulfonamide prepared in stage E.
Stage G: 1-methyl-3-(*N*-phenylsulfomanide)-5-(*N,N*-dimethylcarbamate)quinolinium triflate
   The title compound is prepared according to the procedure reported in stage D of Example 1 prepared from 3-(*N*-phenylsulfomanide)-5-(*N,N*-dimethyl carbamate)quinoline in stage F
Stage H: 1-methyl-5-(*N*,*N*-dimethylcarbamate)-3-(*N*-phenylsulfomanide)-1,4-dihydroquinoline
   The title compound is prepared according to the procedure reported in stage E of Example 1 prepared from 1-methyl-3-(*N*-phenylsulfomanide)-5-(*N*,*N*-dimethyl carbamate)quinolinium triflate in stage G.

### Exemple 13 Preparation of 1-methyl-6,7-di(N,N-dimethylcarbamate)-3-nitro-1,4-dihydroquinoline

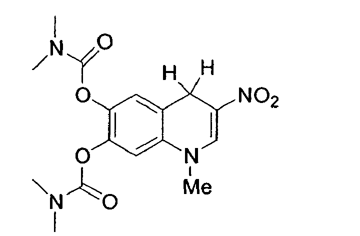

Stage A: 6,7-dihydroxy-3-nitroquinoline
   The title compound is prepared according to the procedure reported in stage B of example 8 from 6,7-dimethoxy-3-nitroquinoline prepared according to a procedure described in the Journal of Medicinal Chemistry, 1994, 37, 2129.
Stage B: 1-methyl 6,7-di(*N*,*N*-dimethyl carbamate)-3-nitro-1,4-dihydroquinoline
   The title compound is prepared from 6,7-dihydroxy-3-nitroquinoline prepared in stage A following the procedures in stage C, D and E of example 1.

### Example 14 Preparation of ethyl 1-methyl-2-phenyl-6,7-di(N,N-dimethylcarbamate)-1,4-dihydroquinoline-3-carboxylate

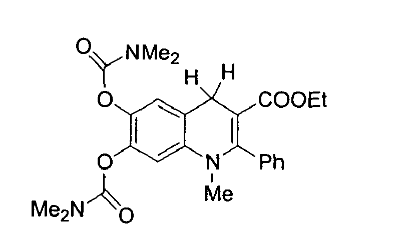

Stage A: 2-phenyl-6,7-hydroxy-3-quinolinecarboxylic acid
   The title compound is prepared according the procedure reported in stage A of example 1 from ethyl 2-phenyl-6,7-methoxyquinoline-3-carboxylate prepared as reported in Organic Letters, 2003, 5, 3061-3063.
Stage B: ethyl 1-methyl-2-phenyl-6,7-di(*N,N*-dimethylcarbamate)-1,4-dihydroquinoline-3-carboxylate
   The title compound is prepared from 6,7-hydroxy-3-quinolinecarboxylic acid synthesized in stage A following the procedures in stage B, C, D and E of example 1.

### Exemple 15 Preparation of ethyl 1,2,4-trimethyl-7-(N,N-dimethylcarbamate)-1,4-dihydro-3-quinolinecarboxylate ;

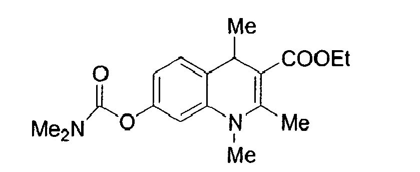

Stage A: ethyl 2,4-dimethyl-7-methoxy-3-quinolinecarboxylate
   To a solution of 2-amino-6-methoxyacetophenone (1.65 g, 10 mmol) prepared as reported in the Journal of Medicinal Chemistry (1989), 32, 807-26 in ethanol (75 mL) is added ethyl 2-acetoacetate (1.3 g, 10 mmol) and a catalytic amount of H₂SO₄. The resultant solution is refluxed for 12 hours. The title compound is obtained after evaporation of the solvent.
Stage B: ethyl 1,2,4-trimethyl-7-(*N*,*N*-dimethylcarbamate)-1,4-dihydroquinoline-3-carboxylate
   The title compound is synthesized from ethyl 2,4-dimethyl-7-methoxyquinoline-3-carboxylate prepared in stage A following the procedures in stage A, B, C, D and E of example 1.

### Example 16 Preparation of 2-methyl-7-(N,N-dimethylcarbamate)-1,2-dihydroisoquinoline

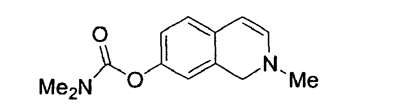

Stage A: 7-hydroxyisoquinoline
   The title compound is prepared according to the procedure reported in stage A of Example 1 starting from 7-methoxyisoquinoline (1.6 g, 10 mmol) reported in Bioorganic and Medicinal Chemistry, 1999, 2647-2666.
Stage B: 2-methyl-7-(*N,N*-dimethylcarbamate)-1,2-dihydroisoquinoline
   The title compound is prepared from 7-hydroxyisoquinoline prepared in stage A following the C, D and E of example 1.

### Example 17 Preparation of 2-methyl-7-(N,N-dimethylthiocarbamate)-1,2-dihydro-7-O-isoquinoline

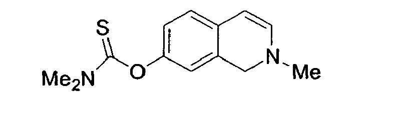

Stage A: 7-(*N*,*N*-dimethylthiocarbamate)-7-*O*-isoquinoline
   Starting from 7-hydroxyisoquinoline (1.45 g, 10 mmol) prepared in stage A of example 16, the title compound is prepared as described in stage A of example 5.
Stage B: 2-methyl-7-(*N,N*-dimethylthiocarbamate)-1,2-dihydro-7-*O*-isoquinoline
   The title compound is synthesized from 7-(*N,N*-dimethylthiocarbamate)-7-*O-*isoquinoline described in stage A following the procedure of stages D and E of example 1.

### Example 18 Preparation of 2-methyl-7-(N,N-dimethylthiocarbamate)-1,2-dihydro-7-S-isoquinoline

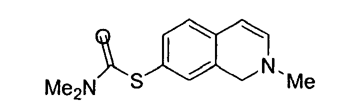

Stage A: 7-(*N*,*N*-dimethylthiocarbamate)-7-*O*-isoquinoline
   The title compound is prepared according to the procedure reported in stage A of example 6, from 7-(*N*,*N*-dimethylthiocarbamate)-7-*O*-isoquinoline (2.23 g, 10 mmol) described in stage A of example 17.
Stage B: 2-methyl-7-(*N,N*-dimethylthiocarbamate)-1,2-dihydro-7-*S*-isoquinoline
   The title compound is prepared following the procedures reported in stages D and E of example 1 from 7-(*N,N*-dimethylthiocarbamate)-*O*-isoquinoline reported in stage A.

### Example 19 Preparation of 1,2-dimethyl-7-(N,N-dimethylthiocarbamate)-1,2-dihydro-7-O-isoquinoline

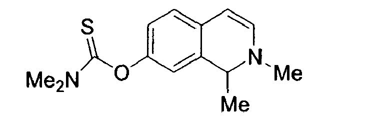

To a solution of 2-methyl-7-(*N,N*-dimethylthiocarbamate)-7-*O*-isoquinolinium triflate (3.8 g, 10 mmol), prepared in example 18, in THF (25 mL) is added a 2M solution of methylmagnesium bromide (5mL, 10 mmol) at -78°C under nitrogen atmosphere. The solution is stirred for 1 hour at this temperature. The solution is stirred a further 2 hours at 20°C. The reaction mixture is quenched with a saturated NH₄Cl solution. After extraction with CH₂Cl₂, the combined organic layers are dried (MgSO₄), the organic solvents are evaporated under vacuum affording the title compound.

### Example 20 Preparation of ethyl 2,3-dimethyl-6,8-di(N,N-dimethylcarbamate)-1,2-dihydro-4-isoquinolinecarboxylate

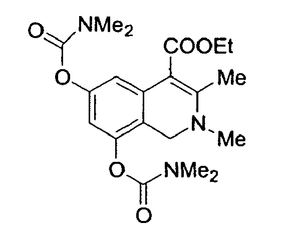

Stage A: 3-methyl 6,8-dihydroxy-3-isoquinolinecarboxylic acid
   The title compound is prepared according the procedure reported in stage A of example 1 from 3-methyl-4-cyano-6,8-dimethoxyisoquinoline reported in Tetrahedron Letters, 1968, 44, 1160-1163.
Stage B: ethyl 2,3-dimethyl-6,8-di(*N*,*N*-dimethylcarbamate)-1,2-dihydro-4-isoquinolinecarboxylate
   The title compound is prepared from 3-methyl-6,8-dihydroxy-3-isoquinolinecarboxylic acid prepared in stage A following the stages B, C, D and E of example 1.

### Example 21 Preparation of 2,3-dimethyl-6,8-di(N,N-dimethylcarbamate)-1,2-dihydro-4-isoquinolinecarboxamide

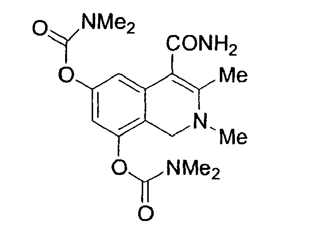

Stage A: 3-methyl-6,8-dimethoxy-4-isoquinolinecarboxamide
   3-methyl-4-cyano-6,8-dimethoxyisoquinoline (2.28 g, 20 mmol) reported in Tetrahedron Letters, 1968, 44, 1160-1163 is added to a finely powdered urea-hydrogen peroxide adduct (1.88 g, 20 mmol) in a glass tube, and the reaction mixture is placed in an oil bath at 85°C for 2 hours. After completion of the reaction, the reaction mixture is extracted with ethyl acetate and the combined extracts are washed with water and dried over anhydrous sodium sulfate. The solvent was removed under reduced pressure to afford the title compound.
Stage B: 2,3-dimethyl-6,8-di(*N,N*-dimethylcarbamate)-1,2-dihydro-4-isoquinolinecarboxamide
   The title compound is prepared from 3-methyl-6,8-dimethoxy-4-isoquinolinecarboxamide prepared in stage A following the procedures reported in stages B of example 8 and stages C, D and E of Example 1.

### Example 22 Preparation of 2,3-dimethyl-4-(4,5-dihydro-4,4-dimethyl-2-oxazolyl)-6,8-di(N,N-dimethylcarbamate)-1,2-dihydroisoquinoline

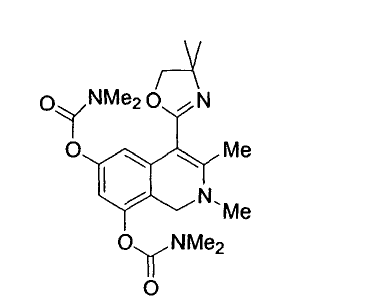

The title compound is prepared from 3-methyl-4-cyano-6,8-dimethoxyisoquinoline (2.28 g, 20 mmol) reported in Tetrahedron Letters, 1968, 44, 1160-1163 following the procedures reported in stages A and B of example 8, and in stages C, D, E of example 1.

### Example 23 Preparation of (+/-)-2,3-dimethyl-4-(4-methylphenylsulfinyl)-6,8-di(N,N-dimethylcarbamate)-1,2-dihydroisoquinoline

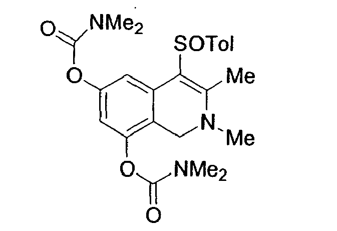

Stage A: 3-methyl-6,8-dimethoxy-4-isoquinolinecarboxylic acid
   The title compound is prepared from 3-methyl-4-cyano-6,8-dimethoxy isoquinoline (2.28 g, 20 mmol) reported in Tetrahedron Letters, 1968, 44, 1160-1163, following the procedure reported in stage A of example 9
Stage B: (+/-)-3-methyl-(4-methylphenylsulfinyl)-6,8-dimethoxy-4-isoquinoline
   The title compound is prepared from 3-methyl-6,8-dimethoxy-4-isoquinolinecarboxylic acid prepared in stage A following the procedures reported in stages A, B and C of example 10
Stage C: (+/-)-2,3-dimethyl-4-(4-methylphenylsulfinyl)-6,8-di(*N*,*N-*dimethylcarbamate)-1,2-dihydroisoquinoline
   The title compound is prepared from (+/-)-3-methyl-(4-methylphenylsulfinyl)-6,8-dimethoxy-4-isoquinoline prepared in stage B, following the procedures reported in stage B of example 8 and stage C, D and E of Example 1.

### Example 24 Preparation of 2,3-dimethyl-4-[4-(methyl)phenylsulfonyl]-6,8-di(N,N-dimethylcarbamate)-1,2-dihydroisoquinoline

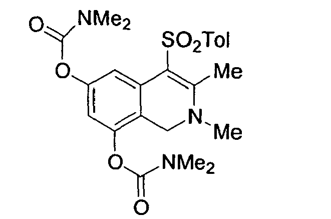

Stage A: (+/-)-3-methyl-(4-methylphenylsulfonyl)-6,8-dimethoxy-4-isoquinoline.
   The title compound is prepared from (+/-)-3-methyl-(4-methylphenylsulfinyl)-6,8-dimethoxy-4-isoquinoline prepared in stage B of example 23 following the procedure reported in stage A of example 11.
Stage B: (+/-)-2,3-dimethyl-4-(4-methylphenylsulfonyl)-6,8-di(*N,N-*dimethylcarbamate)-1,2-dihydroisoquinoline.
   The title compound is prepared from (+/-)-3-methyl-4-(4-methylphenylsulfonyl)-6,8-dimethoxyisoquinoline prepared in stage A, following the procedures reported in stages B of example 9, stages C, D, E of example 1.

### Example 25 Preparation of 2,3-dimethyl-4-(N-phenylsulfonamide)-6,8-di(N,N dimethylcarbamate)-1,2-dihydroisoquinoline.

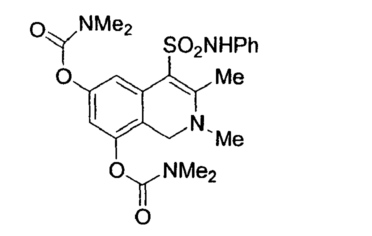

Stage A: 3-methyl-4-bromo-6,8-dimethoxy isoquinoline
   The title compound is prepared according the procedure reported in stage A of example 9 from 3-methyl-6,8-dimethoxy-4-isoquinolinecarboxylic acid prepared in stage A of example 23.
Stage B: 3-methyl-4-(methylthio)-6,8-dimethoxy isoquinoline
   The title compound is prepared according the procedure reported in stage A of example 12 from 3-methyl-4-bromo-6,8-dimethoxy isoquinoline prepared in stage A
Stage C: 3-methyl-4-(methylsulfinyl)-6,8-dimethoxy isoquinoline
   The title compound is prepared according the procedure reported in stage B of example 12 from 3-methyl-4-(methylthio)-6,8-dimethoxy isoquinoline prepared in stage B
Stage D: 3-methyl-6,8-dimethoxy-4-isoquinolinethiol
   The title compound is prepared according the procedure reported in stage C of example 12 from 3-methyl-4-(methylsulfinyl)-6,8-dimethoxy isoquinoline prepared in stage C
Stage E: *N*-phenyl-3-methyl-6,8-dimethoxy-4-isoquinolinesulfonamide
   The title compound is prepared from 3-methyl-6,8-dimethoxy-4-isoquinolinethiol of stage D according the procedure reported in stage D of example 12
Stage F *N*-phenyl-3-methyl-6,8-hydroxy-4-isoquinolinesulfonamide
   The title compound is prepared from N-phenyl-3-methyl-6,8-dimethoxy-4-isoquinolinesulfonamide of stage E according the procedure reported in stage B of example 8
Stage G 2,3-dimethyl-4-(*N*-phenylsulfonamide)-6,8-di(*N,N*-dimethylcarbamate)-1,2-dihydroisoquinoline.
   The title compound is prepared according to the procedures reported in stages C, D and E of example 1.

### Example 26 Preparation of 2,3-dimethyl-4-(trifluoromethyl)-6,8-di(N,N-dimethylcarbamate)-1,2-dihydroisoquinoline.

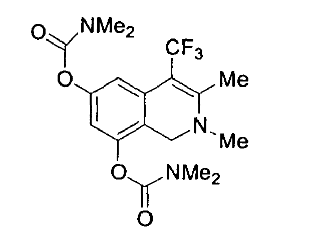

Stage A: 3-methyl-4(trifluoromethyl)-6,8-dimethoxy isoquinoline
   The title compound is prepared from 3-methyl-6,8-dimethoxy-4-isoquinolinecarboxylic acid obtained in Stage A of example 23, according to the procedure reported in stage B of example 9.
Stage B: 3-methyl-4(trifluoromethyl)-6,8-dihydroxy isoquinoline
   The title compound is synthesized according the procedure reported in stages B of example 8 from 3-methyl-4(trifluoromethyl)-6,8-dimethoxy isoquinoline obtained in stage A.
Stage C: 2,3-dimethyl-4-(trifluoromethyl)-6,8-di(*N*,*N*-dimethylcarbamate)-1,2-dihydroisoquinoline
   The title compound is synthesized according the procedure reported in stages stage C, D and E of example 1 from 3-methyl-4(trifluoromethyl)-6,8-dihydroxy isoquinoline obtained in stage B.

### Example 27 Preparation of ethyl 1-methyl-5-(N,N-dimethylcarbamate)-1,4-dihydropyridine-3-carboxylate

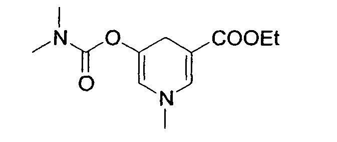

Stage A: ethyl 5-hydroxypyridine-3-carboxylate
   The title compound is synthesized according to the procedure reported in stage B of example 8 from 5-methoxypyridine-3-carboxylate described in the Journal of Medicinal Chemistry, 2000, 43, 3168-3185.
Stage B ethyl 5-(*N*,*N*-dimethylcarbamate) pyridine-3-carboxylate
   The title compound is synthesized following the procedure reported in stage C of example 1 from ethyl 5-hydroxy pyridine-3-carboxylate obtained in stage A.
Stage C: ethyl 1-methyl-5-(*N,N*-dimethylcarbamate)pyridinium-3-carboxylate triflate The title compound is prepared according to the procedure described in stage D of example 1 from ethyl 5-(*N,N*-dimethylcarbamate)pyridine-3-carboxylate obtained in stage B.
Stage D: ethyl 1-methyl-5-(*N*,*N*-dimethylcarbamate)-1,4-dihydropyridine-3-carboxylate
   The title compound is prepared according to the procedure described in stage E of example 1 from Ethyl 1-methyl-5-(*N,N*-dimethylcarbamate)pyridinium-3-carboxylate triflate obtained in stage C.

### Example 28 Preparation of ethyl 1-methyl-5-(N,N-dimethylthiocarbamate)-1,4-dihydro-5-O-pyridine-3-carboxylate

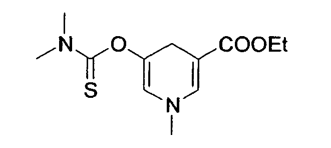

Stage A: ethyl 5-(*N,N*-dimethylthiocarbamate)-5-*O*-pyridine-3-carboxylate
   The title compound is prepared according to the procedure reported in stage A of example 5 from ethyl 5-hydroxypyridine-3-carboxylate obtained in stage A of example 27.
Stage B: ethyl 1-methyl-5-(*N,N*-dimethylthiocarbamate)-1,4-dihydro-5-*O*-pyridine-3-carboxylate
   The title compound is synthesized according to the procedures reported in stage D and E of example 1 from ethyl 5-(*N,N*-dimethylthiocarbamate)-5-*O*-pyridine-3-carboxylate obtained in stage A.

### Example 29 Preparation of ethyl 1-methyl-5-(N,N-dimethylthiocarbamate)-1,4-dihydro-5-S-pyridine-3-carboxylate

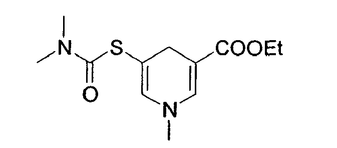

Stage A: ethyl 5-(*N,N*-dimethylthiocarbamate)-5-*S*-pyridine-3-carboxylate
   The title compound is synthesized according the procedure described in A of example 6 from ethyl 5-(*N,N*-dimethylthiocarbamate)-5-*O*-pyridine-3-carboxylate prepared in example 28.
Stage B: Ethyl 1-methyl-5-(*N,N*-dimethylthiocarbamate)-5-*S*-pyridinium-3-carboxylate triflate
   The title compound is prepared following the procedure reported in stage D of example 1 from ethyl 1-methyl 5-(*N,N*-dimethylthiocarbamate)-5-*S*-pyridinium-3-carboxylate prepared in stage A
Stage C: preparation of ethyl 1-methyl-5-(*N*,*N*-dimethylthiocarbamate)-1,4-dihydro-5-S-pyridine-3-carboxylate
   The title compound is prepared according the procedure reported in the stage E of example 1 from ethyl 1-methyl-5-(*N,N*-dimethylthiocarbamate)-5-*S*-pyridinium-3-carboxylate triflate prepared in stage B

### Example 30 Preparation of 1-methyl-3-(methylsulfonyl)-5-(N,N-dimethylcarbamate)-1,4-dihydropyridine

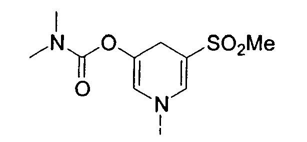

Stage A: 1-methyl-3-(methylsulfonyl)-5-(*N,N*-dimethylcarbamate)pyridinium triflate
   The title compound is synthesized according the procedures described in stages A, C and D of example 1 from 3-methoxy-5-(methylsulfonyl)pyridine reported in Tetrahedron, 1985, pages 173-1384.
Stage B : preparation of 1-methyl-3-(methylsulfonyl)-5-(*N,N*-dimethylcarbamate)-1,4-dihydropyridine
   The title compound is synthesized according the procedure reported in stage E of example 1 from 1-methyl-3-(methylsulfonyl)-5-(*N,N*-dimethylcarbamate)pyridinium triflate prepared in stage 1.

### Example 31 Preparation of 1-methyl-3-(N,N-diethylcarboxamido)-5-(N,N-dimethylcarbamate)-1,4-dihydropyridine

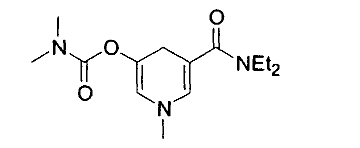

Stage A: 3-(*N,N*-diethylcarboxamido)-5-methoxy pyridine
   The title compound is synthesized according the procedure reported in stages B and C of example 7 from ethyl 5-methoxypyridine-3-carboxylate described in the Journal of medicinal chemistry, 2000, 43, 3168-3185.
Stage B: 1-methyl-3-(*N*,*N*-diethylcarboxamido)-5-(*N*,*N*-dimethylcarbamate) pyridinium triflate
   The title compound is synthesized according the procedure described in stage B of example 8 and in stage C and D of example 1 from 3-(*N,N*-diethylcarboxamido)-5-methoxypyridine obtained in stage A.
Stage C: 1-methyl-3-(*N,N*-diethylcarboxamido)-5-(*N,N*-dimethylcarbamate)-1,4-dihydropyridine
   The title compound is prepared following the procedure reported in stage E of example 1 from 1-methyl-3-(*N,N*-diethylcarboxamido)-5-(*N,N-*dimethylcarbamate)pyridinium triflate prepared in stage B

### Example 32 Preparation of (+/-)-1-methyl-3-(methylsufinyl)-5-(N,N-dimethylcarbamate)-1,4-dihydropyridine

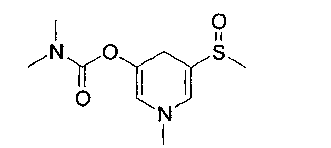

Stage A: (+/-)-1-methyl-3-(methylsufinyl)-5-(*N,N*-dimethylcarbamate) pyridinium triflate
   The title compound is synthesized according the procedure reported in stage A, C and D of example 1 from (+/-)-3-methoxy-5-(methylsulfinyl) pyridine described in Phosphorus, Sulfur and Silicon and the Related Elements, 1992, 66, 127-137.
Stage B: (+/-)-1-methyl-3-(methylsufinyl)-5-(*N,N*-dimethylcarbamate)-1,4-dihydropyridine
   The title compound is prepared according the procedure reported in stage E of example 1 from (+/-)-1-methyl-3-(methylsufinyl)-5-(*N,N-*dimethylcarbamate)pyridinium triflate obtained in stage A.

### Example 33 Preparation of 1-methyl-3-(trifluoromethyl)-5-(N,N-dimethylcarbamate)-1,4-dihydropyridine

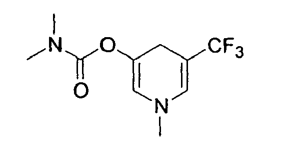

Stage A : 3-methoxy-5-trifluoromethylpyridine
   To a solution of 3-bromo-5-trifluoromethylpyridine (2.25 g 10 mmol, prepared as described in Eur. J. Org. Chem. 2002,327-330, in DMF was added sodium methoxide (0.8 g, 15 mmol). The resultant solution is stirred for 20 hours at 40°C. The title compound is obtained after evaporation of the solvent under vacuum.
Stage B: 1-methyl-3-(trifluoromethyl)-5-(*N,N*-dimethylcarbamate)pyridinium triflate
   The title compound is synthesized following the procedures reported in stage B of example 8 and in stages C and D of example 1 from 3-methoxy-5-trifluoromethylpyridine prepared in stage A
Stage C: 1-methyl-3-(trifluoromethyl)-5-(*N*,*N*-dimethylcarbamate)-1,4-dihydropyridine
   The title compound is synthesized following the procedure in stage E of example 1 from 1-methyl-3-(trifluoromethyl)-5-(*N,N*-dimethylcarbamate)pyridinium triflate prepared in stage B.

### Example 34 Preparation of 1-methyl-3-(4,5-dihydro-4,4-dimethyl-2-oxazolyl)-5-(N,N-dimethylcarbamate)-1,4-dihydropyridine

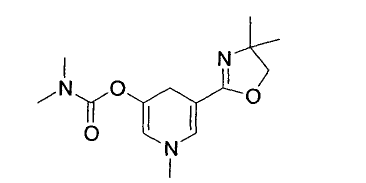

Stage A. 3-(4,5-dihydro-4,4-dimethyl-2-oxazolyl)-5-methoxypyridine
   The title compound is prepared according to the procedure reported in stage A of example 8 from 3-cyano-5-methoxypyridine prepared as reported in the Journal of Medicinal Chemistry, 2000, 43, 3168-3185.
Stage B: 1-methyl-3-(4,5-dihydro-4,4-dimethyl-2-oxazolyl)-5-(*N,N-*dimethylcarbamate)pyridinium triflate.
   The title compound is synthesized as reported in stage B of example 8 from 3-(4,5-dihydro-4,4-dimethyl-2-oxazolyl)-5-methoxypyridine affording 3-(4,5-dihydro-4,4-dimethyl-2-oxazolyl)-5-hydroxypyridine as an intermediate which is subsequently treated as described in stage C and D of example 1 to furnish 1-methyl-3-(4,5-dihydro-4,4-dimethyl-2-oxazolyl)-5-(*N*,*N*-dimethylcarbamate)pyridinium triflate.
Stage C: 1-methyl-3-(4,5-dihydro-4,4-dimethyl-2-oxazolyl)-5-(*N,N-*dimethylcarbamate)-1,4-dihydropyridine
   The title compound is obtained according to the procedure reported in stage E of example 1 from 1-methyl-3-(4,5-dihydro-4,4-dimethyl-2-oxazolyl)-5-(*N,N-*dimethylcarbamate)pyridinium triflate obtained in stage B.

### Example 35 Preparation of N,N-diethyl-1-methyl-5-(N,N-dimethylcarbamate)-1,4-dihydro-3-pyridinesulfonamide

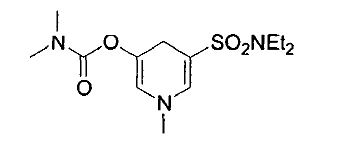

Stage A: *N,N*-diethyl-5-methoxy-3-pyridinesulfonamide
   The title compound is synthesized according to the procedures in stage C and D of example 12 from (+/-)-3-(methylsulfinyl)-5-methoxypyridine described in Phosphorus, Sulfur and Silicon and the Related Elements, 1992, 66, 127-137 and diethylamine.
Stage B: *N,N*-diethyl-1-methyl-5-(*N,N*-dimethylcarbamate)-3-pyridiniumsulfonamide triflate
   The title compound is prepared following the procedures in stage B, C and D of example 1 from *N,N*-diethyl-5-methoxy-3-pyridinesulfonamide prepared in stage A.
Stage C: *N,N*-diethyl-1-methyl-5-(*N,N*-dimethylcarbamate)-1,4-dihydro-3-pyridinesulfonamide
   The title compound is synthesized following the procedure in stage E of example 1 from *N,N*-diethyl-1-methyl-5-(*N,N*-dimethylcarbamate)-3-pyridiniumsulfonamide triflate obtained in stage B.

### Example 36 Preparation of ethyl 1-[2-(N,N-dimethylcarbamate)benzyl]-1,4-dihydro-3-pyridinecarboxylate

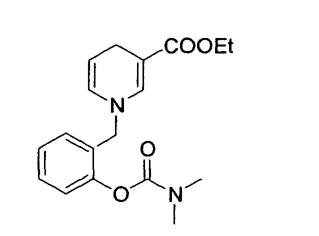

Stage A: ethyl 1-[2-(*N,N*-dimethylcarbamate)benzyl]-3-pyridiniumcarboxylate chloride
   To a solution of ethyl nicotinate (1.51 g, 10 mmol) in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: ethyl 1-[2-(*N,N*-dimethylcarbamate)benzyl]-1,4-dihydro-3-pyridinecarboxylate
   The title compound is synthesized according to the procedure reported in stage E of example 1 from ethyl 1-[2-(*N,N*-dimethylcarbamate)benzyl]-3-pyridiniumcarboxylate chloride prepared in stage A.

### Example 37 Preparation of 1-[2-(N,N-dimethylcarbamate)benzyl]-3-(methylcarbamoyl)-1,4-dihydropyridine

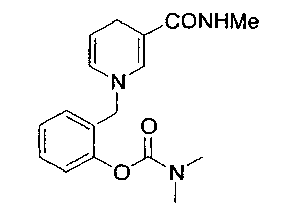

Stage A: 1-[2-(*N,N*-dimethylcarbamate)benzyl]-3-(methylcarbarnoyl)pyridinium chloride
   To a solution of 3-(methylcarbamoyl)pyridine (1.36 g, 10 mmol) described in Synthetic Communication, 1982, 12, 989-993 in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: 1-[2-(*N,N*-dimethylcarbamate)benzyl]-3-(methylcarbamoyl)-1,4-dihydropyridine
   The title compound is synthesized according to the procedure reported in stage E of example 1 from 1-[2-(*N,N*-dimethylcarbamate)benzyl]-3-(methylcarbamoyl)-pyridinium chloride prepared in stage A.

### Example 38 Preparation of 1-[2-(N,N-dimethylcarbamate)benzyl]-3-(4,5-dihydro-4,4-dimethyl-2-oxazolyl)-1,4-dihydropyridine

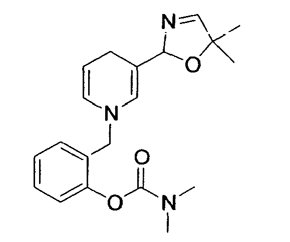

Stage A: 1-[2-(*N,N*-dimethylearbamate)benzyl]-3-(4,5-dihydro-4,4-dimethyl-2-oxazolyl)pyridinium chloride
   To a solution of 3-(4,5-dihydro-4,4-dimethyl-2-oxazolyl)pyridine (1.76 g, 10 mmol) described in Tetrahedron Letters, 1998, 39, 459-462 in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: preparation of 1-[2-(*N,N*-dimethylcarbamate)benzyl]-3-(4,5-dihydro-4,4-dimethyl-2-oxazolyl)-1,4-dihydropyridine.
   The title compound is synthesized according to the procedure reported in stage E of example 1 from 1-[2-(*N*,*N*-dimethylcarbamate)benzyl]-3-(4,5-dihydro-4,4-dimethyl-2-oxazolyl)-pyridinium chloride prepared in stage A.

### Example 39 Preparation of N,N-diethyl-1-[2-(N,N-dimethylcarbamate)benzyl]-1,4-dihydro-3-pyridinesulfonamide

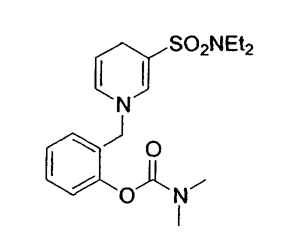

Stage A: *N,N*-diethyl-1-(2-(*N,N*-dimethylcarbamate)benzyl]-3-pyridiniumsulfonamide chloride
   To a solution of *N,N*-diethyl-3-pyridinesulfonamide (2.14 g, 10 mmol) described in the Journal of Organic Chemistry 2003, 68, 8274-8276 in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: *N,N*-diethyl-1-[2-(*N,N*-dimethylcarbamate)benzyl]-1,4-dihydro-3-pyridinesulfonamide
   The title compound is synthesized according to the procedure reported in stage E of example 1 from *N,N*-diethyl-1-[2-(*N,N*-dimethylcarbamate)benzyl]-3-pyridiniumsulfonamide chloride prepared in stage A.

### Example 40 Preparation of ethyl 1-[2-(N,N-dimethylcarbamate)benzyl)-1,4-dihydro-3-quinolinecarboxylate

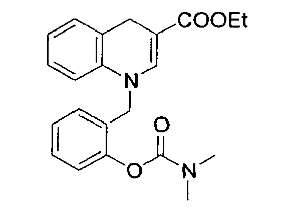

Stage A: ethyl 1-[2-(*N*,*N*-dimethylcarbamate)benzyl]-3-quinoliniumcarboxylate chloride
   To a solution of ethyl 3-quinolinecarboxylate(2.01 g, 10 mmol) in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: ethyl 1-[2-(*N,N*-dimethylcarbamate)benzyl]-1,4-dihydro-3-quinolinecarboxylate
   The title compound is synthesized according to the procedure reported in stage E of example 1 from ethyl 1-[2-(*N,N*-dimethylcarbamate)benzyl]-3-quinoliniumcarboxylate chloride prepared in stage A.

### Example 41 Preparation of 1-[2-(N,N-dimethylcarbamate)benzyl]-1,4-dihydro-3-(dimethylcarbamoyl)-quinoline

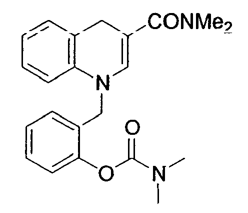

Stage A: 1-[2-(*N,N*-dimethylcarbamate)benzyl]-3-(dimethylcarbamoyl)quinolinium chloride
   To a solution of 3-(dimethylcarbamoyl)quinoline (2.0 g, 10 mmol) in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: 1-[2-(*N,N*-dimethylcarbamate)benzyl]-1,4-dihydro-3-(methylcarbamoyl)quinoline
   The title compound is synthesized according to the procedure reported in stage E of example 1 from 1-[2-(*N,N*-dimethylcarbamate)benzyl]-3-(dimethylcarbamoyl)quinolinium chloride prepared in stage A.

### Example 42 Preparation of 1-[2-(N,N-dimethylcarbamate)benzyl]-1,4-dihydro-3-(4,5-dihydro-4,4-dimethyl-2-oxazolyl) quinoline

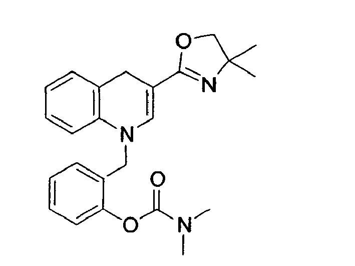

Stage A: 1-[2-(*N,N*-dimethylcarbamate)benzyl]-3-(4,5-dihydro-4,4-dimethyl-2-oxazolyl)quinolinium chloride.
   To a solution of 3-(4,5-dihydro-4,4-dimethyl-2-oxazolyl) quinoline described in Synthesis, 1987, 693-696 (2.26 g, 10 mmol) in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: 1-[2-(*N*,*N*-dimethylcarbamate)benzyl]-1,4-dihydro-3-(4,5-dihydro-4,4-dimethyl-2-oxazolyl)quinoline
   The title compound is synthesized according to the procedure reported in stage E of example 1 from 1-[2-(*N,N*-dimethylcarbamate)benzyl]-3-(4,5-dihydro-4,4-dimethyl-2-oxazolyl)quinolinium chloride prepared in stage A.

### Example 43 Preparation of N,N-dimethyl-1-[2-(N,N-dimethylcarbamate)benzyl]-1,4-dihydro-3-quinolinesulfonamide

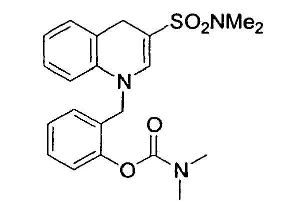

Stage A: *N,N*-dimethyl-3-quinolinesulfonamide
   The title compound is synthesized according to the procedure reported in stage A of example 2 from *N,N*-dimethyl-4-chloro-3-quinolinesulfonamide reported in Heterocycle, 1997, 45, 2015-2021.
Stage B: *N,N*-dimethyl-1-[2-(*N,N*-dimethylcarbamate)benzyl]-3-quinolininiumsulfonamide chloride.
   To a solution of *N,N*-dimethyl-3-quinolinesulfonamide, obtained in stage A, in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage C: *N,N*-dimethyl-1-[2-(*N,N*-dimethylcarbamate)benzyl]-1,4-dihydro-3-quinolinesulfonamide
   The title compound is synthesized according to the procedure reported in stage E of example 1 from *N,N*-dimethyl-1-[2-(*N,N*-dimethvlcarbamate)benzyl]-3-quinolininiumsulfonamide chloride prepared in stage B.

### Example 44 Preparation of ethyl 2-[2-(N,N-dimethylcarbamate)benzyl]-1,2-dihydro-4-isoquinolinecarboxylate

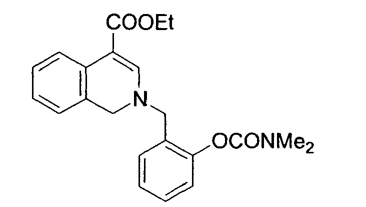

Stage A: ethyl 2-[2-(*N,N-*dimethylcarbamate)benzyl]-4-isoquinoliniumcarboxylate chloride
   To a solution of ethyl 3-isoquinolinecarboxylate described in Tetrahedron: Asymmetry, 2003, 14, 3469-3477 (2.01 g, 10 mmol) in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: ethyl 2-[2-(*N,N*-dimethylcarbamate)benzyl]-1,2-dihydro-4-isoquinolinecarboxylate
   The title compound is synthesized according to the procedure reported in stage E of example 1 from ethyl 2-[2-(*N,N*-dimethylcarbamate)benzyl]-4-isoquinoliniumcarboxylate chloride prepared in stage A.

### Example 45 Preparation of 2-[2-(N,N-dimethylcarbamate)benzyl]-4-(N-β-phenethylcarbamoyl)-1,2-dihydroisoquinoline

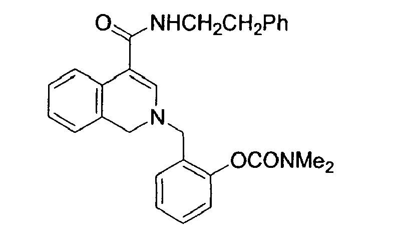

Stage A: 2-[2-(*N,N*-dimethylcarbamate)benzyl]-4-(N-β-phenethylcarbamoyl)isoquinolinium chloride.
   To a solution of 4-(N-β-phenethylcarbamoyl)isoquinoline described in Arch. Pharm. Pharm. Med. Chem, 2003, 336, 258-263 in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: 2-[2-(*N,N*-dimethylcarbamate)benzyl]-4-(N-β-phenethylcarbamoyl)-1,2-dihydroisoquinoline
   The title compound is synthesized according to the procedure reported in stage E of example 1 from 2-[2-(*N,N*-dimethylcarbamate)benzyl]-4-(N-β-phenethylcarbamoyl)isoquinolinium chloride prepared in stage A.

### Example 46: 2-[2-(N,N-dimethylcarbamate)benzyl]-4-(4,5-dihydro-4,4-dimethyl-2-oxazolyl)-1,2-dihydroisoquinoline

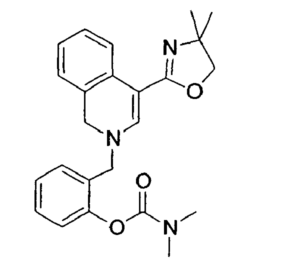

Stage A: 2-[2-(*N,N*-dimethylcarbamate)benzyl]-4-(4,5-dihydro-4,4-dimethyl-2-oxazolyl)isoquinolinium chloride
   To a solution of 4-(4,5-dihydro-4,4-dimethyl-2-oxazolyl)isoquinoline described in Tetrahedron Letters , 1986, 27, 5269-5270 (2.26 g, 10 mmol) in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: 2-[2-(*N,N*-dimethylcarbamate)benzyl]-4-(4,5-dihydro-4,4-dimethyl-2-oxazolyl)-1,2-dihydroisoquinoline
   The title compound is synthesized according to the procedure reported in stage E of example 1 from 2-[2-(*N,N*-dimethylcarbamate)benzyl]-4-(4,5-dihydro-4,4-dimethyl-2-oxazolyl)isoquinolinium chloride obtained in stage A.

### Example 47 Preparation of N,N-diethyl-2-[2-(N,N-dimethylcarbamate)benzyl]-1,2-dihydro-4-isoquinolinesulfonamide

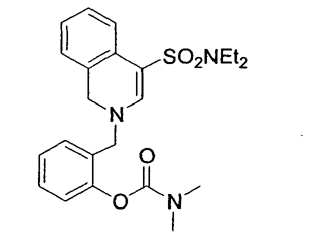

Stage A: *N,N*-diethyl-2-[2-(*N,N*-dimethylcarbamate)benzyl]-4-isoquinolininiumsulphonamide
   To a solution of *N,N*-diethyl-4-isoquinolinesulfonamide (2.64 g, 10 mmol) described in the Journal of Organic Chemistry 2003, 68, 8274-8276 in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: *N,N*-diethyl-2-[2-(*N,N*-dimethylcarbamate)benzyl]-1,2-dihydro-4-isoquinolinesulfonamide
   The title compound is synthesized according to the procedure reported in stage E of example 1 from *N,N*-diethyl-2-[2-(*N,N*-dimethylcarbamate)benzyl]-4-isoquinolininiumsulphonamide obtained in stage A.

### Example 48 Preparation of 1-[2-(N,N-dimethylcarbamate)benzyl]-3-(trifluoromethyl)-1,4-dihydropyridine

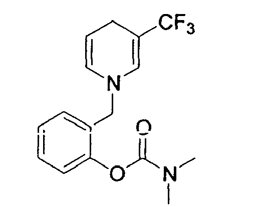

Stage A: 1-[2-(*N,N*-dimethylcarbamate)benzyl]-3-(trifluoromethyl)pyridinium chloride
   To a solution of 3-(trifluoromethyl)pyridine (1.47 g, 10 mmol) described in Eur. J. Org. Chem. 2003, 327-330 in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: 1-[2-(*N,N*-dimethylcarbamate)benzyl]-3-(trifluoromethyl)-1,4-dihydropyridine.
   The title compound is synthesized according to the procedure reported in stage E of example 1 from 1-[2-(*N,N*-dimethylcarbamate)benzyl]-3-(trifluoromethyl)pyridinium chloride obtained in stage A.

### Example 49 Preparation of 1-[2-(N,N-dimethylcarbamate)benzyl]-3-(trifluoromethyl)-1,4-dihydroquinoline

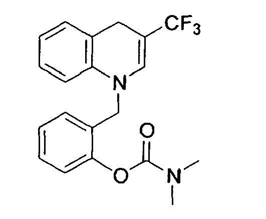

Stage A: 1-[2-(*N,N*-dimethylcarbamate)benzyl]-3-(trifluoromethyl)quinolininium chloride
   To a solution of 3-(trifluoromethyl)quinoline (1.97 g, 10 mmol) described in Journal of the Chemical Society, Chemical Communications (1992),(1), 53-54, in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: 1-[2-(*N,N*-dimethylcarbamate)benzyl]-3-(trifluoromethyl)-1,4-dihydroquinoline.
   The title compound is synthesized according to the procedure reported in stage E of example 1 from 1-[2-(*N,N*-dimethylcarbamate)benzyl]-3-(trifluoromethyl) quinolininium chloride obtained in stage A.

### Example 50. Preparation of 1-[2-(N,N-dimethylcarbamate)benzyl]-3-trifluoromethyl)-1,2-dihydroisoquinoline.

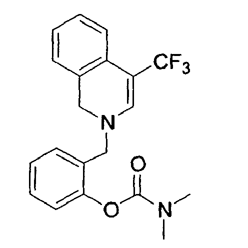

Stage A: 1-[2-(*N,N*-dimethylcarbamate)benzyl]-3-(trifluoromethyl)isoquinolinium chloride
   To a solution of 3-(trifluoromethyl)quinoline (1.97 g, 10 mmol), described in Bulletin of the Chemical Society of Japan (1988), 61(10), 3531-7, in a proper solvent (acetonitrile, DMF, EtOH, acetone) is added carbamic acid, dimethyl-, 2-(chloromethyl)phenyl ester (2.13 g, 10 mmol) reported in Chemical Papers 1985, 39, 413-27. The resultant solution is stirred for 24 hours at the correct temperature (25°C-120°C). The solvent is evaporated to furnish the title compound.
Stage B: 1-[2-(*N,N*-dimethylcarbamate)benzyl]-3-(trifluoromethyl)-1,2-dihydroisoquinoline.
   The title compound is synthesized according to the procedure reported in stage E of example 1 from 1-[2-(*N,N*-dimethylcarbamate)benzyl]-3-(trifluoromethyl)isoquinolinium chloride prepared in stage A.

### Example 51 Preparation of ethyl 1-methyl-2-[2-(N,N-dimethylcarbamate)phenyl]-1,4-dihydro pyridine-3-carboxylate

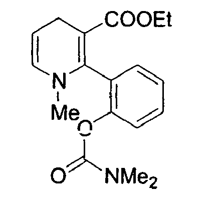

Stage A: ethyl 2-(2-methoxyphenyl)pyridine-3-carboxylate
   A solution of ethyl 2-chloronicotinate (1.85 g, 10 mmol), 2-methoxyphenylboronic acid (1.22 g, 10 mmol), Pd(PPh₃)₄ (5%mol), K₂CO₃ (5g) in DMF/water (3/1) is stirred at 50°C for 12 hours. The reaction mixture is then pored on water (150 mL). The aqueous phase is extracted with ethyl acetate (3x100mL). the combined organic layers are washed with water (3x200mL). The organic phase is dried over magnesium sulfate and evaporated under vacuum to afford the title compound.
Stage B: ethyl 1-methyl-2-[2-(*N,N*-dimethylcarbamate)phenyl)pyridininium-3-carboxylate triflate
   The title compound is prepared according to the procedures in stages A, B, C, D of example 1 from ethyl 2-(2-methoxyphenyl)pyridine carboxylate prepared in stage A.
Stage C: ethyl 1-methyl-2-[2-(*N,N*-dimethylcarbamate)phenyl]-1,4-dihydropyridine-3-carboxylate
   The title compound is prepared according to the procedure in stage E of example 1 from ethyl 1-methyl-2-[2-(*N,N*-dimethylcarbamate)phenyl] pyridininium-3-carboxylate triflate prepared in stage B.

### Example 52 Preparation of 1-methyl-2-[2-(N,N-dimethylcarbamate)phenyl]-1,4-dihydro-N,N-dimethylnicotinamide

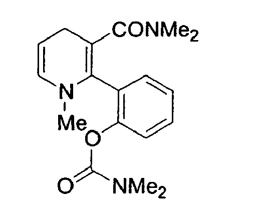

Stage A: 2-(2-methoxyphenyl)-*N,N*-dimethylnicotinamide
   The title compound is prepared according the procedure reported in stage A of example 51 from 2-chloro-*N,N*-dimethylnicotinamide described in the Journal of Medicinal Chemistry (1989), 32(9), 2178-99
Stage B: 1-methyl-2-(2-(*N,N*-dimethylcarbamate)phenyl]-3-(*N,N-*dimethylcarbamoyl) pyridinium triflate.
   The title compound is synthesized following the procedure reported in stage B of example 8 from 2-(2-methoxyphenyl)-*N,N*-dimethylnicotinamide and stages C and D of example 1.
Stage C: 1-methyl-2-[2-(*N,N*-dimethylcarbarnate)phenyl]-1,4-dihydro-*N*,*N-*dimethylnicotinamide.
   The title compound is synthesized following the procedure reported in stage E of example 1 from 1-methyl-2-[2-(*N,N*-dimethylcarbamate)phenyl]-3-(*N,N-*dimethylcarbamoyl)pyridinium triflate prepared in stage B.

### Example 53 Preparation of 1-methyl-2-[2-(N,N-dimethylcarbamate)phenyl]-3-(methylsulfonyl)-1,4-dihydropyridine

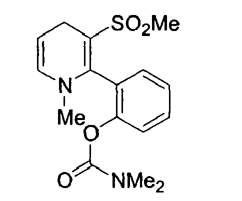

Stage A: 2-(2-methoxyphenyl)-3-(methylsulfonyl)pyridine
   The title compound is synthesized following the procedure reported in stage A of example 51 from 2-chloro-3-(methylsulfonyl)pyridine described in the Journal of Organic Chemistry (1979), 44, 3080-3082.
Stage B: 1-methyl-2-[2-(*N,N*-dimethylcarbamate)phenyl]-3-(methylsulfonyl)pyridinium triflate
   The title compound is prepared according to the procedures reported in stages A, B, C, D of example 1 from 2-(2-methoxyphenyl)-3-(methylsulfonyl)pyridine obtained in stage A.
Stage C: 1-methyl-2-[2-(*N,N*-dimethylcarbamate)phenyl]-3-(methylsulfonyl)-1,4-dihydropyridine
   The title compound is prepared according to the procedure reported in stage E of example 1 from 1-methyl-2-[2-(*N,N*-dimethylcarbamate)phenyl]-3-(methylsulfonyl)pyridinium triflate obtained in stage A.

### Example 54 Preparation of N-methyl-1-methyl-2-[2-(N,N-dimethylcarbamate)phenyl]-1,4-dihydro-3-pyridinesulfonamide

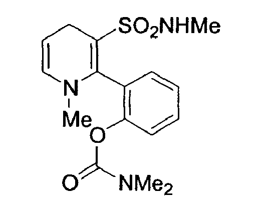

Stage A: *N*-methyl-2-(2-methoxyphenyl)-3-pyridinesulfonamide
   The title compound is synthesized according the procedure described in stage A of example 51 from *N*-methyl-2-chloro-3-pyridinesulfonamide (1.72 g, 10 mmol) reported in Memoires de l'Academie Royale de Medecine de Belgique (1974), 47(3), 131-210.
Stage B: *N*-methyl-1-methyl-2-[2-(*N,N*-dimethylcarbamate)phenyl]-3-pyridiniumsulfonamide triflate
   The title compound is prepared according to the procedures in stages A, B, C, D of example 1 from *N*-methyl-2-(2-methoxyphenyl)-3-pyridinesulfonamide prepared in stage A
Stage C: *N*-methyl-1-methyl-2-[2-(*N,N*-dimethylcarbamate)phenyl]-1,4-dihydro-3-pyridinesulfonamide
   The title compound is synthtsized according the procedure described in stage E of example 1 from *N*-methyl-1-methyl-2-[2-(*N*,*N*-dimethylcarbamate)phenyl]-3-pyridiniumsulfonamide triflate prepared in stage B.

### Example 55 Preparation of ethyl 1-methyl-6-[2-(N,N-dimethylcarbamate)phenyl]-1,4-dihydropyridine-3-carboxylate

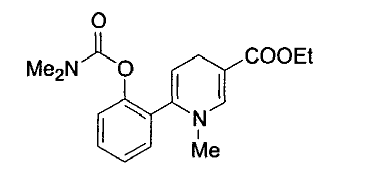

Stage A: 6-(2-methoxyphenyl)nicotinonitrile
   A solution of ethyl 6-bromonicotinonitrile (1.81 g, 10 mmol) described in the Journal of Organic Chemistry 2001, 66, 1500-1502, 2-methoxyphenylboronic acid (1.22 g, 10 mmol), Pd(PPh₃)₄ (5%mol), K₂CO₃ (5g) in DMF/water (3/1) is stirred at 50°C for 12 hours. The reaction mixture is then pored on water (150 mL). The aqueous phase is extracted with ethyl acetate (3x100mL). The combined organic layers are washed with water (3x200mL). The organic phase is dried over magnesium sulfate and evaporated under vacuum to afford the title compound.
Stage B: ethyl 1-methyl-6-[2-(*N,N*-dimethylcarbamate)phenyl]pyridininium-3-carboxylate triflate
   The title compound is prepared according to the procedures in stages A, B, C, D of example 1 from 6-(2-methoxyphenyl)nicotinonitrile prepared in stage A.
Stage C: ethyl 1-methyl-6-[2-(*N,N*-dimethylcarbamate)phenyl]-1,4-dihydropyridine-3-carboxylate
   The title compound is prepared according to the procedure reported in stage E of example 1 from ethyl 1-methyl-6-[2-(*N,N*-dimethylcarbamate)phenyl]pyridininium-3-carboxylate triflate prepared in stage B.

### Example 56 Preparation of ethyl 1-methyl-5-[2-(N,N-dimethylcarbamate)phenyl]-1,4-dihydro pyridine-3-carboxylate

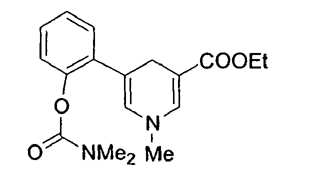

Stage A: ethyl 5-(2-methoxyphenyl)pyridine-3-carboxylate
   A solution of ethyl 5-bromonicotinate (2.29 g, 10 mmol), prepared as reported in the Journal of Medicinal Chemistry 1995, 38, 1608-28, 2-methoxy phenyl boronic acid (1.22 g, 10 mmol), Pd(PPh₃)₄ (5%mol), K₂CO₃ (5g) in DMF/water (3/1) is stirred at 50°C for 12 hours. The reaction mixture is then pored on water (150 mL). The aqueous phase is extracted with ethyl acetate (3x100mL). the combined organic layers are washed with water (3x200mL). The organic phase is dried over magnesium sulfate and evaporated under vacuum to afford the title compound.
Stage B: ethyl 1-methyl-5-[2-(*N,N*-dimethylcarbamate)phenyl]pyridininium-3-carboxylate triflate.
   The title compound is prepared according to the procedures reported in stages A, B, C, D of example 1 from ethyl 5-(2-methoxyphenyl)pyridine carboxylate prepared in stage A.
Stage C: preparation of ethyl 1-methyl-5-[2-(*N*,*N*-dimethylcarbamate)phenyl]-1,4-dihydropyridine-3-carboxylate.
   The title compound is prepared according to the procedure described in stage E of example 1 from ethyl 1-methyl-5-[2-(*N,N*-dimethylcarbamate)phenyl]pyridininium-3-carboxylate triflate prepared in stage B.

### Example 57 Preparation of ethyl 1-methyl-2-[2-(N,N-dimethylcarbamate)phenyl]-1,4-dihydroquinoline-3-carboxylate.

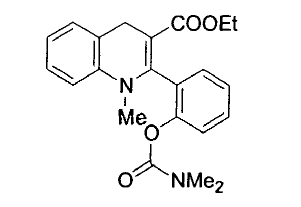

Stage A: ethyl 2-(2-methoxyphenyl)quinoline-3-carboxylate
   A solution of ethyl 2-chloroquinoline-3-carboxylate (2.01 g, 10 mmol), prepared as described in the Journal of Organic Chemistry 2003, 68, 9517-9520, 2-methoxyphenylboronic acid (1.22 g, 10 mmol), Pd(PPh₃)₄ (5%mol), K₂CO₃ (5g) in DMF/water (3/1) is stirred at 50°C for 12 hours. The reaction mixture is then pored on water (150 mL). The aqueous phase is extracted with ethyl acetate (3x100mL). the combined organic layers are washed with water (3x200mL). The organic phase is dried over magnesium sulfate and evaporated under vacuum to afford the title compound.
Stage B: ethyl 1-methyl-2-[2-(*N,N*-dimethylcarbamate)phenyl]quinolinium-3-carboxylate triflate.
   The title compound is prepared according to the procedures reported in stages A, B, C, D of example 1 from ethyl 2-(2-methoxyphenyl)quinoline-3-carboxylate prepared in stage A.
Stage C: preparation of ethyl 1-methyl-2-[2-(*N,N-*dimethylcarbamate)phenyl]-1,4-dihydroquinoline-3-carboxylate.
   The title compound is prepared according to the procedure described in stage E of example 1 from ethyl 1-methyl-2-[2-(*N,N*-dimethylcarbamate)phenyl]quinolinium-3-carboxylate triflate prepared in stage B.

### Example 58 Preparation of ethyl 1-methyl-8-[2-(N,N-dimethylcarbamate)phenyl]-1,4-dihydroquinoline-3-carboxylate.

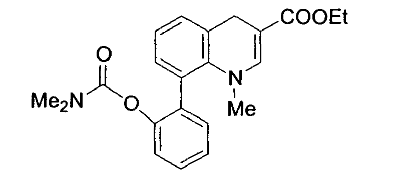

Stage A: ethyl 8-(2-methoxyphenyl)quinoline-3-carboxylate.
   A solution of ethyl 8-bromoquinoline-3-carboxylate (2.01 g, 10 mmol), prepared as described in Patent WO 2001047891, 2-methoxyphenylboronic acid (2.79 g, 10 mmol), Pd(PPh₃)₄ (5%mol), K₂CO₃ (5g) in DMF/water (3/1) is stirred at 50°C for 12 hours. The reaction mixture is then pored on water (150 mL). The aqueous phase is extracted with ethyl acetate (3x100mL). The combined organic layers are washed with water (3x200mL). The organic phase is dried over magnesium sulfate and evaporated under vacuum to afford the title compound.
Stage B: ethyl 1-methyl-8-[2-(*N,N*-dimethylcarbamate)phenyl]quinolinium-3-carboxylate triflate.
   The title compound is prepared according to the procedures reported in stages A, B, C, D of example 1 from ethyl 8-(2-methoxyphenyl)quinoline-3-carboxylate prepared in stage A.
Stage C: ethyl 1-methyl-8-[2-(*N,N*-dimethylcarbamate)phenyl]-1,4-dihydroquinoline-3-carboxylate.
   The title compound is prepared according to the procedure reported in stage E of example 1 from ethyl 1-methyl-8-[2-(*N*,*N*-dimethylcarbamate)phenyl]quinolinium-3-carboxylate triflate prepared in stage B.

### Example 59 Preparation of 1-[2-(N,N-dimethylcarbamate)phenyl]-2-methyl-1,2-dihydroisoquinoline.

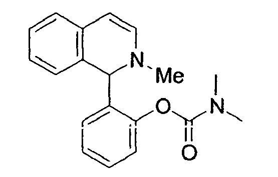

Stage A: 1-(2-methoxyphenyl)isoquinoline.
   A solution of 1-bromoisoquinoline (2.06 g, 10 mmol), prepared as described in European Journal of Organic Chemistry 2002, 4181-4184, 2-methoxyphenylboronic acid (2.79 g, 10 mmol), Pd(PPh₃)₄ (5%mol), K₂CO₃ (5g) in DMF/water (3/1) is stirred at 50°C for 12 hours. The reaction mixture is then pored on water (150 mL). The aqueous phase is extracted with ethyl acetate (3x100mL). The combined organic layers are washed with water (3x200mL). The organic phase is dried over magnesium sulfate and evaporated under vacuum to afford the title compound.
Stage B: 1-[2-(*N,N*-dimethylcarbamate)phenyl]-2-methylisoquinolinium triflate.
   The title compound is prepared according to the procedures reported in stages A, B, C, D of example 1 from 1-(2-methoxyphenyl)isoquinoline prepared in stage A.
Stage C: 1-[2-(*N,N*-dimethylcarbamate)phenyl]-2-methyl-1,2-dihydroisoquinoline.
   The title compound is prepared according to the procedure reported in stage E of example 1 from 1-[2-(*N,N*-dimethylcarbamate)phenyl]-2-methylisoquinolinium triflate prepared in stage B.

### Example 60 Preparation of 2-methyl-4-[2-(N,N-dimethylcarbamate)phenyl]-1,2-dihydroisoquinoline.

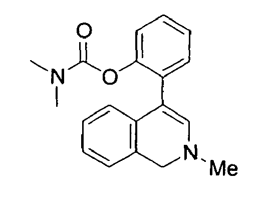

Stage A: 4-(2-methoxyphenyl) isoquinoline.
   A solution of 4-bromoisoquinoline (2.06 g, 10 mmol), prepared as described in the Journal of Organic Chemistry 2003, 68, 9412-9415, 2-methoxyphenylboronic acid (2.79 g, 10 mmol), Pd(PPh₃)₄ (5%mol), K₂CO₃ (5g) in DMF/water (3/1) is stirred at 50°C for 12 hours. The reaction mixture is then pored on water (150 mL). The aqueous phase is extracted with ethyl acetate (3x100mL). The combined organic layers are washed with water (3x200mL). The organic phase is dried over magnesium sulfate and evaporated under vacuum to afford the title compound.
Stage B: 4-[2-(*N,N*-dimethylcarbamate)phenyl]-2-methylisoquinolinium triflate.
   The title compound is prepared according to the procedures reported in stages A, B, C, D of example 1 from 4-(2-methoxyphenyl) isoquinoline prepared in stage A.
Stage C: 4-[2-(*N,N*-dimethylcarbamate)phenyl]-2-methyl-1,2-dihydroisoquinoline.
   The title compound is prepared according to the procedure reported in stage E of example 1 from 4-[2-(*N,N*-dimethylcarbamate)phenyl]-2-methylisoquinolinium triflate obtained in stage B.

### Example 61 Preparation of ethyl 1-methyl-7-(N,N-dimethylcarbamate)-1,2-dihydroquinoline-3-carboxylate

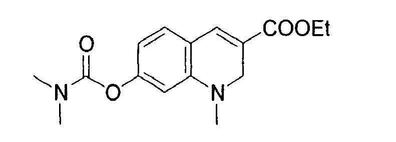

To a solution of ethyl N-methyl-7-(*N,N*-dimethylcarbamate)quinolinium-3-carboxylate triflate (100 mg, 0.22 mmol) prepared in stage D of example 1 in EtOH (10 mL) was added NaBH₄ (38 mg, 1 mmol). The resultant mixture was stirred for 3 hours at room temperature. After addition of water (2 mL) and evaporation of EtOH, the resulting aqueous phase was extracted twice with CH₂C1₂ (2 x 10 mL). After drying (MgSO₄) and evaporation, flash chromatography on silica gel afforded the title compound in 20-60% yield.

### Example 62 Preparation of ethyl 1-methyl-5-(N,N-dimethylthiocarbamate)-1,2-dihydro-5-S-quinoline-3-carboxylate

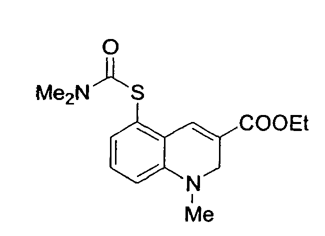

The title compound is prepared as reported in stage A of example 61 from a solution of ethyl 1-methyl-5-(*N,N*-dimethylthiocarbamate)-5-*O*-quinolinium-3-carboxylate triflate prepared in stage B of example 5.

### Example 63 Preparation of 1-methyl-5-(N,N-dimethylcarbamate)-3-(4,5-dihydro-4,4-dimethyl-2-oxazolyl)-1,2-dihydroquinoline

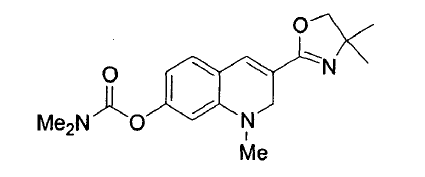

The title compound is prepared as reported in stage A of example 61 from 1-methyl-5-(*N,N*-dimethylcarbamate)-3-(4,5-dihydro-4,4-dimethyl-2-oxazolyl)quinolinium triflate prepared in stage D of example 8.

### Example 64 Preparation of ethyl 1-methyl-5-(N,N-dimethylcarbamate)-1,2-dihydropyridine-3-carboxylate

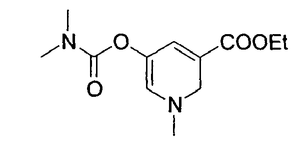

The title compound is prepared as reported in stage A of example 61 from ethyl 1-methyl-5-(*N,N*-dimethylcarbamate)pyridinium-3-carboxylate triflate prepared in stage C of example 27.

### Example 65 Preparation of ethyl-1-methyl-5-(N,N-dimethylthiocarbamate)-1,2-dihydro-5-S-pyridine-3-carboxylate

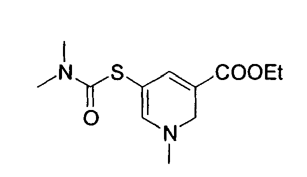

The title compound is prepared as reported in stage A of example 61 from ethyl 1-methyl 5-(*N,N*-dimethylthiocarbamate)-5-*O*-pyridinium-3-carboxylate prepared in stage B of example 29.

### Example 66 Preparation of 1-methyl-3-(N,N-diethylcarboxamido)-5-(N,N-dimethylcarbamate)-1,2-dihydropyridine.

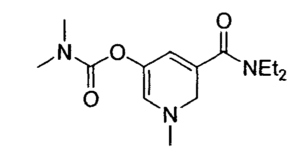

The title compound is prepared as reported in stage A of example 61 from 1-methyl-3-(*N,N*-diethylcarboxamido)-5-(*N,N*-dimethylcarbamate)pyridinium triflate prepared in stage B of example 31.

### Pharmacological tests.

### Example 67

### In vitro inhibition of acetylcholinesterase (AChE).

AChE was extracted from control human erythrocytes. The molecular form of AChE was separated by ultracentrifugation obtained by lysing of erythrocytes. The membranes were centrifuged at 38,000 rpm in a SW55B rotor in a Centrikon T-1055 (Kontron)ultracentrifuge for 2 hours. In vitro inhibition of AChE was determined by Ellman's spectrophotometric method using method using acetylthiocholine (ACThCh) (Ellman, G.L., Courtney, K.KD., Andres, V., and Featherstone, R.M. A new and rapid colorimetric determination of acetylcholinesterase activity Biochem. Pharmacol. 1961, (7) 88-95)

### Example 68

In this example, a pharmaceutical composition was prepared for injection.

This pharmaceutical composition contained:
- the compound of Example 1: 500 mg
- a sterile aqueous excipient: 10 ml.

## Claims

1. Compound comprising at least one radical C=Y, Y being O or S, and an oxidable and non protonable nitrogen atom N wherein the distance (d) between the at least one carbon atom of the radical group C=Y and the nitrogen atom, when oxidized, is comprised between 0.3 and 0.8 nanometers.

2. Compound according to claim 1 wherein the distance d is between 0.4 and 0.7 nm.

3. Compound which is an acetylcholinesterase inhibitor, at least 500, preferably at least 1000 times more active in central nervous system CNS than in peripheral nervous system PNS.

4. Compound according to claim 3 which is an acetylcholinesterase inhibitor, at least 500 or at least 1000 times more active in central nervous system CNS under its oxidized form than in peripheral nervous system PNS under its non oxidized form

5. Compound of formula G 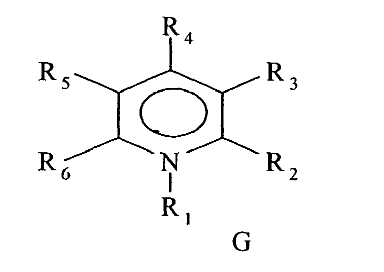 wherein:
the dotted circle line represents one double bond between CR₅-CR₆, and another double bond between either CR₂-CR₃ or CR₃-CR₄ ; and either
a) R₁ R₂ R₃ R₄ R₅ R₆ which may be identical or different are hydrogen, OH, (C₁-C₈) alkyl, aryl, heteroaryl, (C₁-C₈) alkylaryl, aryl (C₁-C₈) alkyl, alkoxy, hydroxy (C₁-C₈) alkyl, alkoxy (C₁-C₈) alkyl, phenyl, (CH₂)ₙ-COOH, Z, Z₁;
or
b) R₄ and R₅ or c) R₅ and R₆ taken together with the carbon atoms to which they are attached form a 6-membered aromatic ring or form a 5- or 6-membered heterocyclic ring being optionally substituted by one or more identical or different group defined as OH, (C₁-C₈) alkyl, aryl, heteroaryl, (C₁-C₈) alkylaryl, aryl (C₁-C₈) alkyl, alkoxy, hydroxy (C₁-C₈) alkyl, alkoxy (C₁-C₈) alkyl, phenyl, (CH₂)ₙ-COOH, Z, Z₁;
in all case a) and b) and c);
Z is a group defined by formula -(L)ₘ-Z₁, L is (C₁-C₈) alkyl, aryl, heteroaryl, phenyl, (C₁-C₈) alkylaryl, aryl(C₁-C₈) alkyl;
Z₁ is defined by formula 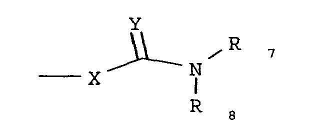
wherein X, Y is O, S; R₇, R₈ which may be identical or different are hydrogen, (C₁-C₈) alkyl, aryl, heteroaryl, (C₁-C₈) alkylaryl, phenyl, cyclopropyl, (CH₂)ₙ-COOH;
wherein n and m are an integer ≥ 1, preferably m is comprised between 1 and 4 and n is comprised between 1 and 6;
provided that at least one group R₁ to R₆ is Z or Z₁ and that R₁ is not H or Z₁; or a pharmaceutical salts or a stereoisomer thereof.

6. Compound according to claim 5 of formula G⁺ 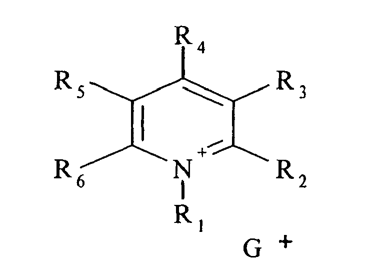 optionally under a ammonium salt form G⁺ W⁻ wherein W is the leaving group of an alkylating agent of formula R1-W or under a pharmacological acceptable salt.

7. Compound according to claim 5 or 6 of formula G1a or G1b or G1⁺

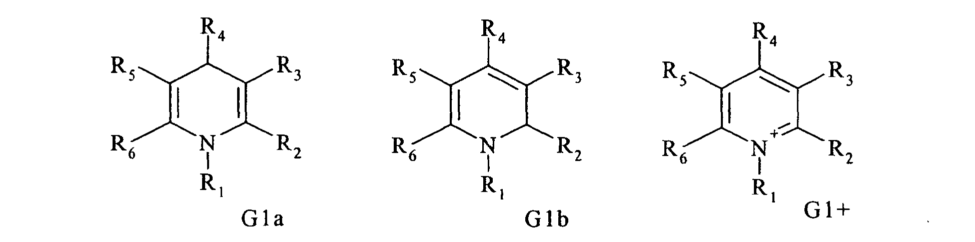

8. Compound according to claim 5 or 6 of formula G2 or G2⁺

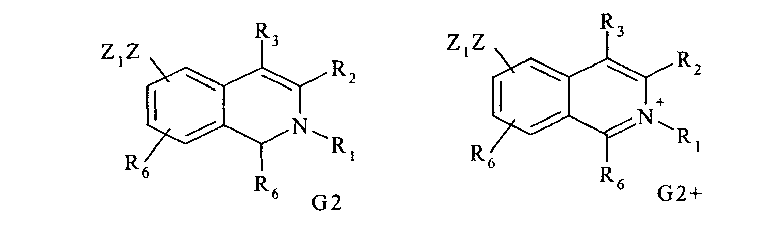

9. Compound according to claim 5 or 6 of formula G3a or G3b or G3⁺

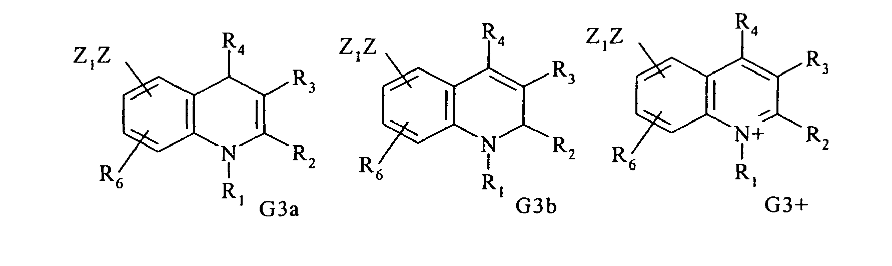

10. Compound according to any one of the claims 5 to 9 comprising an electron withdrawing group being in the position of the R₃ group, and R₃ is selected from the group comprising COOR, CONRR', CN, CF₃, -SO-R, -SO_{z}-R, -SO₂NRR', NO₂, halogen, or oxazolin, R, R' being a group alkyl, aryl, heteroaryl.

11. Compound according to any one of the claims 5 to 10 wherein
R₁ is (C₁-C₄) alkyl, -(L)ₘ-Z₁ wherein L is aryl, m is 1;
R₂ is H, (C₁-C₄) alkyl, phenyl, aryl;
R₃ is COOR, CONRR', CN, CF₃, -SO-R, -SO₂-R, -SO₂NRR', NO₂ halogen, or oxazolin, R, R' being a group (C₁-C₄) alkyl, aryl;
Z₁ is 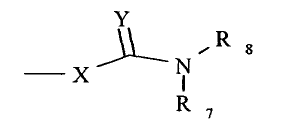 wherein X and Y are O, or X is O and Y is S or X is S and Y is O; R₇, R₈ which may be identical or different are hydrogen, (C₁-C₄) alkyl or (C₁-C₄) alkylaryl or phenyl.

12. Compound according to any one of the claims 5 to 11 comprising at least one radical C=Y, Y being O or S, and an oxidable and non protonable nitrogen atom N
wherein the distance d between the at least one carbon atom of the radical group C=Y and the nitrogen atom, when oxidized, is comprised between 0.3 and 0.8 nanometers, preferably 0,4 and 0,7 nanometers.

13. Compound according to any one of the claims 5 to 12 which is an acetylcholinesterase inhibitor, at least 500, preferably at least 1000 times more active in central nervous system CNS than in peripheral nervous system PNS.

14. Compound according claim 13 which is an acetylcholinesterase inhibitor, at least 500, preferably at least 1000 times more active in central nervous system CNS under its oxidized form than in peripheral nervous system PNS under its non oxidized form.

15. Compound according to any one of the claims 5 to 11 of the following formula G'1, G'2, G'3;

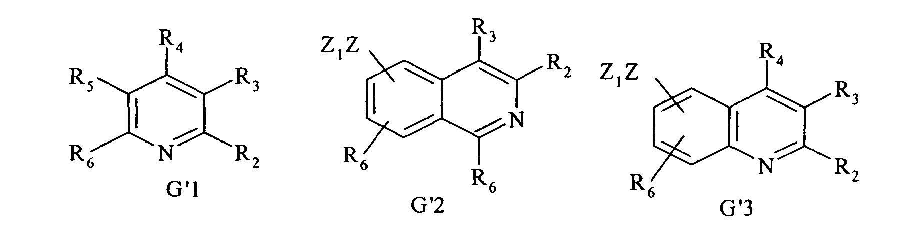

16. Process for the preparation of a compound according to any one of the claims 5, 7 to 11, which comprises the step of reduction of a compound of formula (G⁺ or Gᵢ⁺) W⁻ i being 1, 2 or 3, in the presence of a reducing agent.

17. Process for the preparation of a compound of formula (G⁺ or Gᵢ⁺)W⁻ i being 1, 2 or 3 as defined in any one of claims 6 to 11, which comprises a step of quaternization of the nitrogen atom of a compound of formula G'1, G'2, G'3 as defined in claim 15, by an alkylating agent R₁-W, R₁ being (C₁-C₈) alkyl, aryl, (C₁-C₈) alkylaryl, aryl(C₁-C₈) alkyl, alkoxy, hydroxy(C₁-C₈) alkyl, alkoxy(C₁-C₈) alkyl, phenyl, (CH₂)ₙ-COOH; W being a leaving group preferably selected from halogen, OTriflate, sulfate, tosylate, mesylate.

18. Process for the preparation of a compound of formula G'1, G'2, G'3 according to claim 15 which comprise a step of carbamoylation of a compound of the following formula E l or E2 or E3 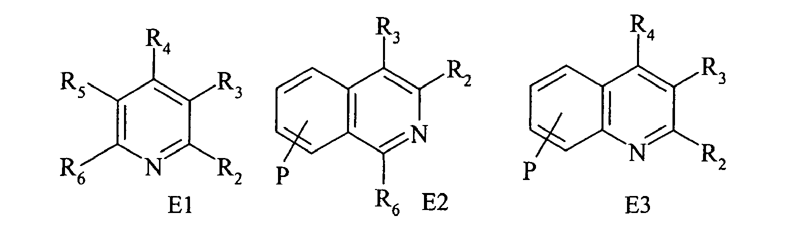 wherein P is OH, (L)ₘ OH and at least one R₅ or R₆ in formula E1 is OH or (L)ₘ OH; with an agent of formula W'-Z or W'-Z'₁, wherein Z'1 is 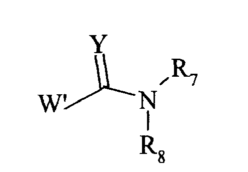 and W' is a leaving group preferably selected from halogen, OTriflate, sulfate, tosylate, mesylate and R₂ R₃ R₄ R₅ R₆ L, m, Y, R₇, R₈ have the same meaning as defined in the preceding claims.

19. Process for the preparation of compound of formula G⁺W or Gᵢ⁺ W⁻ i being 1, 2 or 3 as defined in any one of claims 6 to 11 and with R₁ being Z, which comprises a step of quaternization of a compound of the formula E1, E2 or E3 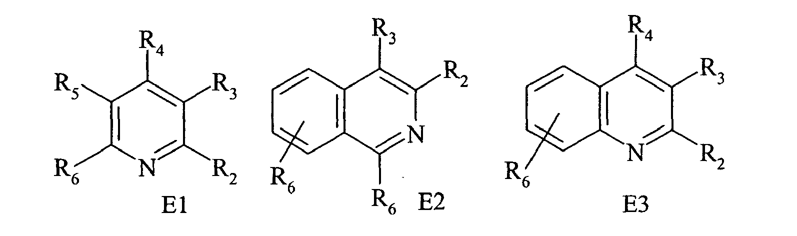 with an alkylating agent bearing a carbamate group of formula W-Z, and R₂ R₃ R₄ R₅, R₆, Z and W have the same meaning as defined in the preceding claims.

20. Process according to any one of claims 16 to 19 wherein R₃ is hydrogen, alkyl, phenyl, (CH₂)ₙ-COOH with n being ≥ 1 or an electron withdrawing group selected from the group comprising COOR, CONRR', CN, CF₃, -SO-R, -SO₂-R, -SO₂NRR', NO₂ halogen, or oxazolin, R, R' being a group alkyl, aryl, heteroaryl.

21. A pharmaceutical composition comprising at least one compound according to any one of the claims 1 to 14, 28 and a pharmacologically acceptable carrier.

22. A pharmaceutical composition according to claim 21 for its use as an acetylcholinesterase inhibitor in the CNS.

23. A pharmaceutical composition according to claim 21 or 22 for its use in the treatment of neurodegenerative diseases, preferably Alzheimer's disease in a human or other animal subject.

24. A pharmaceutical composition comprising a compound of formula G⁺, G1⁺, G2⁺ or G3⁺ according to any one of claims 6 to 12, 28 for its use as acetylcholinesterase inhibitor in the PNS.

25. A pharmaceutical composition according to claim 24 for its use in the treatment of myasteny disease in a human or other animal subject.

26. Use of a safe and effective amount of a compound of any one of formula G, G1a, G1b, G2, G3a, G3b as defined in any one of the claims 5 to 14, 28 for the manufacture of a prodrug for the treatment of disorders associated to neurodegenerative diseases in a human or other animal subject, wherein said treatment comprises administering said prodrug to said subject.

27. Use of a safe and effective amount of a compound of any one of formula G+, G1+, G2+, G3+ as defined in any one of the claims 6 to 12, 28 for the manufacture of a drug for the treatment of disorders associated to neurodegenerative diseases in a human or other animal subject, wherein said treatment comprises delivering said drug to the PNS of said subject.

28. Compound of formula G or G⁺ as defined in claims 5 or 6, the names of which follow;
*1*. Ethyl 1-methyl-7-(*N,N*-dimethylcarbamate)-1,4-dihydroquinoline-3-carboxylate;
*2*. Ethyl 1-methyl-5-(*N,N*-dimethylcarbamate)-1,4-dihydroquinoline-3-carboxylate;
*3*. Ethyl 1-methyl-5,7-di(*N,N*-dimethylcarbamate)-1,4-dihydroquinoline-3-carboxylate;
*4*. Ethyl 1-methyl-5,8-di(*N,N*-dimethylcarbamate)-1,4-dihydroquinoline-3-carboxylate;
*5*. Ethyl 1-methyl-5-(*N,N*-dimethylthiocarbamate)-1,4-dihydro-5-*O*-quinoline-3-carboxylate;
*6*. Ethyl 1-methyl-5-(*N,N*-dimethylthiocarbamate)-1,4-dihydro-5-*S*-quinoline-3-carboxylate;
*7*. 1-Methyl-5-(*N,N*-dimethylcarbamate)-3-(*N,N*-diethylcarboxamido)-1,4-dihydroquinoline;
*8*. 1-Methyl-7-(*N,N*-dimethylcarbamate)-3-(4,5-dihydro-4,4-dimethyl-2-oxazolyl)-1,4-dihydroquinoline;
*9*. 1-Methyl-5-(*N,N*-dimethylcarbamate)-3-trifluoromethyl-1,4-dihydroquinoline;
*10.* (+/-)-1-Methyl-3-(4-methylphenylsulfinyl)-5-(*N,N*-dimethylcarbamate)-1,4-quinoline;
*11.* 1-Methyl-3-(4-methylphenylsulfonyl)-5-(*N,N*-dimethylcarbamate)-1,4-dihydroquinoline;
*12.* 1-Methyl-5-(*N,N*-dimethylcarbamate)-3-(*N*-phenylsulfomanide)-1,4-dihydroquinoline;
*13.* 1-Methyl-6,7-di(*N,N*-dimethylcarbamate)-3-nitro-1,4-dihydroquinoline;
*14.* Ethyl 1-methyl-2-phenyl-6,7-di(*N,N*-dimethylcarbamate)-1,4-dihydroquinoline-3-carboxylate;
*15*. Ethyl 1,2,4-trimethyl-7-(*N,N*-dimethylcarbamate)-1,4-dihydro-3-quinolinecarboxylate;
*16*. 2-Methyl-7-(*N*,*N*-dimethylcarbamate)-1,2-dihydroisoquinoline;
*17*. 2-Methyl-7-(*N*,*N*-dimethylthiocarbamate)-1,2-dihydro-7-*O*-isoquinoline;
*18*. 2-Methyl-7-(*N*,*N*-dimethylthiocarbamate)-1,2-dihydro-7-*S*-isoquinoline;
*19*. 1,2-Dimethyl-7-(*N*,*N*-dimethylthiocarbamate)-1,2-dihydro-7-*O*-isoquinoline;
*20*. Ethyl 2,3-dimethyl-6,8-di(*N*,*N*-dimethylcarbamate)-1,2-dihydro-4-isoquinolinecarboxylate;
*21*. 2,3-Dimethyl-6,8-di(*N*,*N*-dimethylcarbamate)-1,2-dihydro-4-isoquinolinecarboxamide;
*22*. 2,3-Dimethyl-4-(4,5-dihydro-4,4-dimethyl-2-oxazolyl)-6,8-di(*N,N-*dimethylcarbamate)-1,2-dihydroisoquinoline;
*23*. (+/-)-2,3-Dimethyl-4-(4-methylphenylsulfinyl)-6,8-di(*N,N*-dimethylcarbamate)-1,2-dihydroisoquinoline;
*24*. 2,3-Dimethyl-4-(methylphenylsulfonyl)-6,8-di(*N,N*-dimethylcarbamate)-1,2-dihydroisoquinoline;
*25*. 2,3-Dimethyl-4-(*N*-phenylsulfonamide)-6,8-di(*N,N*-dimethylcarbamate)-1,2-dihydroisoquinoline;
*26*. 2,3-Dimethyl-4-(trifluoromethyl)-6,8-di(*N,N*-dimethylcarbamate)-1,2-dihydroisoquinoline;
*27*. Ethyl 1-methyl-5-(*N,N*-dimethylcarbamate)-1,4-dihydropyhdine-3-carboxytate
*28*. Ethyl 1-methyl-5-(*N,N*-dimethylthiocarbamate)-1,4-dihydro-5-*O*-pyridine-3-carboxylate;
*29*. Ethyl 1-methyl-5-(*N*,*N*-dimethylthiocarbamate)-1,4-dihydro-5-*S*-pyridine-3-carboxylate;
*30*. 1-Methyl-3-(methylsulfonyl)-5-(*N,N*-dimethylcarbamate)-1,4-dihydropyridine;
*31*. 1-Methyl-3-(*N,N*-diethylcarboxamido)-5-(*N,N*-dimethylcarbamate)-1,4-dihydropyridine;
*32*. (+/-)-1-Methyl-3-(methylsufinyl)-5-(*N,N*-dimethylcarbamate)-1,4-dihydropyridine;
*33*. 1-Methyl-3-(trifluoromethyl)-5-(*N,N*-dimethylcarbamate)-1,4-dihydropyridine;
*34*. 1-Methyl-3-(4,5-dihydro-4,4-dimethyl-2-oxazolyl)-5-(*N,N*-dimethylcarbamate)-1,4-dihydropyridine;
*35*. *N,N*-Diethyl-1-methyl-5-(*N,N*-dimethylcarbamate)-1,4-dihydro-3-pyridinesulfonamide;
*36*. Ethyl 1-[2-(*N,N*-dimethylcarbamate)benzyl]-1,4-dihydro-3-pyridinecarboxylate;
*37*. 1-[2-(*N,N*-dimethylcarbamate)benzyl]-3-(methylcarbamoyl)-1,4-dihydropyridine;
*38*. 1-[2-(*N,N*-dimethylcarbamate)benzyl]-3-(4,5-dihydro-4,4-dimethyl-2-oxazolyl)-1,4-dihydropyridine;
*39*. *N,N*-Diethyl-1-[2-(*N,N*-dimethylcarbamate)benzyl]-1,4-dihydro-3-pyridinesulfonamide;
*40*. Ethyl 1-[2-(*N,N*-dimethylcarbamate)benzyl]-1,4-dihydro-3-quinolinecarboxylate;
*41*. 1-[2-(*N*,*N*-dimethylcarbamate)benzyl]-1,4-dihydro-3-(dimethylcarbamoyl)-quinoline;
*42*. 1-[2-(*N*,*N*-dimethylcarbamate)benzyl]-1,4-dihydro-3-(4,5-dihydro-4,4-dimethyl-2-oxazolyl) quinoline;
*43*. *N,N*-Dimethyl-1-[2-(*N,N*-dimethylcarbamate)benzyl]-1,4-dihydro-3-quinolinesulfonamide;
*44*. Ethyl 2-[2-(*N,N*-dimethylcarbamate)benzyl]-1,2-dihydro-4-isoquinolinecarboxylate;
*45*. 2-[2-(*N,N*-dimethylcarbamate)benzyl)-4-(N-β-phenethylcarbamoyl)-1,2-dihydroisoquinoline;
*46*. 2-[2-(*N,N*-dimethylcarbamate)benzyl]-4-(4,5-dihydro-4,4-dimethyl-2-oxazolyl)-1,2-dihydroisoquinoline;
*47*. *N,N*-diethyl-2-[2-(*N,N*-dimethylcarbamate)benzyl]-1,2-dihydro-4-isoquinolinesulfonamide;
*48*. 1-[2-(*N,N*-dimethylcarbamate)benzyl]-3-(trifluoromethyl)-1,4-dihydropyridine;
*49*. 1-[2-(*N,N*-dimethylcarbamate)benzyl]-3-(trifluoromethyl)-1,4-dihydroquinoline;
*50*. 1-[2-(*N,N*-dimethylcarbamate)benzyl]-3-(trifluoromethyl)-1,2-dihydroisoquinoline;
*51*. Ethyl 1-methyl-2-[2-(*N*,*N*-dimethylcarbamate)phenyl]-1,4-dihydropyridine-3-carboxylate;
*52*. 1-Methyl-2-[2-(*N*,*N*-dimethylcarbamate)phenyl]-1,4-dihydro-*N*,*N-*dimethylnicotinamide;
*53*. 1-Methyl-2-(2-(*N,N*-dimethylcarbamate)phenyl]-3-(methylsulfonyl)-1,4-dihydropyridine;
*54. N*-Methyl 1-methyl-2-[2-(*N,N*-dimethylcarbamate)phenyl]-1,4-dihydro-3-pyridinesulfonamide;
*55.* Ethyl 1-methyl-6-[2-(*N,N*-dimethylcarbamate)phenyl]-1,4-dihydropyridine-3-carboxylate;
*56.* Ethyl 1-methyl-5-[2-(*N,N*-dimethylcarbamate)phenyl]-1,4-dihydropyridine-3-carboxylate;
*57.* Ethyl 1-methyl-2-[2-(*N,N*-dimethylcarbamate)phenyl]-1,4-dihydroquinoline-3-carboxylate;
*58.* Ethyl 1-methyl-8-[2-(*N,N*-dimethylcarbamate)phenyl]-1,4-dihydroquinoline-3-carboxylate;
*59.* 1-[2-(*N,N*-dimethylcarbamate)phenyl]-2-methyl-1,2-dihydroisoquinoline;
*60.* 2-Methyl-4-[2-(*N,N*-dimethylcarbamate)phenyl]-1,2-dihydroisoquinoline;
*61.* Ethyl 1-methyl-7-(*N,N*-dimethylcarbamate)-1,2-dihydroquinoline-3-carboxylate;
*62.* Ethyl 1-methyl-5-(*N,N*-dimethylthiocarbamate)-1,2-dihydro-5-*S*-quinoline -3-carboxylate;
*63.* 1-Methyl 5-(*N,N*-dimethylcarbamate)-3-(4,5-dihydro-4,4-dimethyl-2-oxazolyl)1,2-dihydroquinoline;
*64.* Ethyl 1-methyl-5-(*N,N*-dimethylcarbamate)-1,2-dihydropyridine-3-carboxylate;
*65.* Ethyl 1-methyl-5-(*N,N*-dimethylthiocarbamate)-1,2-dihydro-5-*S*-pyridine-3-carboxylate;
*66.* 1-Methyl-3-(*N,N*-diethylcarboxamido)-5-(*N,N*-dimethylcarbamate)-1,2-dihydropyridine
or their corresponding ammonium form thereof.
